# EUROPEAN PATENT APPLICATION

(11) **EP 3 156 487 A1**
(43) Date of publication of application: **19.04.2017**
(21) Application number: 15807551.5
(22) Date of filing: 20.04.2015
(51) Int. Cl.: C12N 15/70, C12N 15/68, C12N 15/69, C12N 15/62, A61K 39/385, A61K 39/00, A61P 37/04

(54) **ESCHERICHIA COLI T7 EXPRESSION VECTOR, VECTORS FOR THE CO-EXPRESSION AND CO-PURIFICATION OF RECOMBINANT PEPTIDES IN/WITH CARRIER PROTEINS, USE OF EXPRESSION VECTORS FOR OBTAINING COMPLEXES WITH MULTIPLE ANTIGENS AND IMMUNOMODULATORS**

(30) Priority: 13.06.2014 BR 102014145028
(71) Applicant: Ouro Fino Saúde Animal Ltda., 14140-000 Cravinhos - SP (BR)
(72) Inventor: ROMERO RAMOS, Celso Raul, 14020-380 Ribeirão Preto - SP (BR); MARROQUIN QUELOPANA, Miryam, 14020-380 Ribeirão Preto - SP (BR)
(74) Representative: Herzog, Fiesser & Partner Patentanwälte PartG mbB
(86) International application number: PCT/BR2015/050047
(87) International publication number: WO 2015/188240

(57) **Abstract**

The present invention relates to a vector for the expression of recombinant proteins, antigens, pathogen-like particles and immunogenic complexes, said vector (pMRKA vector) being produced by modifying the plasmids containing the gene sequence of the T7 promoter of E. coli, this modification being mainly characterized by the substitution of the ampicillin-resistance gene by the kanamycin-resistance gene, and by the insertion of the *par* sequence (partition sequence which determines the efficient segregation of the plasmids in daughter cells during cell division). Also provided are expression vectors based on the pMRKA plasmid, which additionally comprise at least one of the gene sequences of the exosome of P. abyssi, which vectors are designated pMRKA-EXO, pMRKA-RING and pSUMAC. The invention also provides the vectors additionally comprising gene sequences with immunomodulatory or immunoregulatory activity, preferably the pMRKA-Z-Z-EXO and pMRKA-Z-Z-RING vectors. Other aspects of the invention include the method for producing said expression vectors and the use of the obtained vectors.

## Description

### Technical Field

The present invention refers to a vector for the expression of recombinant proteins herein called pMRKA, comprising a sequence of the T7 bacteriophage promoter of *E*. *coli,* kanamycin resistant gene and *par* sequence (partition sequence which determines the efficient segregation of the plasmids in daughter cells during cell division), in order to increase plasmids stability. Additionally, it is provided expression vectors based on pMRKA plasmids additionally comprising at least one of the *P. abyssi* exosome gene sequences, such vectors herein called pMRKA-EXO, pMRKA-RING and pSUMAC, that allow the co-expression and co-purification of recombinant proteins, particularly for obtaining complexes with multiples vaccine antigens and immunomodulators.

Other aspects of the invention include a method for producing said expression vectors and the use of the obtained vectors.

### Background of the Invention

Numerous *E*. *coli* expression vectors have been developed using strong promoters, the first one used was T7 promoter of T7 bacteriophage. The expression systems based on T7 are broadly used for super-expression in large scale of recombinant proteins in prokaryotes and eukaryotes cells. The system uses polymerase RNA of T7 that is a highly active enzyme, extending RNA chains eight times faster than the RNA polymerase of *E*. *coli.*

T7 vectors of *E*. *coli* are broadly known, for example, pAE vector was described by Ramos et al. (Ramos et al. 2004, A high-copy T7 Escherichia coli expression vector for the production of recombinant proteins with a minimal N-terminal His-tagged fusion peptide. Braz J Med Biol Res.37(8):1103-9), and having a nucleotides sequence of SEQ ID No:1. pAE vector is a derivative from the commercial vector pRSETA (Life Technologies), from which were taken the sequences of Xpress Epitope, enterokinase cutting site, in order to remain in the modified plasmid only a minimal fusion of non-removable 6xHis. pAE vector is successfully used in bench scale, but its performance is reduced in fermenters with high density cell cultures, because pAE is an unstable plasmid under these fermentation conditions.

Thus, the systems of recombinants proteins expression need to be improved, in order to make possible, among other factors, the recombinant proteins production in industrial scale which require high density cell fermentation; greater number of plasmid copies per cell to assure the desired protein expression; improved stability of the produced proteins; use of selection markers that do not interfere with living organisms, particular animals and humans.

One of the selection markers being used is the ampicillin resistant, antibiotic with broad use in treating human beings and animals. It is obvious that this marker is not desirable due to the consequences that may occur. Hence, this marker has been subject of researches for replacement with another antibiotic not used in humans and animals. For example, document EP1925670 describes *E*. *coli* expression vector in which the ampicillin resistant gene was replaced with a kanamycin resistant gene.

Despite the advances already made in the expression vector field, there is still a need for improvements in both the multipurpose plasmids approach and in plasmids oriented for the expression of specific proteins in order to achieve improved conditions for industrial scale production of recombinant proteins.

The vaccines based on monomeric antigens have a low immunogenicity and they cannot provide similar protection as the conventional vaccines, which use whole organisms as attenuated or inactivated bacteria and viruses (Morein B, & Simons K (1985) "Subunit vaccines against enveloped viruses: virosomes, micelles and other protein complexes". Vaccine. 3:83-93). Due to this, despite the great number of candidate vaccines and publications related to them, there are no vaccines based in this type of antigens available in the market.

Recent studies show that the great molecular mass complexes formation, such as VLPs (Virus Like Particles), Virosomes and ISCOM (antigen-containing lipids vesicles), PLPs (Pathogen Like Particles, obtained by nanotechnology), are highly immunogenic. (Rosenthal J.A. et al. (2014) Pathogen-like particles: biomimetic vaccine carriers engineered at the nanoscale. Current Opinion in Biotechnology (Nanobiotechnology •Systems biology) 28: 51-58). Nevertheless, the process for obtaining such antigens is expensive and complex, thus their use is limited.

Antigen carrier structures with lower complexity were obtained using sequences tending to form fibers and successfully used in laboratory tests: Rudra JS et al. (2010) "A self-assembling peptide acting as an immune adjuvant". Proc Natl Acad Sci USA. 107(2):622- 7; Rudra JS. et al. (2012) "Self-assembled peptide nanofibers raising durable antibody responses against a malaria epitope". Biomaterials. 33(27):6476-84; Pepponi I. et al. (2013) Immune-complex mimics as a molecular platform for adjuvant-free vaccine delivery. PLoS One. 8(4):e60855; e Miyata T, et al. (2011) Tricomponent immunopotentiating system as a novel molecular design strategy for malaria vaccine development. Infect Immun. 79:4260-75. In the meantime, the complexes known in the prior art can carry only one antigen and one modulator, and in order to obtain effective protection against bacteria and parasites, it is necessary the use of one more antigen in vaccine formulation.

In the present application, it became possible the use of a archae exosome complex, particularly *Pyrococcus abyssi,* as a system capable of carrying more than one antigen and immunomodulators, in such a way that is possible co-expressing and co-purifying the antigens from one single culture. As *P. abyssi* is hyperthermophile, fusion proteins containing the exosome proteins can present thermostability.

### Summary of the Invention

The objective of the present invention is to provide stable plasmids that allows an enhanced production, in industrial scale, of recombinant proteins both in separated form and as forming multiprotein complexes (these could be thermostable) with application in developing multi antigenic vaccines.

According to the first aspect of the present invention, it is provided a T7 expression vector of *Escherichia coli* characterized by: (a) having a high number of copies per cell; (b) being highly stable in high density cellular fermentation conditions; (c) sized about 3 Kpb; (d) having an antibiotic resistant marker non-used in humans and animals. More preferably, T7 expression vector of *E*. *coli* of the invention is pMRKA plasmid and it comprises a nucleotide sequence SEQ ID No: 19.

According to a second aspect of the invention, there is provided a vector for expressing fusion polypeptides for carrier proteins, having as characteristics: (a) a vector according the first aspect of the invention; and (b) at least one *P. abyssi* exosome gene encoding at least one immunogenic protein, antigens or immunoregulatory molecules carrier protein. More preferably, said vectors are: pMRKA-EXO plasmid comprising a nucleotide sequence SEQ ID No: 36, pMRKA-RING plasmid comprising a nucleotide sequence SEQ ID NO: 37, and the pSUMAC plasmid comprising the nucleotide sequence SEQ ID NO: 40.

According to a third aspect of the invention, there is provided vectors for expressing fusion polypeptides for carrier proteins, said vectors additionally having immunomodulatory activity and presenting the following characteristics: (a) a vector according to the first aspect of the invention; and (b) at least one *P*. *abyssi* exosome gene encoding at least one immunogenic proteins, antigens or immunoregulatory molecules carrier protein and (c) at least one immunomodulation segment, such as the Z domain. More preferably, said vectors are pMRKA-ZZ-EXO plasmid comprising a nucleotide sequence SEQ ID NO: 43, and pMRKA-ZZ-RING plasmid comprising a nucleotide sequence SEQ ID NO: 45.

According to a fourth aspect of the invention, it is provided a method for obtaining an expression vector according to the first aspect of the invention, comprising the following steps: (i) exchange the ampicillin resistant gene in vector of SEQ ID NO: 1 with the kanamycin resistant gene; (ii) amplification of the plasmid fragment of SEQ ID NO: 1 corresponding to the segment located among nucleotides 804 to 1857; (iii) binding the amplified segments from step (ii) with the kanamycin resistant gene; (iv) amplification of the plasmid obtained in step (iii) to insert restriction sites in base 2607 of pMK plasmid; (v) amplification of *par* sequence and insertion of the amplified sequence resulting among the restriction sites located at the base 2607 from pMK for obtaining pMRK plasmid; and (vi) eliminating the restriction sites of *BglII* and *NcoI* in the kanamycin resistant gene in pMRK vector to obtain pMRKA plasmid.

In a fifth aspect, the present invention refers to a method for obtaining an expression vector according to the second aspect of the invention, comprising the following steps: (i) preparing pMRKA plasmid according to the method of the fourth aspect of the invention; (ii) preparing at least one *P. abyssi* exosome gene sequence encoding at least one immunogenic proteins, antigens or immunoregulatory molecules carrier protein; (iii) synthesis of one double-stranded DNA containing said at least one *P. abyssi* exosome gene sequence and (iv) cloning said double-stranded DNA obtained in step (iii) into the said pMRKA plasmid. Such method allows obtaining fusion vectors pMRKA-EXO, pMRKA-RING and pSUMAC.

In a sixth aspect, the present invention refers to a method for obtaining an expression vector according to the third aspect of the invention, comprising the following steps: (i) preparing pMRKA plasmid according to the method of the fourth aspect of the invention; (ii) preparing at least on *P. abyssi* exosome gene sequence encoding at least one carrying immunogenic proteins, antigens or immunoregulatory molecules carrier protein; (iii) preparing at least one sequence presenting immunomodulatory activity, preferably at least one sequence of Z domain; (iv) synthesis of one double-stranded DNA containing said at least one gene sequence of *P. abyssi* exosome and at least one *P. abyssi* exosome and at least one sequence with immunomodulatory activity; and (v) cloning said double-stranded DNA obtained in step (iv) into the said pMRKA plasmid. Such method allows obtaining pMRKA-ZZ-EXO and pMRKA-ZZ-RING fusion vectors.

In a seventh aspect, the present invention refers to the use of vectors according to the first, second and third aspects of the invention, obtained according to the methods from the third, fourth and fifth aspects of the invention on preparing in large scale the thermostable recombinant proteins and additionally with immunomodulatory activity.

### Brief Description of the Drawings

Figure 1 shows the modifying sequence flow for obtaining vectors of the present invention: pMRKA, pMRKA-EXO, pMRKA-RING and pSUMAC.
Figure 2 shows the pAE vector map and fusion sequence (6xHis) and cloning sites, such vector was the plasmid base of modifications which result in the vectors of the invention.
Figure 3 illustrates the strategy used for exchanging the selection marker (antibiotic) into pAE vector for obtaining pMK vector.
Figure 4 shows the strategy used for the insertion of *par* sequence into pMK vector for obtaining pMRK vector.
Figure 5 shows the analysis of plasmids derived from pAE vector: pmK - pAE vector with KanR marker instead of AmpR; and pMRK - pAE vector with two modifications, kanamycin resistant sequence and *par* sequence; KAN - corresponding to the amplification products of kanamycin gene, *par -* amplification products of *par* sequence of pSC1010 plasmid.
Figure 6 illustrates the scheme of modifications performed by mutagenesis for obtaining pMRKA plasmid.
Figure 7 shows the scheme of locus containing PAB0419, PAB0420 and PAB0421 genes which encode Rrp4, Rrp41 and Rrp42 exosome proteins, respectively.
Figure 8 presents the diagram of *Pyrococcus abyssi* exosome structure surface; in the middle, the diagram of RNA surface bound to the exosome.
Figure 9 shows the synthetic gene for in-fusion cloning in the end of N- and C-terminal of Rrp4, Rrp41 and Rrp42 proteins of *P. abyssi* exosome.
Figure 10 illustrate a cloning strategy of synthetic gene containing genes of *P. abyssi* proteins in pMRKA vector.
Figure 11 shows the co-purification gel from *P. abyssi* exosome complex protein, co-expressed by pMRKA-EXO plasmid in *E*. *coli.*
Figure 12 illustrates a diagram of exosome core surface of *Pyrococcus abyssi,* formed by Rrp41 and Rrp42 proteins; in the central region it can be viewed the diagram of the RNA surface bound to the "RING" complex.
Figure 13 shows a cloning strategy of genes corresponding to the core of *P. abyssi* exosome in pMRKA vector for obtaining pMRKA-RING.
Figure 14 shows co-purification of Rrp41 and Rrp42 proteins, co-expressed in pMRKA-RING plasmid, by ion-exchange chromatography. 80°C - input, material treated at 80°C for 30 minutes followed by centrifugation; 1-5 fractions corresponding to the elution peak.
Figure 15 illustrates a diagram of complex surface formed by Rrp42 protein.
Figure 16 shows the strategy for cloning Rrp42 protein in pMRKA vector for obtaining pSUMAC vector.
Figure 17 shows purified Rrp42 protein gel in a step of cell lysis at 80°C for 30 minutes, followed by centrifugation.
Figure 18 illustrates a strategy for cloning Z-Z domains into pMRKA-EXO plasmid.
Figure 19 illustrates a strategy for cloning Z-Z domains into pMRKA-RING plasmid.
Figure 20 illustrates the analysis per SOS-PAGE from the co-purified complex containing *P. abyssi* exosome proteins with fusion of Z-Z domains in Rrp42 protein. M denotes the Molecular Mass Marker.
Figure 21 illustrates the analysis per SOS-PAGE from the co-purified complex containing *P. abyssi* exosome ring (RING) proteins with fusion of Z-Z domains in Rrp42 protein. M denotes the Molecular Mass Marker. 1 - denotes the purified ZZ-RING complex.

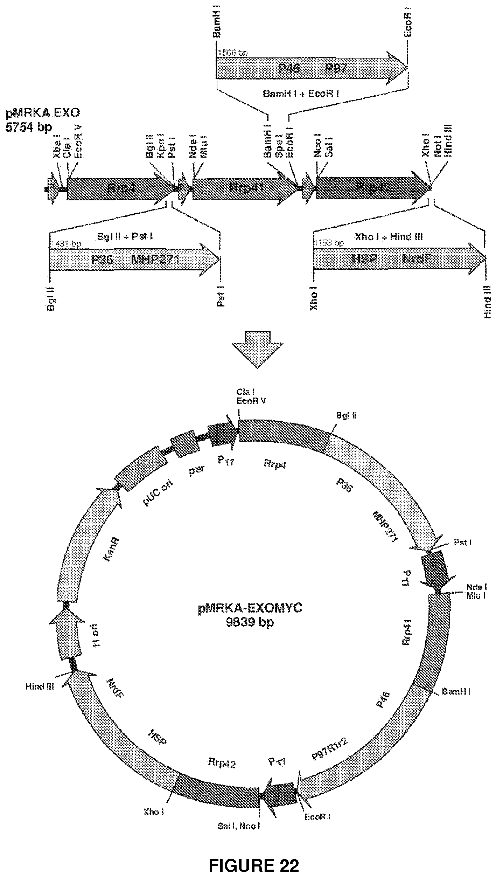

Figure 22 illustrates the strategy for cloning synthetic genes of vaccine antigens of *Mycoplasma hyopneumoniaeno* in pMRKA-EXO plasmid for obtaining pMRKA-EXOMYC vector.
Figure 23 illustrates the analysis for SOS-PAGE of polyproteins expression from antigens complexes of *Mycoplasma hyopneumoniae* with proteins from the *P. abyssi* exosome. M - denotes Molecular Mass Marker; 1 - denotes Rosetta (OE3)/pMRKA-EXOMYC strain; 2 - denotes Rosetta (OE3)/pMRKA-RINGMYC strain; 3 - denotes Rosetta (OE3)/pSUMAC-MYC strain; Non-induced denotes culture without the inducer presence; induced - denotes the induced culture with 1 mM ITPG.
Figure 24 illustrates the result of EXOMYX complex purification in *Escherichia coli,* by tangential filtration. M... - denotes the molecular mass marker; L - denotes he analysis of 2 µL of purified protein; 2 - denotes the analysis of 4 µL of purified protein.
Figure 25 illustrates the strategy for cloning synthetic genes from Inhibin antigen, as described and claimed by the patent application PI102014005376-0, in pSUMAC vector.
Figure 26 illustrates the analysis for SOS-PAGE of the fusion protein expression Rrp42-IniOF during the formation (induction) and purified product for thermal treatment (100 kDa retained).

### Detailed Description of the Invention

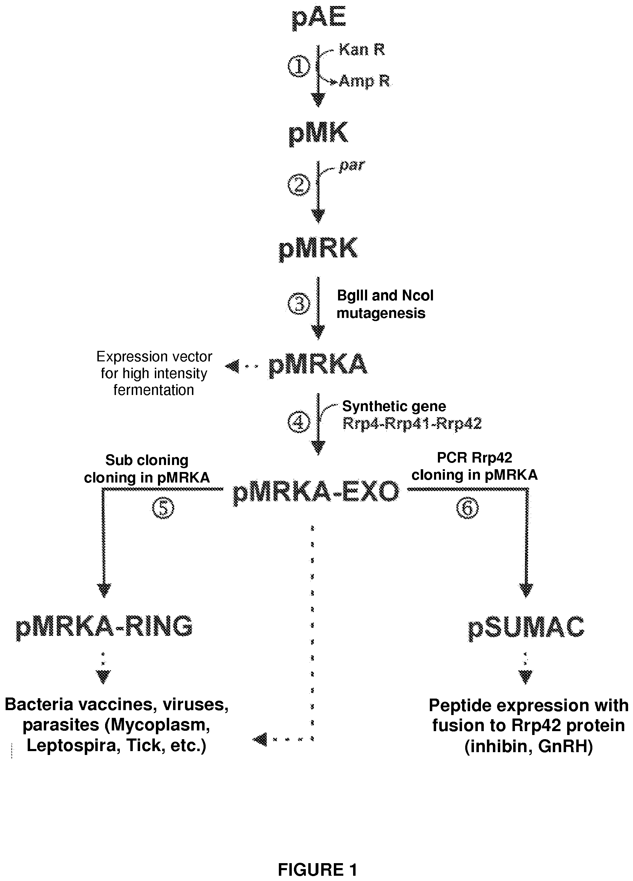

The present invention refers to the obtaining of expression vectors for producing thermostable recombinant proteins, including vectors comprising plasmids for the expression of fusion recombinant proteins with carrier proteins arising from the P. abyssi exosome. More particularly, the invention refers to vectors obtained from pAE plasmids (Ramos et al., 2004), which was obtained by modifying pRSETA commercial plasmid (Life Technologies), mainly by modifying the antibiotic resistant gene (selection marker) and through the insertion of *par* sequence of pSC101 plasmid. The flowchart of modifications introduced in pAE plasmid and subsequent plasmids resulting from each modification step is represented in Figure 1.

The term "expression vector" is intended to mean, in the present description, the plasmids in its final form, resulting from the modifications introduced in the initial pAE plasmid and into the subsequent intermediate plasmids.

The terms "immunomodulatory sequence" and "immunoregulatory sequence" are herein used in interchangeably and meaning the sequences that provide immunoregulatory activity to the proteins/polypeptides containing gene sequences with this feature, for example, Z domain from A protein of S. aureus.

The terms "polypeptide" and "recombinant protein" are used herein in interchangeably and meaning amino acid sequences encoded from the plasmids of the invention, said sequences corresponding to recombinant proteins with improved thermostability.

As previously mentioned, pAE vector is adequate for expressing proteins in bench scale, but due to its fermenting instability in high density cultures, its performance becomes unacceptable for producing recombinant proteins in industrial scale. To solve these shortcoming of pAE vector, the following modifications were made according to the invention: (a) substitution of antibiotic resistant gene giving rise to the plasmid herein called pMK; (b) introducing the partition sequence "*par*" of pSC101 plasmid, resulting in the plasmid herein called pMRK; and (c) eliminating *BglII* and NcoI restriction sites from kanamycin resistant gene in pMRK vector.

### pMRK vector

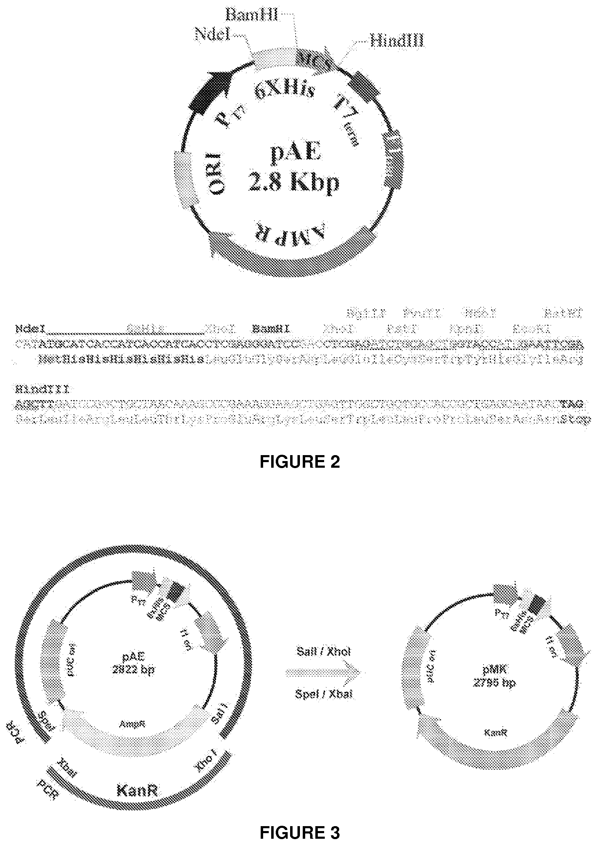

pMK vector results from the substitution of ampicillin resistant gene with kanamycin resistant gene in pAE vector, which comprises the nucleotide sequence SEQ ID NO: 1, and its map is represented in Figure 2.

The need to modify the pAE plasmid was due to the following reasons: (a) as the ampicillin resistant gene encodes a beta-lactamase which is located in periplasmic space of *E*. *coli* in high density culture, this enzyme has the possibility to migrate of the culture means and, consequently, to degrade the antibiotic, decreasing the selective pressure of the means and providing the buildup of cells without plasmid; and (b) in the production of expression vectors in industrial scale it is not recommended to use antibiotic similar to the ones used in medical and veterinary fields, as it is the case of ampicillin. As kanamycin does not have medical application, the ampicillin resistant gene in pAE vector was replaced with kanamycin resistant gene.

To achieve such replacement of antibiotic resistant gene, it was made a modification into the skeleton of pAE vector using "whole plasmid PCR" technique, which allows operating precise exchanges, as represented in the scheme showed in Figure 3.

According to the invention, it was used kanamycin resistant gene of pK18 plasmid (access GenBank number M17626.1 [Pridmore RD 1987. New and versatile cloning vectors with kanamycin-resistance marker. Gene. 56(2-3):309-12]). Such plasmid contains the transponson gene Tn5 that encodes a neomycin phosphotransferase enzyme (NPT), which provides resistance both to a neomycin (neo) and as well as kanamycin (kan). For the amplification, by PCR, of the region that encodes NPT together with its promoter (of the nucleotide 208 to nucleotide 1182, in the plasmid sequence pK18 (M17626.1 GenBank No.) the following primers were designed:
Kan-For - SEQ ID No: 2
   ATCTCGAGTTATGGACAGCAAGCGAACC
3'Kan-Rev - SEQ ID No: 3'
   CATCTAGAATTTCGAACCCCAGAGTCC 3'

Kan-For primer contains the restriction site Xhol and Kan-Rev primer the Xbal site (both underlined in the primer sequences). PCR was made with a high fidelity enzyme KOO DNA polymerase (Novagen) and used as mold the pK18 plasmid. As a result, the sequence containing kanamycin resistant gene (SEQ ID No: 4) was amplified, and the resulting gene encodes the NPT enzyme (SEQ ID No: 5).

Additionally, it was amplified, by PCR, a pAE plasmid fragment, from nucleotide 804 to nucleotide 1857, which does not contain ampicillin resistant gene. This amplification was made using the primers:
VecMar-For - SEQ ID No: 6
   5' CGACTAGTGCATTGGT AACTGTCAGACC 3'
VecMar-Rev - SEQ ID No: 7
   5' ATGTCGACGTGCCACCT AAATTGTAAGCG 3'

The VecMar-For primer contains the restriction site Spel, and the VecMar-Ver primer contains the restriction site Sall (both underlined in the primer sequences) in order to provide action of the enzymes that do not have cutting sites in pAE sequence. The amplification was achieved using a high fidelity enzyme KOO XL DNA polymerase (Novagen), indicated for amplifying DNA extensive fragments, as integral plasmids.

The cuts with Sall enzymes, in the amplified DNA of pAE vector, and Xhol in amplified DNA of kanamycin resistant gene, generate compatible ends. Such fact allows the gathering of these DNAs, at the same time wherein, in case the original cutting sites are lost, it is not necessary the introduction of cutting sites into the new vector during its construction. This also happens with Spel enzymes (in the amplified segment of the vector) and Xbal (in the amplified segment of KanR) that generate compatible ends after cut (see Figure 3). The binding of the vector amplified segments (cut with enzymes Sall and Spel and from KanR (cut with Xhol and Xba, result on plasmid called pMK, selected by its kanamycin resistance (see Figure 3) (SEQ ID No: 8)).

### pMRK vector

Despite improved by the substitution of the antibiotic resistant gene, pMK vector still presents unsatisfying stability, which was overcome, according to the present invention, by the input of a sequence, known as "*par*", that has the attribute to enhance plasmids stability.

In order to increase the stability of the plasmids of the invention, it was used the strategy of introducing the partition sequence "*par*" (that determines the efficient segregation of plasmids in the daughter cells during cell division) of pSC101 plasmid in pAE vector. The use of *par* sequence to enhance plasmid stability was previously described by Meacock et al. (Meacock PA and Cohen SN (1980) Partitioning of bacterial plasmids during cell division: a cis-acting locus that accomplishes stable plasmid inheritance. Cell. 20(2):529-42; WO 1984001172 A 1). It is worth mentioning that, although this strategy is very interesting under the point of view of stability of the obtained plasmids, currently, the systems broadly disseminated in research field for example, pET vectors) and in industrial scale production, they do not include the use of "*par*" sequence.

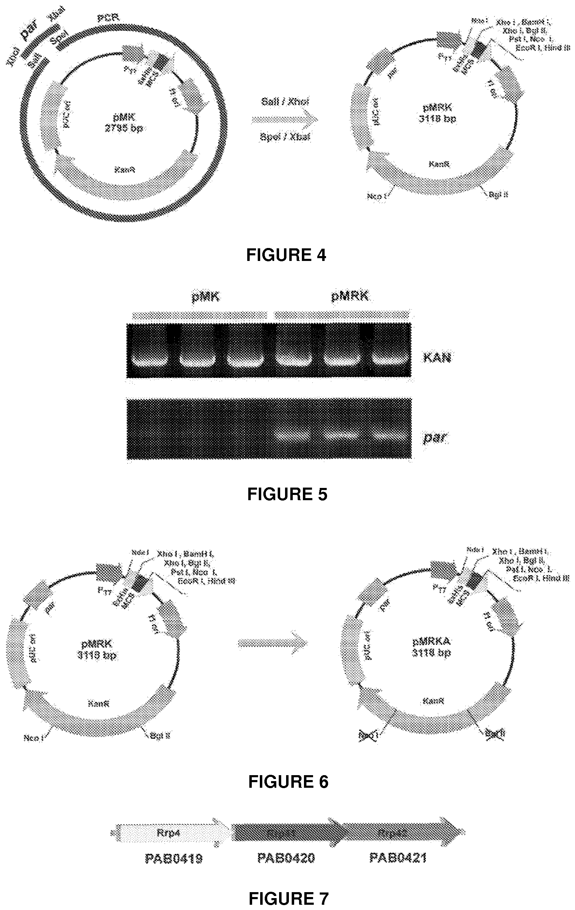

Thus, to increase stability of plasmids based on pAE vector, the partition sequence "*par*" was input in pMK plasmid using, one more time, a "whole plasmid PCR" technique, as shown in Figure 4.

In pMRK plasmid diagram, shown in Figure 4, it is highlighted the position of the cutting sites for restriction enzymes *BglII* and *NcoI*, both in selection marker KanR, as in cloning multiple site.

As source of "*par*" sequence, it was used the pSC101 plasmid itself (GenBank Access N° NC_002056, [Yamaguchi &Masamune Y. 1985. Autogenous regulation of synthesis of the replication protein in plasmid pSC101. Mal Gen Genet. 200(3):362-7]). To amplify the *par* sequence (from the nucleotide 4605 to nucleotide 4876, of GenBank sequence NC_002056) and subsequent insertion of such sequence in pMK vector, the following primers were designed:
ParFor - SEQ ID No: 9
   3' ATCTCGAGTTTGTCTCCGACCATCAGG 3'
ParRev - SEQ ID No: 10
   5' GTTCTAGACGGGAT AATCCGAAGTGG 3'

ParFor primer contains the restriction site XbaI, and Par-Rev primer contains the site for XhoI (both underlined in the primers sequence). PCR was made with a high fidelity enzyme KOO DNA polymerase (Novagen) and used as mold the pSC101 plasmid. As a result of the amplification, it was obtained the "*par*" sequence (SEQ ID NO: 11).

Additionally, pMK plasmid was amplified by PCR to input restriction sites in the base position 2607 from this plasmid, thus providing the insertion of "*par*" sequence amplified segment, through the use of the following primers:
VecPar-For - SEQ ID No: 12
   5' ATACTAGTACGCGGCCTTTTTACGGTTCC 3'
VecPar-Rev - SEQ ID No: 13
   5' ATGTCGACTGCTGGCGTTTTTCCAT AGG 3'

The VecMar-For primer contains SpeI restriction site, and the VecPar-Ver primer contains Sall site (both underlined in the primer sequences) in order to provide action of the enzymes that do not have cutting sites in pMK sequence. The amplification was achieved using a high fidelity enzyme KOO XL DNA polymerase (Novagen), which is indicated for amplifying whole plasmids.

As it was previously described, cuts with SalI and XhoI enzymes generate compatible ends of the digested DNA, in the same way as the cuts with Spel and XbaI enzymes. The binding of the vector amplified segments (cut with enzymes SalI and Spel and "*par*" sequence (cut with XhoI and Xba, resulted in the plasmid called pMRK, (SEQ ID NO:14)) (see Figure 4) selected by its kanamycin resistance.

Figure 5 shows the analysis by PCR of pMK and pMRK plasmids in relation to the presence of kanamycin resistant marker and *par* sequence.

### pMRKA vector

Finally, in order to eliminate restriction BglII and NcoI sites in kanamycin resistant gene, i.e., in KanR marker from pMRK vector, it was made two site-directed mutagenesis, using the PCR technique and using the "Quick Change Site-Directed Mutagenesis Kit" (Stratagene), as shown in Figure 6.

Firstly, it was made the mutagenesis in *BglII* site. This site is located after the promoter region of the gene which encodes neomycin phosphotransferase (NPT) enzyme, that provides kanamycin (kan) resistance. To effect C976T mutation, that corresponds to cytosine from site (AGATCT), BglFor and BglRev primer were designed, whose sequences are shown as follows:
BglFor (SEQ ID No: 15)
   GATGGCGCAGGGGATCAAGATTTGATCAAGAGACAGGATGAG
BglRev (SEQ ID No: 16)
   CTCATCCTGTCTCTTGATCAAATCTTGATCCCCTGCGCCATC

The position of the nucleotides that form BglII site is underlined and in the table is outstanding the mutagenic nucleotide. After mutagenesis cycle by PCR using "QuikChange Site-Directed Mutagenesis Kit" (Stratagene), the resulting plasmids were characterized by restriction analysis with BglII enzyme and *par* sequencing with Kan-For primer (SEQ ID No: 2) in order to verify if the mutagenesis was successful.

Subsequently, it was made the mutagenesis on NcoI site. This site is located on the region that encodes kanamycin resistance (kanR). To make the neutral mutation T1571C in the Thymine from the site (CCAIGG), that corresponds to a third position in encoding CAT codon of His188 in Neomycin Phosphotransferase, the primers NcoFor and NcoRev were designed. The alteration from CAT to CAC does not change this amino acid residue once the CAC codon keeps encoding Histidine.
NcoFor - SEQ ID No: 17
   ATCTCGTCGTGACCCACGGCGATGCCTGCTTGC
NcoRev - SEQ ID No: 18
   GCAAGCAGGCATCGCCGTGGGTCACGACGAGAT

The position of the nucleotides that form NcoI site is underlined and it is highlighted, in the table, the mutagenic nucleotide. After mutagenesis cycle by PCR using "QuikChange Site-Directed Mutagenesis Kit" (Stratagene), the resulting plasmids were characterized by restriction analysis with Ncol enzyme and *par* sequencing with Kan-Rev primer (SEQ ID No: 3) in order to verify if the mutagenesis was successful.

After the two mutagenesis cycle, BglII and NcoI sites, in cloning multiple sites, became single sites into the plasmid sequence, and thus they could be used for the desired cloning. The plasmid without cleavage sites BglII and Ncol in kanamycin resistant gene was named pMRKA (SEQ ID No: 19) and it corresponds to the first aspect of the present invention. Such vector encodes 6xHis fusion protein (SEQ ID No: 20) and NPT enzyme, with neutral mutation T1571C (SEQ I D No: 21).

The stability of pMRKA in industrial scale fermentation culture (increased cell density) was successfully tested.

pMRKA plasmid corresponds to the vector of the first aspect of the invention and it represents an advantageous option in relation to pET vector, which is the commercial vector used in most processes for producing recombinant proteins. The advantages of pMRKA are as follows:
- Smaller size: 2,8 Kpb against 5.4 kbp of pET; thus, pMRKA can carry bigger sized sequences.
- Higher number of copies per cell: pMRKA vector has 300-500 copies per cell against 20-50 copies of pET per cell; thereby pMRKA has a bigger gene charge and, in some cases, bigger expression compared with pET vector. pMRKA also facilitates the experiments of cloning and sequencing.
- Greater stability: the presence of "*pa*r" sequence makes pMRKA more stable in relation to pET plasmid.

### pMRKA-EXO vector

The advantages of pMRKA above mentioned allow using this plasmid for constructing a derivative vector expressing fusion proteins with carrier proteins, such vector comprises sequences encoding Rrp4, Rrp41 and Rrp42 proteins of *P. abyssi* proven important as antigens or immunogenic complexes-carrying proteins.

The bacteria *Pyrococcus abyssi* is a hyperthermophile archaebacterium, which lives near the hydrothermal vents on the ocean floor. This archae tolerates temperatures above 100°C and a pressure up to 200 atmospheres, however, it does not represent a health hazard for human beings, animals or plants. To tolerate such high temperatures and pressures, the evolution made *P. abyssi* to have extremely stable proteins, resistant to denaturation by temperature or pressure.

The genes that encode the proteins composing exosome complex of *Pyrococcus abyssi* are located in a single locus in genome of such archaebacteria (Koonin EV et al. (2001) Prediction of the archaeal exosome and its connections with the proteasome and the translation and transcription machineries by a comparative-genomic approach. Genome Res. 11:240-252) (see Figure 7).

In the process of the present invention, the exosome complex of *P*. *abyssi* was assembled *in vivo* and *in vitro* using recombinant proteins expressed in *Escherichia coli.* This complex was previously functionally and structurally described (Ramos CR, et al. (2006) The Pyrococcus exosome complex: structural and functional characterization. J Biol Chem. 281 :6751-6759; e Navarro MV et al. 2008. Insights into the mechanism of progressive RNA degradation by the archaeal exosome. J Biol Chem. 283:14120-14131).

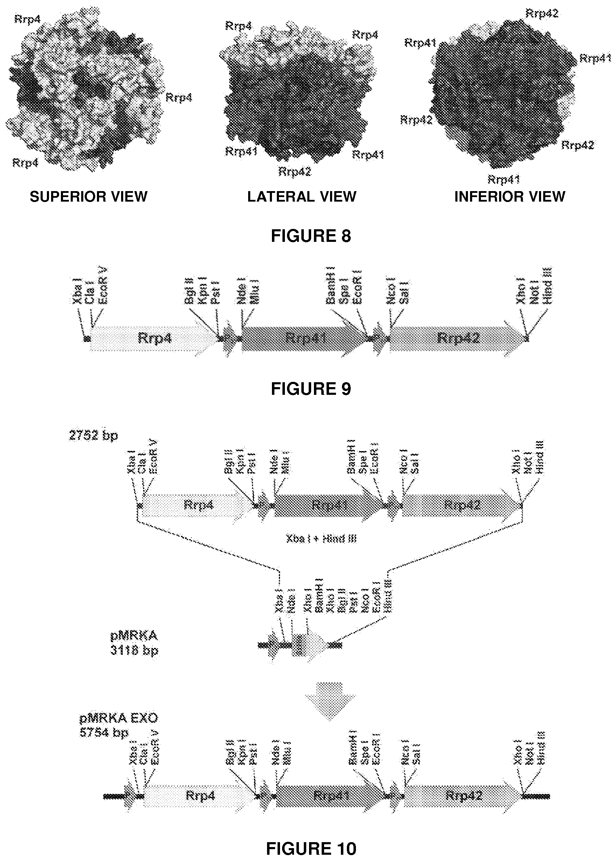

In Figure 8, it is shown surface models of exosome structure of *P*. *abyssi.* The structure of the complex named "RNases PH ring" formed by Rrp41 and Rrp42 was determined by crystallography (Navarro et al. 2008), while the structure and position of Rrp4 was modeled using as mold the archae exosomes structures Archaeoglobus fulgidus (Buttner K et al. (2005) Structural framework for the mechanism of archaeal exosomes in RNA processing. Mal Cell. 20:461-471) and *Sulfolobus solfataricus* (Lorentzen E, & Conti E. Crystal structure of a 9-subunit archaeal exosome in pre-catalytic states of the phosphorolytic reaction. Archaea. 2012:721869).

On the structure base of the exosome, it can be visualized a hexameric ring ("RING") formed by three copies of Rrp41 and Rrp42 proteins. This ring has binding activity to the RNA ad catalytic activity (RNase). Above the ring there are located three copies of Rrp4 protein, which contains binding domains to RNA.

This compact structure is highly soluble and stable, and can be expressed in high level in Escherichia coli, such characteristics facilitate its obtaining.

### The exosome as carrier of vaccine antigens

The archae exosome complex has unique characteristics that could be explored on developing new generation vaccines named "pathogen like particles", which use biomimetic to enhance immunologic response, such as:
- Capability of specific self-assembly when said complex is expressed in cells of *Escherichia coli.*
- High expression, solubility and thermostability of the complex, which facilitates the purification and formulation of recombinant proteins.

This complex also provides higher stability to the immunogenic protein during formulation and the storage of completed products, important aspect on developing biological products.

Ability to carry more than one antigen and immunomodulatory protein because, according to the structure determined by crystallography, the amino ends and terminal carboxyl from the three proteins are exposed to the solvent.

The technique of the present invention, using gene sequences of the *P. abyssi* exosome, represents a great advance in relation to the use of recombinant antigens alone in vaccine formulation, these antigens that cannot provide enough protection level as the level that is reached with whole organism as inactivated bacteria and viruses (Rosenthal J.A. et al. (2014) Pathogen-like particles: biomimetic vaccine carriers engineered at the nanoscale. Current Opinion in Biotechnology (Nanobiotechnology • Systems biology) 28: 51-58).

The possibility of gathering in a complex more than one antigen and immunomodulators make the proposed system of the present invention be better than the strategies reported using sequences tending to form fibers such as Rudra JS et al. (2010) "A self-assembling peptide acting as an immune adjuvant". Proc Natl Acad Sci U S A. 107(2):622-7; Rudra JS. et al. (2012) "Self-assembled peptide nanofibers raising durable antibody responses against a malaria epitope". Biomaterials. 33(27):6476-84; Pepponi I. et al. (2013) Immune-complex mimics as a molecular platform for adjuvant-free vaccine delivery. PLoS One. 8(4):e60855; and, Miyata T, et al. (2011) Tricomponent immunopotentiating system as a novel molecular design strategy for malaria vaccine development. Infect Immun. 79:4260-75.

### Synthetic gene for expression of P. abyssi exosome

To explore the possibility of using the exosome as a multiple protein carrying complex, according to the invention, it was designed a DNA sequence containing genes for Rrp4, Rrp41 and Rrp42 proteins of Pyrococcus abyssi (in this order, as they are found in genome of P. abyssi, see Figure 7), with restriction sites that allow in-fusion cloning of recombinant proteins in N- or C-terminal ends of exosome components.

Figure 9 shows projected sequence scheme according to the invention.

The construction shown in Figure 9 is planned for the cloning in pMRKA vector between XbaI and HindIII sites. Thereby, only the first gene (Rrp4) stays under control of T7 promoter of the vector. For genes Rrp41 and Rrp42 it was designed sequences containing T7 promoter, in similar design to the co-expression vectors pETDuet-1 and pACYDuet-1 (EMO Millipore), but without the operating sequence of operon lac, present in these commercial vectors.

### Sequences among encoding regions of exosome proteins

The first fragment design contains, from the 5' end, the cutting site for XbaI enzyme, the sequence transcribed from T7 promoter of pMRKA vector, the ribosome binding site (Ribosome Binding Site (RBS)), the translation start codon (highlighted in the square) and restriction sites ClaI and EcoRV for N-terminal in-fusion cloning with Rrp4 protein, as shown below.
Rrp4 RBS - SEQ ID NO: 22

Xbal RBS Clar EcoRV
TCTAGAAATAATTTTGTTTAACTTTAAGAAGGAGATATATATATGGCTATCG
ATGCGGATATCGCC M A I D A D I A

The second designed fragment contains the cutting site for BglII enzyme, aiming C-terminal in-fusion cloning of Rrp4 protein, stop codon and KpnI and PstI sites for unidirectional cloning. It follows the following sequences: a sequence containing T7 promoter for Rrp41 protein, the ribosome binding site, the translation start codon (highlighted in the square) and restriction sites Ndel and Mlul for N-terminal fusion cloning with Rrp41 protein, as shown below.
Rrp4 end and Promotor of Rrp41 and start - SEQ ID NO: 23

BglII KpnI PstI
AGATCTTAAGGTACCCTGCAGGCTTAAGTCGAACAGAAAGTAATCGTATTG
TACACGGCCGCAACT

R S * Rrp4

T7 promoter

TCGAAATCAATACGACTCACTATAGGGAGACCACAACGGTTTCCCATCTTA
GTATATTAGTTTAAC RBS NdeI MluI

TTTAAGAAGGAGATATACATATGGCTACGCGTGCA M A T R
A Rrp41

The third designed fragment contains the cutting site for BamHI enzyme for C-terminal in-fusion cloning with Rrp41 protein, stop codon and Spel and EcoRI sites, for unidirectional cloning. Continuing, it follows the following sequences: a sequence containing T7 promoter for Rrp42 protein, the ribosome binding site, the translation start codon (highlighted in the square) and restriction sites NcoI and SalI for N-terminal in-fusion cloning with Rrp42 protein:
Rrp41 end and Promoter for Rrp42 and start - SEQ ID NO: 24

BamHI SpeI EcoRI
GGATCCTAAACTAGTGAATTCGCTTAAGTCGAACAGAAAGTAATCGTATTG
TACACGGCCGCATA G S * Rrp41

T7 Promoter

ATCGAAATTAATACGACTCACTATAGGGAGACCACAACGGTTTCCCATCTT
AGTATATTAGTTAA

RBS NcoI SalI GTATAAGAAGGAGATATACCATGGGTGTCGAC

M G V D Rrp42

At last, the fourth designed fragment contains the cutting site for XhoI enzyme for C-terminal in-fusion cloning with Rrp42 protein, the stop codon, and NatI and HindIII sites (in 3' end), for unidirectional cloning.
Rrp42 end(SEQ ID NO: 25)

Rrp42 end(SEQ ID NO: 25)

XhoI NotI HindIII CTCGAGTAAGCGGCCGCAAGCTT

L E * Rrp42

### Design of synthetic genes for Rrp4. Rrp41 and Rrp42 proteins:

For designing synthetic genes, it was used the strategy based on the theory known as "Codon Harmonization" (Angov E. et al. (2011) Adjustment of codon usage frequencies by codon harmonization improves protein expression and folding. Methods Mol Biol. 705:1-13). According the theory of codon harmonization, the rare codons (that are less used than other codons in a given organism) have lower amount of tRNAs and perform an important role on protein folding. They are located in protein loops, among the defined secondary structure segments. These codons could decrease the protein synthesis speed through the ribosome, allowing the folding of the synthetized sequence before the beginning of a synthesis of another protein fragment.

The rare codons on PAB0419, PAB0420 and PAB0421 genes, that encodes Rrp4, Rrp41 and Rrp42 proteins, respectively, were identified through the use of ANACONDA program (http://bioinformatics.ua.pt/software/anaconda) feed with the usage of P. abyssi codons table, obtained from the database " *Codon Usage Database"* (http://www.kazusa.or.jp/codon/).

The localization of encoded amino acids by "rare codons" in tridimensional structures obtained for each protein was made using Swiss-Pdb-Viewer 4.0.1 program (http://spdbv.vitaHt.ch). In the "harmonization" of the synthetic gene, it was considered the rare codons that encode amino acids located in loops regions among secondary structure sequences, alpha-helix and beta-sheet.

For designing the synthetic gene, it was used Gene Designer 2.0 program (DNA2.0), manually selecting "rare" codons" of E. coli for each one of the amino acids identified by ANACONDA program and located in the structure by Swiss-Pdb-Viewe 4.0.1, in view to enhance protein folding. The Gene Designer 2.0 program was feed with the table called Usage of Codons for Highly Expressed Proteins in E.coli (Villalobos et al. (2006) GeneDesigner: a synthetic biology tool for constructing artificial DNA segments. BMC Bioinformatics. 7:285).

The amino acid sequences used for designing synthetic genes correspond to those related in GenBank for Pyrococcus abyssi strain GE5:

**Table 1: Gene sequences for P. abyssi used in the present invention**

| Protein | GenBank | No aa | Considered for synthesis | Synthetic Gene | Encoded protein |
|---|---|---|---|---|---|
| Rrp4 | NP 126300 | 265 | 2-262 | SEQ ID No: 26 | SEQ ID No: 27 |
| Rrp1 | NP 126301.1 | 249 | 3-246 | SEQ ID No: 28 | SEQ ID No: 29 |
| Rrp42 | NP 126302.1 | 274 | 2-274 | SEQ ID No: 30 | SEQ ID No: 31 |

In the drawings it was not considered the proteins starting methionine (e.g., Rrp41 has two methionines in the beginning of the translation).

Rrp41 protein contains catalytic site responsible for the RNase activity of the exosome. As this activity is not desired for the developing of vaccines, the present amino acids in active site were replaced, based on published results (Navarro MV et al. 2008. Insights into the mechanism of progressive RNA degradation by the archaeal exosome. J Biol Chem. 283:14120-14131). In this paper it is mentioned that the double mutation R89E and K91E in Rrp41 abolished the RNase activity of the exosome. In the synthetic gene it was made exchanges with R89T and K91S that resulted in a smaller change in the molecule charge compared with mutations reported in the literature.

Thus, the Rrp4 binding activity (that contains the binding domains to RNA S1 and KH) to ssRNA was maintained, as well as the ring structure (RING) formed by Rrp41-Rrp42 proteins. Without being limited to a specific theory, it is believed that the ssRNA of E. coli, that is co-purified with the exosome, can active TLR7 receptor and, thus, act as an adjuvant in exosome based vaccines.

At last, the sequences were gathered in the following order: SEQ ID No:22-SEQ ID No: 26-SEQ ID No: 23-SEQ ID No: 28-SEQ ID No: 24-SEQ ID No: 30-SEQ ID No: 25, to obtain a complete sequence corresponding to the diagram in Figure 10 - SEQ ID NO: 32.

The Rrp4, Rrp41 and Rrp42 protein sequences, with modifications in amino ends and carboxyl terminal contained in sequence SEQ ID No: 32 are presented in sequences SEQ ID No: 33, SEQ ID No: 34 and SEQ ID No: 35, respectively.

Additionally, a double-stranded DNA containing the sequence SEQ ID No: 32 was synthetized and cloned by XbaI and HindIII sites in pMRKA vector, as shown in diagram in Figure 10, resulting in pMRKA-EXO vector of the present invention.

According to the method of the fourth aspect of the invention, for the construction of pMRKA-EXO vector, pMRKA vector is the preferred on for being stable, with greater number of copies per cell, when compared with the vector form pET series and, furthermore, because it has a smaller size, which facilitates cloning of other genes in fusion with exosome proteins of P. abyssi. pMRKA-EXO plasmid comprises SEQ ID No: 36.

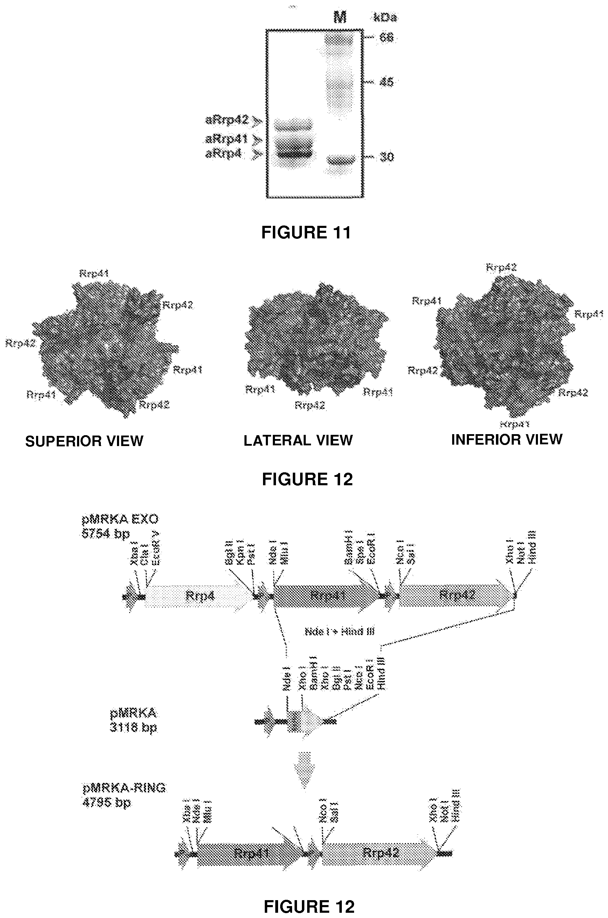

Figure 11 shows the result of the co-purification of Rrp4, Rrp41 and Rrp42 proteins, expressed in Escherichia coli by pMRKA-EXO plasmid.

### pMRKA-RING vector

### Ring of exosome RNases PH of P. abyssi

The core of the exosome is constituted by the hexamer formed by Rrp41 and Rrp41 proteins. Such structure in gel form is known as "RNases PH ring" and is herein called "RING" (see Figure 12).

This structure, simpler than the whole exosome structure in Figure 8, can also be used to fuse antigens and immunoregulatory molecules in the "pathogens like particles" concept as previously described. The RNase activity was abolished before R89T and K91S mutations in Rrp41 protein, while the binding activity of one-stranded RNA (ssRNA) was maintained. It is believed that ssRNA of E. coli, which co-purified together with the "RING" (already previously detected in crystalline structure of this complex (Navarro et al. 2008)) can activate the TLR receptor and, thus, act as an adjuvant, improving the immunologic response of vaccines based on this complex.

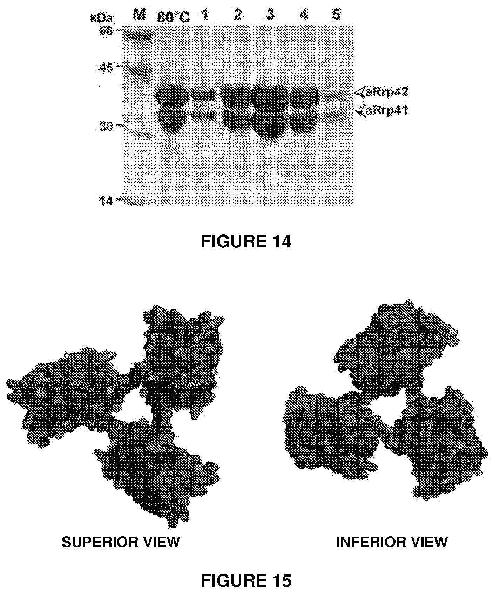

Figure 13 shows a cloning strategy for obtaining the vector according to the second aspect of the present invention, which contains Rrp41 and Rrp42 proteins of P. abyssi, and it is herein named pMRKA-RING, that comprises the sequence SEQ ID No: 37. Figure 14 shows the result of Rrp41 and Rrp42 proteins co-purification, expressed in E. coli transformed with pMRKA-RING plasmid of the present invention.

### pSUMAC vector

During the individual characterization of exosome proteins of P. abyssi, it was verified that Rrp42 was more soluble and thermostable when compared with Rrp4 and Rrp41 proteins. The analysis of this protein by gel-filtration shows a spin radius corresponding to a trimeric structure, as can be viewed on structural model in Figure 15.

In the same way as the commercial vectors, such as MBP, GST and SUMO, for the fusion of recombinant proteins, these characteristics of Rrp42 protein (high expression, solubility, thermostability and multimerization) are extremely helpful when producing a protein, e.g., fusion immunogenic protein for increase the recombinant proteins solubility.

Rrp42 protein was tested by means of BepiPred 1.0 program (www.cbs.dtu.dk/services/BepiPred), during the development of recombinant vaccines, it has been proved that its greater immunogenicity compared with other molecules used as peptides and proteins carrier, such as ovalbumin, BSA, chloramphenicol acetyltransferase (CAT), among others. In other words, besides facilitating purification of this type of fusions, Rrp42 provides a greater immunogenicity than the recombinant protein carrier commercially available.

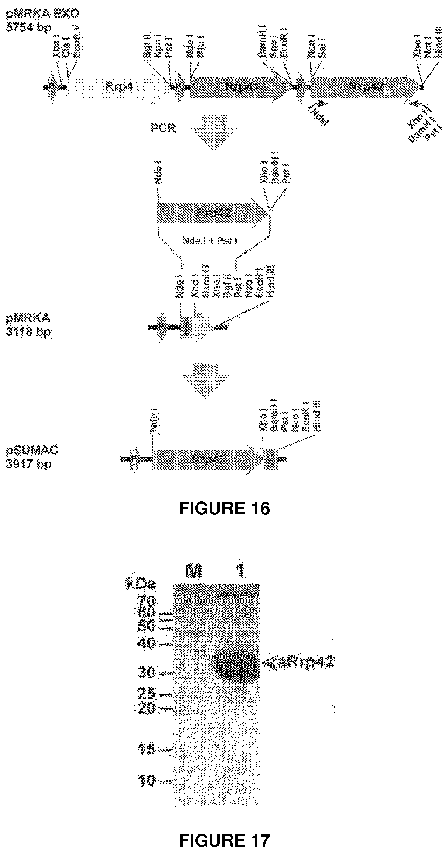

Thus, according to an aspect of the invention, it was constructed a vector for in-fusion expression with Rrp42 protein. Figure 16 shows the cloning strategy used to reach this purpose.

The sequence corresponding to Rrp42 protein was amplified by PCR from pMRKA-EXO plasmid, using the following nucleotides:
P42-For - SEQ ID No: 38
   Nde I
      ACCATATGGGTTCTGATAATGAAATCGTG
P42-Rev (SEQ ID No: 39)
   Pstl BamHI XhoI
      AACTGCAGGGATCCCTCGAGACCACCCTGTTTGGCCTTCTCAACAGC

The sequences for cutting sites for Ndol, Pstl, BamHI and Xhol restriction enzymes were underlined. The amplified DNA fragment was digested with Ndeol and Pstl enzymes and clones within the corresponding sites in pMRKA vector, as shown in Figure 16. Thereby, it was obtained, according to the second aspect of the invention, pSUMAC vector for expressing recombinant proteins in fusion with Rrp42 protein, such vector comprises SEQ ID No: 40.

Figure 17 shows the result of thermal lysis of E. coli cells transformed with pSUMAC plasmid.

As can be verified from the previous description, pMRKA and a T7 expression vector of E. coli, with high number of copies per cell is highly stable in fermenting conditions with high cellular density.

This vector, which corresponds to the first aspect of the invention, can be used for expressing recombinant proteins with or without N-terminal fusion of 6xHis tail. Additionally, according to the second aspect of the invention, pMRKA-EXO, pMRKA-RING and pSUMAC vectors are vectors derived from pMRKA for expressing proteins of C- or N-terminal fusion with exosome components of *Pyrococcus abyssi.*

Briefly, pMRKA-EXO vector comprises pMRKA vector and Rrp4-Rrp41-Rp42 protein encoding gene sequences of Pyrococcus abyssi exosome; pMRKA-RING vector comprises pMRKA vector and Rrp41-Rp42 protein encoding gene sequences of *Pyrococcus abyssi* exosome, and pSUMAC vector comprises pMRKA vector and Rp42 protein encoding gene sequence of Pyrococcus abyssi exosome.

According to the second aspect of the invention, pMRKA-EXO and pMRKA-RING could be used for co-expressing and purification several antigens at the same time, fused to the exosome components, intending to form pathogens like particles.

Still according to the second aspect of the invention, pSUMAC plasmid could be used as peptide vaccines carrier, providing greater solubility and thermostability to such vaccines and facilitating purification by thermal denaturing of host proteins (E. coli). Such characteristic is unique among fusion and carrier proteins currently used, facilitating the production of recombinant proteins in industrial scale with low cost.

Additionally, fuse plasmids with archae exosome proteins, i.e., pMRKA-EXO, pMRKA-RING and pSUMAC, provide greater stability for the produced recombinant proteins, both in formulation and in storing the completed product containing them.

According to the third aspect of the invention, there are provided pMRKA plasmids derivatives that besides comprising fusion of such plasmid to P. abyssi carrier proteins, they also include at least one sequence with immunomodulatory activity, particularly at least one Z domain sequence, which provides to the resulting polypeptides complexes, characteristics of immunomodulation of antigenic activity of said complexes. Z domains is a derivative from the A protein immunoglobulins binding domain of Staphylococcus aureus (Ljungberg, et al. (1993) "The interaction between different domains of staphylococcal protein A and human polyclonal IgG, IgA, IgM and F(ab)2: separation of affinity from specificity". Mal. Immunol. 30:1279- 1285). This domain has the ability to stimulate an immune response (Bekeredjian-Ding et al. (2007) "Staphylococcus aureus Protein A Triggers T Cell-Independent B Cell Proliferation by Sensitizing B Cells for TLR2 Ligands". J Immunol.178:2803- 2812), such characteristic can be used for driving the interaction of chimeric antigens with cells presenting antigens, in order to increase vaccines immune response. Recently, Miyata et al. (Miyata et al., 2011, "Tricomponent immunopotentiating system as a novel molecular design strategy for malaria vaccine development". Infect Immun. 79:4260-75) showed that the efficacy of the complex containing a malaria antigen, Z domain and a polymerization sequence, wherein Z domain has a fundamental role for inducing a immunoprotective response.

Miyata et al. (2011) report the difficulty of processing the obtained complex due to the inclusive corpuscle formation that require denaturation and refolding to obtain a soluble complex. Contrary to the results obtained by these researchers, the complexes obtained in the present invention are soluble and can be easily purified.

Besides Z domain, there are other immunogenic domains that could be used in the present invention to provide immunomodulatory activity to the polyproteins complexes from the vectors of the present invention.

As follows, there are provided examples that illustrate and represent specific embodiments of the invention. The following examples should not be understood as limiting the present invention.

### EXAMPLES

### Example 1:

### - Preparing the inoculum:

Unfreezing the aliquot of producer strain and inoculation in LB-kanamycin medium.

Subsequently, the mixture is cultured and stirred in Shaker, in 1L erlenmeyer with 200 ml of LB-KAN (25 µg/ml). Culture at 37°C, 200 rpm for 14-16 hours (DO₆₀₀ₙₘ from 1 to 2).

### - Fermentation:

The obtained culture in the previous step is transferred to a vat filled with 5 L of the medium complex high density (MCHD). After reaching DO₆₀₀ₙₘ of 20, it is started the induction for feeding with Lactose, until complete for 22 hours of culture.

### - Cell collection:

Cells are collected by centrifugation in 6000 rpm for 30 minutes, in temperature of 8°C in 1 L bottles.

### - Cell lysis:

The cells are resuspended in buffer (5 ml per gram of pellet): 30 mM Tris-HCl pH 9,0; 0,1% Triton X-100;; 5mM 2-mercaptoethanol.

Afterwards, the cells are treated for 1 hour at 90°C, in hot water bath, homogenizing after each ten minutes in Turrax homogenizer (5000 rpm). - Lysate clarification:

The lysed and homogenized cells are subjected to filtration through a 0.2 µm membrane in room temperature. - Treatment with benzonase enzyme:

The filtered material is treated with benzonase enzyme for 8 hours and 37°C temperature in order to eliminate contaminating nucleic acids.

### - 100 kDa membrane filtering

The withdrawal of digested nucleic acid and benzonase debris and final conditioning of the recombinant protein in the storing buffer made through diafiltration in the tangential flow filtering system, through a 100 kDa hollow membrane.

### - Sterile filtration through a 0.2 µm membrane

At last, it is executed a filtration in sterile environment through a 0.2 µm membrane. The produced batch is stored in sterile form until its use at 4°C.

The fermentation process described in this Example 1 corresponds to a general methodology that could be used on preparing and purifying polyproteins and polyprotein complexes obtained from vectors of the present invention.

### Example 2: Fusion of Z-Z domains from A protein of Staphylococcus aureus in Pyrococcus exosome Rrp42 protein

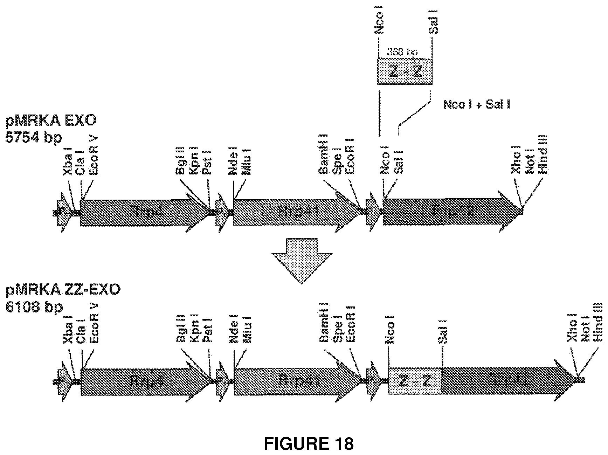

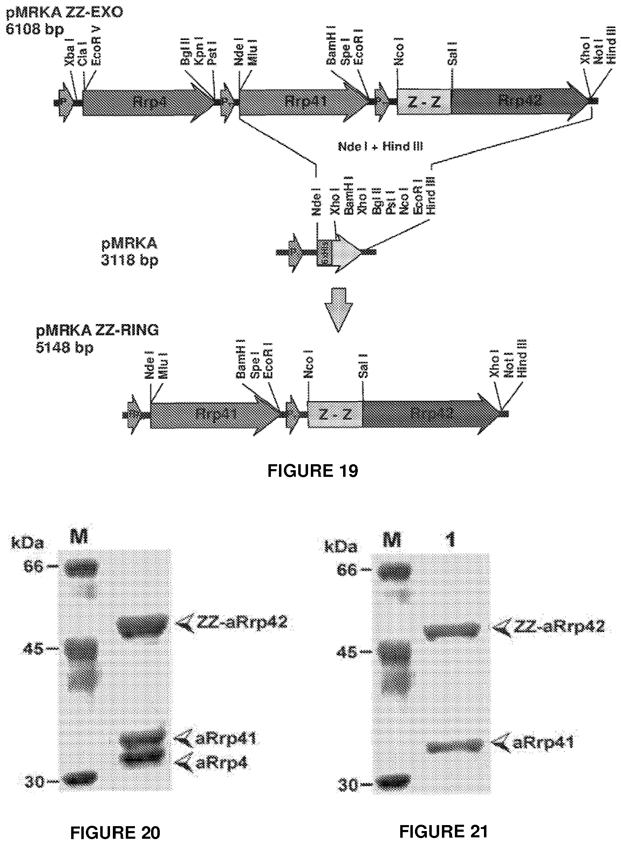

The immunomodulatory activity of Z domain together with exosome protein complex, that in the present invention is used as antigen carrier, was verified by means of a gene synthesis (SEQ ID No: 41), that encodes an amino acid sequence through the expression, in tandem, of two Z-domain copies, herein named Z-Z domains (SEQ ID No: 42). The nucleotide sequence (SEQ ID N: 41) was in-fusion cloned in region corresponding to an Rrp42 protein N-terminal end, using Ncol and Sall sites in pMRKA-EXO and pMRKA-RING plasmids. Figures 18 and 19 present representative diagrams of this strategy, resulting in pMRKA-ZZ-EXO (SEQ ID No: 43) and pMRKA-ZZ-RING (SEQ ID No:45) ) plasmids, respectively, and consequently in Rrp42 protein expression with Z-Z domain fusion in its N-terminal end (SEQ ID No: 44).

To verify if the ZZ domains fusion interferes with thermostability and purification of the complexes related to Pyrococcus abyssi exosome, Rosetta (DE3) competent cells were transformed with pMRKA-ZZ-EXO and pMRKA-ZZ-RING plasmids. After the transformation, it was made a recombinant protein induction experiment. In the end of such induction, the cells were recovered, resuspended in 300 mM Tris-HCl buffer pH 8.0, and lysed by high pressure homogenization (105 kPa (1000 Bar)). The homogenized was incubated at 80°C for 30 minutes and cooled in ice for 5 minutes before centrifuging for 30 minutes at 15000xg. The clarified and thermally treated lysate was charged in a chromatographic column packed with Q-sepharose XL resin and balanced with lysis buffer (30 mM Tris-HCl pH 8.0). After column washing with the same buffer, the bind protein was eluted with NaCl gradient (0-500 mM) in the same buffer. Figures 20 and 21 show the purification result of ZZ-EXOSOME and ZZ-RING complexes, respectively.

The results shown in Figures 20 and 21 indicate that the fusion with Z-Z domains does not interfere either with the solubility or stability of whole exosome complexes and exosome RING (RING), and that it is possible to obtain them with high level of purity with only a single chromatographic step.

In each complex, there are three copies of Rrp42 protein (see Figure 8 and Figure 12), therefore, there are also three copies of Z-Z domain, resulting in an amount of 6 Z-domains per complex, which could facilitate the interaction of these complexes with antigens presenting cells. Further, C-terminal ends are free of exosome proteins to fuse antigens, what represents greater ability than the complex described in Miyata et al. Therefore, pMRKA-ZZ-EXO (SEQ ID No: 43) and pMRKA- ZZ-RING (SEQ ID No: 45) plasmids could also be used to charge antigens in part of the exosome and from the exosome core ring, respectively, together with Z-Z domains.

### Example 3: Obtaining protein complexes containing Mycoplasma hyopneumiae antigens

Mycoplasma hyopneumoniae is one of the main pathogenic agents that affects pig breeding. Various vaccine candidates have already been identified, however, none of them, separately, can induce protection against disease in the same level as the commercial bacteria. Aiming to use the exosome complex to charge several antigens with Mycoplasma hypneumoniae, three chimeric proteins encoding genes were synthetized: 36-MHP271; P46-P97R1 R2 and HSP70-NrdF, corresponding to vaccine antigen pairs. The synthetic genes were clone in pMRKA-EXO vector by BgIII-KpnI; BamHI-EcoRI and XhoI-HindIII sites, respectively.

The resulting pMRKA-EXOMYC plasmid can express the following polyproteins:
- Rrp4- P36 - MHP271 (80,5 kDa)
- Rrp41 - P46 - P97R1 R2 (82,7 kDa)
- Rrp42 - HSP - NrdF (72,6 kDa).

In similar way, P46-P97R1 R2 and HSP70-NrdF chimeric proteins genes were cloned in pMRKA-RING vector by BamHI-EcoRI and XhoI-HindIII sites, respectively, obtaining pMRKA-RINGMYC plasmid which express the polyproteins:
Rrp41 - P46 - P97R1 R2 (82,7 kDa)
Rrp42 - HSP - NrdF (72,6 kDa).

At last the HSP70-NrdF chimeric protein gene was cloned in pSUMAC vector by XhoI-HindIII sites, obtaining pSUMAC-MYC plasmid which express the polyprotein:
( a ) Rrp42 - HSP - NrdF (72,6 kDa).

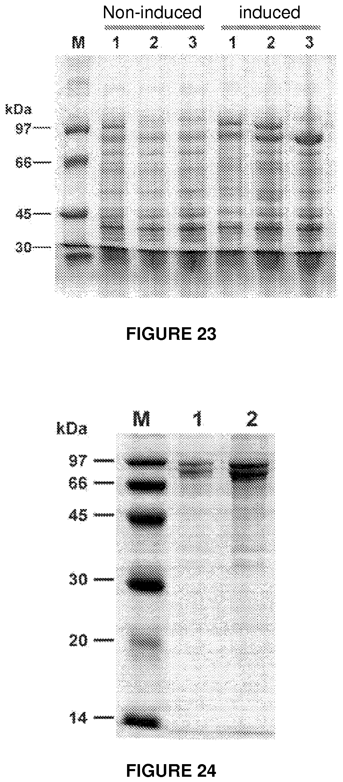

The Rosetta (DE3) competent cells were transformed with pMRKA-EXOMYC, pMRKA-RINGMYC, pSUMAC-MYC plasmids. After transformation, an induction experiment was made to visualize the induced ribbon in each transforming strain. Figure 23 shows the result of this experiment.

The result in Figure 23 confirms polyprotein expression through vectors of the present invention, including co-expression of 3 polyproteins in one single vector, a fact without precedent in the prior art. The induced ribbon that migrate among markers 66 and 97 corresponds to the Rrp42-P216A

HSP-NrdF protein (expressed by pMRK-Rrp42-EXO plasmids compared with strip 3 in Figure 23). It is also possible to conclude, by the comparison of pMRK-EXOMYC (strip 1 in Figure 23) and by pMRK-RING-MYC (strip 2 in Figure 23) induced proteins, that there is a migration overlapping in ribbons corresponding to Rrp4-P216B-P36-MHP271 (80,5 kDa) and Rrp41-P46-P97R1 R2 (82,7 kDa) polyproteins into SOS-PAGE gel.

### Example 4: Purification of protein complexes containing Mycoplasma hyopneumiae antigens

BL21 *Escherichia coli* (DE3) strain transformed with pMRK-EXOMYC plasmid was added in 200 ml of LB medium containing kanamycin (25 µg/ml). The culture was incubated at 37°C, 200 rpm for 16 hours.

The 200 ml culture was inoculated in 5L of MCHD (Medium Complex High Density) in 15 liter fermenter. The fermentation was made batch fed constant regimen, at 37°C, 30% dissolved oxygen, having glycerol as carbon source. In DO₆₀₀ₙₘ = 15, the feeding by glycerol was changed with lactose (induction) and the fermentation continued for more 12 hours at 28°C.

The cells were collected by 6000 rpm centrifuging for 30 minutes and resuspended in lyse buffer (50 mM Tris-HCl, pH 8.0; 0.1 % Triton X- 100; 0.25% Sarcozyl) and lysed in high pressure homogenizer.

The lysed and homogenized cells were subjected to filtering through a 0.2 µm membrane (end-filtration Millipore system) at room temperature.

MgCl₂ was added up to a 5 mM final concentration and 90 µm Benzonase (final concentration), and the mixture was incubated at room temperature for 6 hours.

The material treated with Benzonase was concentrated and diafiltered in the tangential filtration system using a 500 kDa hollow fiber membrane of MWCO, in 20 mM Tris-HCl, pH 8,0; 100mM NaCl and 0.1mM EDTA buffer. The retained protein is sterilized by micro filtering with a 0.2 µm membrane and stored at 4°C. Figure 24 shows the result of polyproteins co-purification which composes a Exomyc complex. The antibody formation induction against each one of the antigens of *M*. *hyopneumoniae* included in the complex (data not shown) confirm the usefulness of P. abyssi exosome complex as great complexity antigen carrier.

### Example 5: Purification of Rrp42-inhibin fusion protein

The vaccination with inhibin is a promising technique in the field of animal fertilization, as well as for increasing egg production in poultry. In the prior art, a number of studies about this application can be found. This vaccine would prevent the use of hormone that are more difficult and expensive to produce and formulate (see Findlay et I. (1993) "Inhibin as a fecundity vaccine". Animal Reproduction Science. 33: 325-343.

The most used antigen for this type of vaccine is a peptide containing the first 29 amino acids of inhibin alpha subunit conjugated with carrier proteins to stimulate immune response. Nevertheless, the immune response is low. To improve the response, it is used great amounts of fusion antigen, reaching amounts of 1 to 5 mg per dose, and application of up to 5 doses (see Wang et al. (2009) "The long-term effect of active immunization against inhibin in goats". Theriogenology. 71: 318-22).

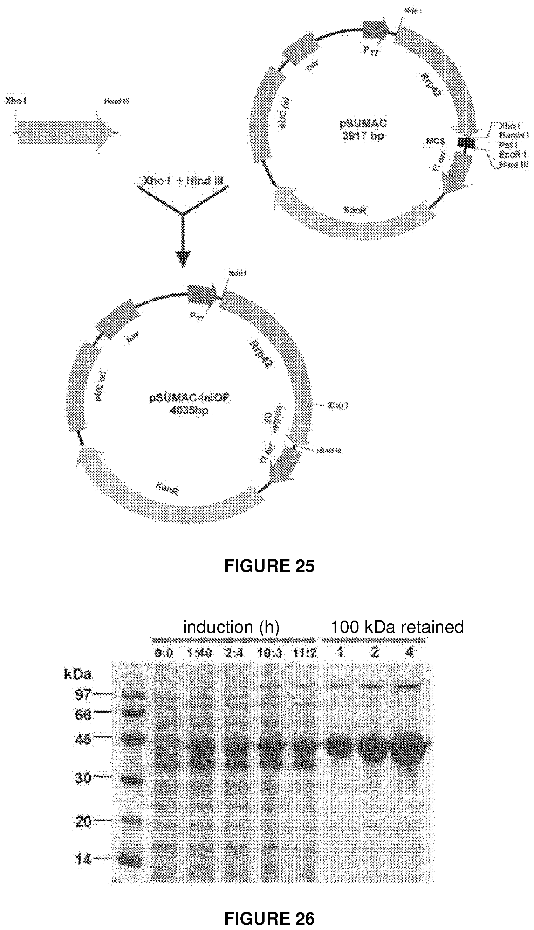

The present invention made possible to design and synthetize a gene which encodes a 49 amino acids proteins containing more alpha-inhibin immunogenic regions. This sequence was clones by XhoI and HindIII sites in pSUMAC vector, as shown in Figure 25. The protein containing more immunogenic regions of inhibin and its obtaining are detailed in co-pending patent application PI102014005376-0.

BL21 Escherichia coli (DE3) strain transformed with pSUMAC-IniOF plasmid was added in 200 ml of LB medium containing kanamycin (25 µg/ml). The culture was incubated at 37°C, 200 rpm for 16 hours. The culture was used to inoculate 5 L of MCHD (Medium Complex High Density) for executing the fermentation in the same way as in the previous example.

After the fermentation, the cells were collected by 6000 rpm centrifuging for 30 minutes, at 8°C in 1 liter bottles and resuspended in 30mM Tris-HCl pH 8.0; 0.1% Triton X-100 buffer. The cells were incubated for 1 hour at 90°C, in hot water bath, homogenizing after each ten minutes in Turrax homogenizer (5000 rpm).

The thermally lysed cells were subjected to filtration through a 0.2 µm membrane at room temperature, to clarify the lysate. The filtered material is treated with benzonase enzyme for 8 hours and 37°C temperature in order to eliminate contaminating nucleic acids. The withdrawal of digested nucleic acid and benzonase debris for final conditioning of the recombinant protein in the storing buffer was made through diafiltration in the tangential flow filtering system, through a 100 kDa hollow membrane.

Figure 26 shows the result of the fermentation and final purified product using only tangential filtration.

The results shown indicate that fusion with Pyrococcus abyssi exosome proteins, despite its complexity, results in proteins soluble in Escherichia coli cytoplasm. In some cases, these fusion proteins are also thermo resistant, which can be used for purifying recombinant proteins in a simple manner, by thermal treatment for removing contaminant proteins arising from E. coli, a microorganism that is mesothermal.

## Claims

1. *Escherichia coli* T7 expression vector **characterized by**: (a) having a high number of copies per cell; (b) being highly stable in high density cellular fermentation conditions; (c) sized about 2 Kpb to 4kpb; (d) having an antibiotic resistant marker.

2. Vector of claim 1, **characterized by** having 300 to 500 copies per cell.

3. Vector of claim 1, **characterized in that** the stability in high density cellular fermentation conditions is provided by the presence of a *par* sequence of pSC101 plasmid.

4. Vector of claim 1, **characterized in that** the *par* sequence comprises SEQ ID No: 11.

5. Vector of claim 1, **characterized in that** said antibiotic resistant marker is the kanamycin resistant gene.

6. Vector of any previous claims, **characterized in that** pMRKA plasmid comprises an nucleotide sequence SEQ ID No: 19.

7. Vector for polypeptides expression of fusion to carrier proteins, **characterized in that** it comprises: (a) a vector according to any of claims 1 to 6; and (b) at least one P. abyssi exosome gene encoding at least one immunogen proteins, antigens or immunoregulatory molecules-carrier protein.

8. Vector of claim 7, **characterized in that** it comprises P. abyssi exosome gene encoding at least one of Rrp4, Rrp41 and Rrp42 proteins.

9. Vector of claim 8, **characterized in that** it comprises P. abyssi exosome genes encoding at least two of the Rrp4, Rrp41 and Rrp42 proteins.

10. Vector of claim 8, **characterized in that** it comprises P. abyssi exosome genes encoding three of the Rrp4, Rrp41 and Rrp42 proteins.

11. Vector of claim 8, **characterized in that** the vector is pMRKA-EXO and it comprises the nucleotide sequence SEQ ID No: 36.

12. Vector of claim 9, **characterized in that** it comprises P. abyssi exosome genes encoding Rrp41 and Rrp42 proteins.

13. Vector of claim 10, **characterized in that** the vector is pMRKA-RING plasmid and it comprises the nucleotide sequence SEQ ID No: 37.

14. Vector of claim 8, **characterized in that** it comprises P. abyssi exosome genes encoding Rrp42 protein.

15. Vector of claim 14, **characterized in that** the vector is pSUMAC plasmid and it comprises the nucleotide sequence SEQ ID No: 40.

16. Vector of any of the preceding claims, **characterized in that** additionally comprises at least one immunoregulatory gene sequence.

17. Vector of claim 16, **characterized in that** said at least one immunoregulatory gene sequence is the Z domain sequence from A. protein of S. aureus.

18. Vector of claim 17, **characterized in that** said Z domain sequence from A protein of S. aureus is a Z-Z nucleotide sequence that comprises the SEQ ID No: 41 encoding protein of SEQ ID No: 42.

19. Vector of claim 18, **characterized in that** said Z-Z nucleotide sequence comprises the SEQ ID No: 41 and it is in-fusion cloned in the encoding region of N-terminal end of said Rrp42 composed in pMRKA-EXO plasmid, resulting in pMRKA-ZZ-EXO vector that comprises the nucleotide sequence SEQ ID No: 43.

20. Vector of claim 18, **characterized in that** said Z-Z nucleotide sequence comprises the SEQ ID No: 41 and it is in-fusion cloned in the encoding region of N-terminal end of said Rrp42 comprised in pMRKA-RING plasmid, resulting in pMRKA-ZZ-RING vector that comprises the nucleotide sequence SEQ ID No: 45.

21. Method for obtaining an expression vector as defined in claims 1 to 6, **characterized in that** it comprises the steps: (i) exchange the ampicillin resistant gene in vector of SEQ ID NO: 1 with the kanamycin resistant gene; (ii) amplification of the plasmid fragment of SEQ ID NO: 1 corresponding to the segment located among nucleotides 804 to 1857; (iii) binding the amplified segments from step (ii) with the kanamycin resistant gene; (iv) amplification of the plasmid obtained in step (iii) to insert restriction sites in base 2607 of pMK plasmid; (v) amplification of *par* sequence and insertion of the amplified sequence resulting among the restriction sites located at the base 2607 from pMK for obtaining pMRK plasmid; and (vi) eliminating the restriction sites of BgIII and Ncol in the kanamycin resistant gene in pMRK vector for obtaining pMRKA plasmid.

22. Method of claim 21, **characterized in that** said resistant gene exchange of step (i) is made by amplifying the segment obtained from primers of SEQ ID No: 2 and SEQ ID No: 3, and the amplified segment comprises SEQ ID No: 4.

23. Method of claim 21, **characterized in that** said amplification step (ii) is executed from primers of SEQ ID No: 6 and SEQ ID No: 7.

24. Method of claim 21, **characterized in that** said binding step (iii) results in pMK plasmid that comprises SEQ ID No: 8.

25. Method of claim 21, **characterized in that** said *par* sequence amplification of step (iv) is executed from primers of SEQ ID No: 9 and SEQ ID No: 10, resulting in the amplified sequence SEQ ID No: 11.

26. Method according to any of the claims 21 to 25, **characterized in that** said pMRK plasmid is obtained by amplifying pMK plasmid, from the primers SEQ ID No: 12 and SEQ ID No: 13, and insertion into SEQ ID No: 11, resulting in the plasmid of SEQ ID No: 14.

27. Method of claim 21, **characterized in that** said elimination of BgIII and Ncol restriction sites in kanamycin resistant gene of step (vi) is executed by site-directed mutagenesis, and the elimination of said BgIII restriction site is executed using primers of SEQ ID No: 15 and SEQ ID No: 16, and the elimination of said Ncol restriction site is executed using primers of SEQ ID No: 17 and SEQ ID No: 18.

28. Method, according to any of claims 21 to 27, **characterized in that** said pMRKA plasmid comprises the SEQ ID No: 19.

29. Method for obtaining an expression vector as defined in claims 7 to 15, **characterized in that** it comprises the steps:
(i) preparing pMRKA plasmid according to the method defined in claims 21 to 28; (ii) preparing at least on gene sequence of P. abyssi exosome encoding at least one protein carrying immunogenic proteins, antigens or immunoregulatory molecules; (iii) synthesis of one double-stranded DNA containing said at least one gene sequence of P. abyssi exosome and (iv) cloning said double-stranded DNA obtained in step (iii) into the said pMRKA plasmid.

30. Method of claim 29, **characterized in that** said cloning in step (iv) is executed between Xbal and Hindlll sites of pMRKA plasmid.

31. Method, according to any of the claims 29 and 30, **characterized in that** said at least one P. abyssi exosome gene sequence of step (ii) comprises gene sequences encoding Rrp4, Rrp41 and Rrp42 proteins of P. abyssi exosome.

32. Method of claim 31, **characterized in that** there are additionally provided sequences SEQ ID No: 22, SEQ ID No: 23, SEQ ID No: 24 and SEQ ID No: 25 to allow in-fusion cloning of said gene sequences encoding Rrp4, Rrp41 and Rrp42 proteins of P. abyssi exosome.

33. Method of claim 31, **characterized in that** said gene sequences comprises SEQ ID No: 26, SEQ ID No: 28 and SEQ ID No: 30 encoding Rrp4, Rrp41 and Rrp42 proteins, respectively, of P. abyssi exosome.

34. Method, according to any of claims 29 to 32, **characterized in that** P. abyssi exosome gene sequences are gathered in the order SEQ ID No: 22-SEQ ID No: 26-SEQ ID No: 23- SEQ ID No: 28-SEQ ID No: 24-SEQ ID No: 30-SEQ ID No: 25.

35. Method of claim 34, **characterized in that** the gathering of said gene sequences forms SEQ ID No: 32.

36. Method of claim 35, **characterized in that** it is additionally synthetized a double-stranded DNA comprising SEQ ID No: 32 that is cloned in pMRKA plasmid, between Xbal and Hindlll plasmid, to form SEQ ID No: 36 comprised in pMRKA-EXO plasmid.

37. Method, according to any of the claims 29 and 30, **characterized in that** said at least one P. abyssi exosome gene sequence of step (ii) comprises gene sequences encoding Rrp41 and Rrp42 proteins of P. abyssi exosome.

38. Method of claim 37, **characterized in that** said gene sequences comprises the sequences SEQ ID No: 28 and SEQ ID No: 30 respectively encoding Rrp41 and Rrp42 proteins of P. abyssi exosome.

39. Method of claim 38, **characterized in that** said P. abyssi exosome gene sequences were amplified from pMRKA-EXO plasmid to form SEQ ID No: 37 comprised in pMRKA-RING plasmid.

40. Method, according to any of the claims 29 and 30, **characterized in that** said at least one P. abyssi exosome gene sequence of step (ii) comprises gene sequences encoding Rrp42 protein of P. abyssi exosome.

41. Method of claim 40, **characterized in that** said sequence encoding Rrp42 protein of P. abyssi exosome was amplified from pMRKA-EXO plasmid, using oligonucleotides with SEQ ID No: 38 and SEQ ID No: 39

42. Method of claim 41, **characterized in that** the DNA fragment resulting from the amplification of sequences SEQ ID No: 38 and SEQ ID No: 39 was cloned in pMRKA vector to form SEQ ID No: 40 comprised in pSUMAC plasmid.

43. Method, according to any of claims 21 to 42, **characterized in that** it additionally includes in pMRKA-EXO and pMRKA-RING plasmids at least on immunoregulatory gene sequence.

44. Method of claim 43, **characterized in that** said at least one immunoregulatory gene sequence is the Z domain sequence from A protein of S. aureus for tandem expression of two Z domain, Z-Z domains copies, that is in-fusion cloned with the region encoding N-terminal end of Rrp42 protein comprised in pMRKA-EXO and pMRKA-RING vectors

45. Method of claim 44, **characterized in that** said gene sequence encoding Z-Z domains is in-fusion cloned with the region encoding the N-terminal end of Rrp42 protein among Ncol and Sail sites in pMRKA-EXO e pMRKA-RING plasmids.

46. Method of claim 45, **characterized in that** pMRKA-ZZ-EXO vector is produced and it comprises SEQ ID No: 43.

47. Method of claim 45, **characterized in that** pMRKA-ZZ-RING vector is produced and it comprises the SEQ ID No: 45

48. Usage of the expression vectors defined in claims 1 to 6, **characterized by** being in expression of recombinant proteins, antigens and immunogenic complexes.

49. Usage of claim 48, **characterized in that** the vector is the pMRKA plasmid.

50. Usage of the expression vectors defined in claims 7 to 15, **characterized by** being in expression of recombinant proteins, antigens and immunogenic complexes fused to the carrier proteins.

51. Usage of claim 50, **characterized in that** said carrier proteins are P. abyssi exosome proteins, preferably Rrp4, Rrp41 and Rrp42 proteins..

52. Usage of claim 51, **characterized in that** the vector is the plasmid selected from pMRKA-EXO and pMRKA-RING, said vector being especially usable in the expression and co-purification of recombinant proteins to form pathogen like particles.

53. Usage of claim 50, **characterized in that** the vector is the pSUMAC plasmid that is especially usable in obtaining carrier proteins of peptide vaccines.

54. Usage of the expression vectors defined in claims 16 to 20, **characterized by** being in expression of recombinant proteins, antigens and immunogenic complexes fused to the carrier proteins and additionally having immunomodulatory activity.


SEQUENCE LISTING

<110>    OUROFINO SAÚDE ANIMAL LTDA

<120>    \223Escherichia coli T7 expression vector, vectors for the
co-expression and co-purification of recombinant polypeptides
        in/with carrier proteins, Use of expression vectors for obtaining
complexes with multiples antigens and immunomodulatos

<130>    EXOSOME VECTORS

<150>    BR 10 2014 014502 8
<151>    2014-06-13

<160>    40

<170>    PatentIn versão 3.5

<210>    SEQ ID No: 1
<211>    2822
<212>    DNA
<213>    Artificial sequence

<220>
<223>    Sequence of T7 expression pAE vector

<220>
<221>    primer_binding
<222>    (825) ... (843)
<223>    primer binding to VecMar-Rev

<220>
<221>    primer_binding
<222>    (1818) ... (1837)
<223>    primer binding to VecMar-For

<300>
<301>    Ramos CR, Abreu PA, Nascimento AL, Ho PL
<302>    A high-copy T7 Escherichia coli expression vector for the
production of recombinant proteins with a minimal N-terminal His-tagged
fusion peptide
<303>    Braz J Med Biol Res.
<304>    37
<305>    8
<306>    1103-1109
<307>    08/2004
<313>    (1)..(2822)

<400>    1

gatctcgatc ccgcgaaatt aatacgactc actataggga gaccacaacg gtttccctct      60

agaaataatt ttgtttaact ttaagaagga gatatacata tgcatcacca tcaccatcac     120

ctcgagggat ccgacctcga gatctgcagc tggtaccatg gaattcgaag cttgatccgg     180

ctgctaacaa agcccgaaag gaagctgagt tggctgctgc caccgctgag caataactag     240

cataacccct tggggcctct aaacgggtct tgaggggttt tttgctgaaa ggaggaacta     300

tatccggatc tggcgtaata gcgaagaggc ccgcaccgat cgcccttccc aacagttgcg     360

cagcctgaat ggcgaatggg acgcgccctg tagcggcgca ttaagcgcgg cgggtgtggt     420

ggttacgcgc agcgtgaccg ctacacttgc cagcgcccta gcgcccgctc ctttcgcttt 480

cttcccttcc tttctcgcca cgttcgccgg ctttccccgt caagctctaa atcgggggct 540

ccctttaggg ttccgattta gtgctttacg gcacctcgac cccaaaaaac ttgattaggg 600

tgatggttca cgtagtgggc catcgccctg atagacggtt tttcgccctt tgacgttgga 660

gtccacgttc tttaatagtg gactcttgtt ccaaactgga acaacactca accctatctc 720

ggtctattct tttgatttat aagggatttt gccgatttcg gcctattggt taaaaaatga 780

gctgatttaa caaaaattta acgcgaattt taacaaaata ttaacgctta caatttaggt 840

ggcacttttc ggggaaatgt gcgcggaacc cctatttgtt tatttttcta aatacattca 900

aatatgtatc cgctcatgag acaataaccc tgataaatgc ttcaataata ttgaaaaagg 960

aagagtatga gtattcaaca tttccgtgtc gcccttattc ccttttttgc ggcattttgc 1020

cttcctgttt ttgctcaccc agaaacgctg gtgaaagtaa aagatgctga agatcagttg 1080

ggtgcacgag tgggttacat cgaactggat ctcaacagcg gtaagatcct tgagagtttt 1140

cgccccgaag aacgttttcc aatgatgagc acttttaaag ttctgctatg tggcgcggta 1200

ttatcccgta ttgacgccgg gcaagagcaa ctcggtcgcc gcatacacta ttctcagaat 1260

gacttggttg agtactcacc agtcacagaa aagcatctta cggatggcat gacagtaaga 1320

gaattatgca gtgctgccat aaccatgagt gataacactg cggccaactt acttctgaca 1380

acgatcggag gaccgaagga gctaaccgct tttttgcaca acatggggga tcatgtaact 1440

cgccttgatc gttgggaacc ggagctgaat gaagccatac caaacgacga gcgtgacacc 1500

acgatgcctg tagcaatggc aacaacgttg cgcaaactat taactggcga actacttact 1560

ctagcttccc ggcaacaatt aatagactgg atggaggcgg ataaagttgc aggaccactt 1620

ctgcgctcgg cccttccggc tggctggttt attgctgata aatctggagc cggtgagcgt 1680

gggtctcgcg gtatcattgc agcactgggg ccagatggta agccctcccg tatcgtagtt 1740

atctacacga cggggagtca ggcaactatg gatgaacgaa atagacagat cgctgagata 1800

ggtgcctcac tgattaagca ttggtaactg tcagaccaag tttactcata tatactttag 1860

attgatttaa aacttcattt ttaatttaaa aggatctagg tgaagatcct ttttgataat 1920

ctcatgacca aaatccctta acgtgagttt tcgttccact gagcgtcaga ccccgtagaa 1980

aagatcaaag gatcttcttg agatcctttt tttctgcgcg taatctgctg cttgcaaaca 2040

aaaaaaccac cgctaccagc ggtggtttgt ttgccggatc aagagctacc aactcttttt 2100

ccgaaggtaa ctggcttcag cagagcgcag ataccaaata ctgttcttct agtgtagccg 2160

tagttaggcc accacttcaa gaactctgta gcaccgccta catacctcgc tctgctaatc 2220

ctgttaccag tggctgctgc cagtggcgat aagtcgtgtc ttaccgggtt ggactcaaga 2280

cgatagttac cggataaggc gcagcggtcg ggctgaacgg ggggttcgtg cacacagccc 2340

agcttggagc gaacgaccta caccgaactg agatacctac agcgtgagct atgagaaagc 2400

gccacgcttc ccgaagggag aaaggcggac aggtatccgg taagcggcag ggtcggaaca 2460

ggagagcgca cgagggagct tccaggggga aacgcctggt atctttatag tcctgtcggg 2520

tttcgccacc tctgacttga gcgtcgattt ttgtgatgct cgtcaggggg gcggagccta 2580

tggaaaaacg ccagcaacgc ggccttttta cggttcctgg ccttttgctg gccttttgct 2640

cacatgttct ttcctgcgtt atcccctgat tctgtggata accgtattac cgcctttgag 2700

tgagctgata ccgctcgccg cagccgaacg accgagcgca gcgagtcagt gagcgaggaa 2760

gcggaagagc gcccaatacg caaaccgcct ctccccgcgc gttggccgat tcattaatgc 2820

ag 2822

<210> 2
<211> 28
<212> DNA
<213> Artificial sequence

<220>
<223> Forward primer of kanamycin gene for PCR

<400> 2

atctcgagtt atggacagca agcgaacc 28

<210> 3
<211> 27
<212> DNA
<213> Artificial sequence

<220>
<223> Reverse primer of kanamycin gene for PCR

<40> 3

catctagaat ttcgaacccc agagtcc 27

<210> SEQ ID No: 4
<211> 990
<212> DNA
<213> Escherichia coli

<220>
<221> CDS
<222> (165) ... (959)

<400> 4
atctcgagtt atggacagca agcgaaccgg aattgccagc tggggcgccc tctggtaagg 60
ttgggaagcc ctgcaaagta aactggatgg ctttcttgcc gccaaggatc tgatggcgca 120
ggggatcaag atctgatcaa gagacaggat gaggatcgtt tcgc atg att gaa caa 176
gat gga ttg cac gca ggt tct ccg gcc gct tgg gtg gag agg cta ttc 224
ggc tat gac tgg gca caa cag aca atc ggc tgc tct gat gcc gcc gtg 272
ttc cgg ctg tca gcg cag ggg cgc ccg gtt ctt ttt gtc aag acc gac 320
ctg tcc ggt gcc ctg aat gaa ctc caa gac gag gca gcg cgg cta tcg 368
tgg ctg gcc acg acg ggc gtt cct tgc gca gct gtg ctc gac gtt gtc 416
act gaa gcg gga agg gac tgg ctg cta ttg ggc gaa gtg ccg ggg cag 464
gat ctc ctg tca tct cac ctt gct cct gcc gag aaa gta tcc atc atg 512
gct gat gca atg cgg cgg ctg cat acg ctt gat ccg gct acc tgc cca 560
ttc gac cac caa gcg aaa cat cgc atc gag cga gca cgt act cgg atg 608
gaa gcc ggt ctt gtc gat cag gat gat ctg gac gaa gag cat cag ggg 656
ctc gcg cca gcc gaa ctg ttc gcc agg ctc aag gcg cgg atg ccc gac 704
ggc gag gat ctc gtc gtg acc cat ggc gat gcc tgc ttg ccg aat atc 752
atg gtg gaa aat ggc cgc ttt tct gga ttc atc gac tgt ggc cgg ctg 800
ggt gtg gcg gac cgc tat cag gac ata gcg ttg gct acc cgt gat att 848
gct gaa gag ctt ggc ggc gaa tgg gct gac cgc ttc ctc gtg ctt tac 896
ggt atc gcc gct ccc gat tcg cag cgc atc gcc ttc tat cgc ctt ctt 944
gac gag ttc ttc tga gcgggactct ggggttcgaa atgtctagat g 990

<210> SEQ ID No: 5
<211> 264
<212> Protein
<213> Escherichia coli

<400> 5

<210>    SEQ ID No: 6
<211>    28
<212>    DNA
<213>    Artificial sequence

<220>
<223>    Foward primer for PCR amplification of pAE vector without the gene
AmpR

<400>    6

cgactagtgc attggtaact gtcagacc                                          28

<210>    SEQ ID No: 7
<211>    29
<212>    DNA
<213>    Artificial sequence

<220>
<223>   Reverse primer for PCR amplification of pAE vector without the gene
AmpR

<400> 7

atgtcgacgt gccacctaaa ttgtaagcg                                         29

<210>    SEQ ID No: 8
<211>    2835
<212>    DNA
<213>    Artificial sequence
<220>
<223>    pAE-derived vector with marker KanR instead of AmpR

<220>
<221>     primer_binding

<222>    (2590) ... (2609)
<223>    PCR VecPar-Rev primer binding

<220>
<221>    primer_binding
<222>    (2610) ... (2630)
<223>    PCR VecPar-For primer binding

<210>    8

gatctcgatc ccgcgaaatt aatacgactc actataggga gaccacaacg gtttccctct      60

agaaataatt ttgtttaact ttaagaagga gatatacata tgcatcacca tcaccatcac     120

ctcgagggat ccgacctcga gatctgcagc tggtaccatg gaattcgaag cttgatccgg     180

ctgctaacaa agcccgaaag gaagctgagt tggctgctgc caccgctgag caataactag     240

cataacccct tggggcctct aaacgggtct tgaggggttt tttgctgaaa ggaggaacta     300

tatccggatc tggcgtaata gcgaagaggc ccgcaccgat cgcccttccc aacagttgcg     360

cagcctgaat ggcgaatggg acgcgccctg tagcggcgca ttaagcgcgg cgggtgtggt     420

ggttacgcgc agcgtgaccg ctacacttgc cagcgcccta gcgcccgctc ctttcgcttt     480

cttcccttcc tttctcgcca cgttcgccgg ctttccccgt caagctctaa atcgggggct     540

ccctttaggg ttccgattta gtgctttacg gcacctcgac cccaaaaaac ttgattaggg     600

tgatggttca cgtagtgggc catcgccctg atagacggtt tttcgccctt tgacgttgga     660

gtccacgttc tttaatagtg gactcttgtt ccaaactgga acaacactca accctatctc     720

ggtctattct tttgatttat aagggatttt gccgatttcg gcctattggt taaaaaatga     780

gctgatttaa caaaaattta acgcgaattt taacaaaata ttaacgctta caatttaggt     840

ggcacgtcga gttatggaca gcaagcgaac cggaattgcc agctggggcg ccctctggta     900

aggttgggaa gccctgcaaa gtaaactgga tggctttctt gccgccaagg atctgatggc     960

gcaggggatc aagatctgat caagagacag gatgaggatc gtttcgcatg attgaacaag    1020

atggattgca cgcaggttct ccggccgctt gggtggagag gctattcggc tatgactggg    1080

cacaacagac aatcggctgc tctgatgccg ccgtgttccg gctgtcagcg caggggcgcc    1140

cggttctttt tgtcaagacc gacctgtccg gtgccctgaa tgaactccaa gacgaggcag    1200

cgcggctatc gtggctggcc acgacgggcg ttccttgcgc agctgtgctc gacgttgtca    1260

ctgaagcggg aagggactgg ctgctattgg gcgaagtgcc ggggcaggat ctcctgtcat    1320

ctcaccttgc tcctgccgag aaagtatcca tcatggctga tgcaatgcgg cggctgcata    1380

cgcttgatcc ggctacctgc ccattcgacc accaagcgaa acatcgcatc gagcgagcac    1440

gtactcggat ggaagccggt cttgtcgatc aggatgatct ggacgaagag catcaggggc    1500

tcgcgccagc cgaactgttc gccaggctca aggcgcggat gcccgacggc gaggatctcg    1560

tcgtgaccca tggcgatgcc tgcttgccga atatcatggt ggaaaatggc cgcttttctg    1620

gattcatcga ctgtggccgg ctgggtgtgg cggaccgcta tcaggacata gcgttggcta     1680

cccgtgatat tgctgaagag cttggcggcg aatgggctga ccgcttcctc gtgctttacg     1740

gtatcgccgc tcccgattcg cagcgcatcg ccttctatcg ccttcttgac gagttcttct     1800

gagcgggact ctggggttcg aaattctagt gcattggtaa ctgtcagacc aagtttactc     1860

atatatactt tagattgatt taaaacttca tttttaattt aaaaggatct aggtgaagat     1920

cctttttgat aatctcatga ccaaaatccc ttaacgtgag ttttcgttcc actgagcgtc     1980

agaccccgta gaaaagatca aaggatcttc ttgagatcct ttttttctgc gcgtaatctg     2040

ctgcttgcaa acaaaaaaac caccgctacc agcggtggtt tgtttgccgg atcaagagct     2100

accaactctt tttccgaagg taactggctt cagcagagcg cagataccaa atactgttct     2160

tctagtgtag ccgtagttag gccaccactt caagaactct gtagcaccgc ctacatacct     2220

cgctctgcta atcctgttac cagtggctgc tgccagtggc gataagtcgt gtcttaccgg     2280

gttggactca agacgatagt taccggataa ggcgcagcgg tcgggctgaa cggggggttc     2340

gtgcacacag cccagcttgg agcgaacgac ctacaccgaa ctgagatacc tacagcgtga     2400

gctatgagaa agcgccacgc ttcccgaagg gagaaaggcg gacaggtatc cggtaagcgg     2460

cagggtcgga acaggagagc gcacgaggga gcttccaggg ggaaacgcct ggtatcttta     2520

tagtcctgtc gggtttcgcc acctctgact tgagcgtcga tttttgtgat gctcgtcagg     2580

ggggcggagc ctatggaaaa acgccagcaa cgcggccttt ttacggttcc tggccttttg     2640

ctggcctttt gctcacatgt tctttcctgc gttatcccct gattctgtgg ataaccgtat     2700

taccgccttt gagtgagctg ataccgctcg ccgcagccga acgaccgagc gcagcgagtc     2760

agtgagcgag gaagcggaag agcgcccaat acgcaaaccg cctctccccg cgcgttggcc     2820

gattcattaa tgcag                                                      2835


<210>    SEQ ID No: 9
<211>    27
<212>    DNA
<213>    Artificial sequence

<220>
<223>    Foward primer for PCR amplification of par sequence of plasmid
pSC101

<400>    9

atctcgagtt tgtctccgac catcagg                                         27


<210>    SEQ ID No: 10
<211>    26
<212>    DNA

<213>    sequência artificial

<220>
<223>    Reverse primer for PCR amplification of par sequence of plasmid
pSC101

<400>    10

gttctagacg ggataatccg aagtgg                                          26



<210>    SEQ ID No: 11
<211>    287
<212>    DNA
<213>    Artificial sequence
<220>
<223>    Amplified par sequence of plasmid pSC101

<300>
<301>    Miller CA, Tucker WT, Meacock PA, Gustafsson P, Cohen SN
<302>    Nucleotide sequence of the partition locus of Escherichia coli
plasmid pSC101
<303>    Gene
<304>    24
<305>    2
<306>    309-315
<307>    10/1983
<313>    (7)..(281)

<300>
<301>    Miller CA, Tucker WT, Meacock PA, Gustafsson P, Cohen SN
<302>    Nucleotide sequence of the partition locus of Escherichia coli
plasmid pSC101
<303>    Genes and Development
<304>    24
<305>    2
<306>    309-315
<307>    10/1983
<313>    (7)..(281)

<400>    11

atctcgagtt tgtctccgac catcaggcac ctgagtcgct gtctttttcg tgacattcag      60

ttcgctgcgc tcacggctct ggcagtgaat gggggtaaat ggcactacag gcgcctttta     120

tggattcatg caaggaaact acccataata caagaaaagc ccgtcacggg cttctcaggg     180

cgttttatgg cgggtctgct atgtggtgct atctgacttt ttgctgttca gcagttcctg     240

ccctctgatt ttccagtctg accacttcgg attatcccgt ctagaac                   287



<210>    SEQ ID No: 12
<211>    29
<212>    DNA
<213>    Artificial sequence

<220>
<223>    Foward primer for PCR amplification of plasmid pMK for insertion of
par sequence

<400> 12

atactagtac gcggcctttt tacggttcc                                    29

<210>    SEQ ID No: 13
<211>    28
<212>    DNA
<213>    Artificial sequence

<220>
<223>   Reverse primer for PCR amplification of plasmid pMK for insertion of
par sequence

<400>    13

atgtcgactg ctggcgtttt tccatagg                                     28

<210>    SEQ ID No: 14
<211>    3118
<212>    DNA
<213>    Artificial sequence

<220>
<223>   pAE-derived vector with the marker KanR and the insert of par
sequence

<400>    14

gatctcgatc ccgcgaaatt aatacgactc actataggga gaccacaacg gtttccctct      60

agaaataatt ttgtttaact ttaagaagga gatatacata tgcatcacca tcaccatcac     120

ctcgagggat ccgacctcga gatctgcagc tggtaccatg gaattcgaag cttgatccgg     180

ctgctaacaa agcccgaaag gaagctgagt tggctgctgc caccgctgag caataactag     240

cataacccct tggggcctct aaacgggtct tgaggggttt tttgctgaaa ggaggaacta     300

tatccggatc tggcgtaata gcgaagaggc ccgcaccgat cgcccttccc aacagttgcg     360

cagcctgaat ggcgaatggg acgcgccctg tagcggcgca ttaagcgcgg cgggtgtggt     420

ggttacgcgc agcgtgaccg ctacacttgc cagcgcccta gcgcccgctc ctttcgcttt     480

cttcccttcc tttctcgcca cgttcgccgg ctttccccgt caagctctaa atcgggggct     540

ccctttaggg ttccgattta gtgctttacg gcacctcgac cccaaaaaac ttgattaggg     600

tgatggttca cgtagtgggc catcgccctg atagacggtt tttcgccctt tgacgttgga     660

gtccacgttc tttaatagtg gactcttgtt ccaaactgga acaacactca accctatctc     720

ggtctattct tttgatttat aagggatttt gccgatttcg gcctattggt taaaaaatga     780

gctgatttaa caaaaattta acgcgaattt taacaaaata ttaacgctta caatttaggt     840

ggcacgtcga gttatggaca gcaagcgaac cggaattgcc agctggggcg ccctctggta 900
aggttgggaa gccctgcaaa gtaaactgga tggctttctt gccgccaagg atctgatggc 960
gcaggggatc aagatctgat caagagacag gatgaggatc gtttcgcatg attgaacaag 1020
atggattgca cgcaggttct ccggccgctt gggtggagag gctattcggc tatgactggg 1080
cacaacagac aatcggctgc tctgatgccg ccgtgttccg gctgtcagcg caggggcgcc 1140
cggttctttt tgtcaagacc gacctgtccg gtgccctgaa tgaactccaa gacgaggcag 1200
cgcggctatc gtggctggcc acgacgggcg ttccttgcgc agctgtgctc gacgttgtca 1260
ctgaagcggg aagggactgg ctgctattgg gcgaagtgcc ggggcaggat ctcctgtcat 1320
ctcaccttgc tcctgccgag aaagtatcca tcatggctga tgcaatgcgg cggctgcata 1380
cgcttgatcc ggctacctgc ccattcgacc accaagcgaa acatcgcatc gagcgagcac 1440
gtactcggat ggaagccggt cttgtcgatc aggatgatct ggacgaagag catcaggggc 1500
tcgcgccagc cgaactgttc gccaggctca aggcgcggat gcccgacggc gaggatctcg 1560
tcgtgaccca tggcgatgcc tgcttgccga atatcatggt ggaaaatggc cgcttttctg 1620
gattcatcga ctgtggccgg ctgggtgtgg cggaccgcta tcaggacata gcgttggcta 1680
cccgtgatat tgctgaagag cttggcggcg aatgggctga ccgcttcctc gtgctttacg 1740
gtatcgccgc tcccgattcg cagcgcatcg ccttctatcg ccttcttgac gagttcttct 1800
gagcgggact ctggggttcg aaattctagt gcattggtaa ctgtcagacc aagtttactc 1860
atatatactt tagattgatt taaaacttca tttttaattt aaaaggatct aggtgaagat 1920
cctttttgat aatctcatga ccaaaatccc ttaacgtgag ttttcgttcc actgagcgtc 1980
agaccccgta gaaaagatca aaggatcttc ttgagatcct ttttttctgc gcgtaatctg 2040
ctgcttgcaa acaaaaaaac caccgctacc agcggtggtt tgtttgccgg atcaagagct 2100
accaactctt tttccgaagg taactggctt cagcagagcg cagataccaa atactgttct 2160
tctagtgtag ccgtagttag gccaccactt caagaactct gtagcaccgc ctacatacct 2220
cgctctgcta atcctgttac cagtggctgc tgccagtggc gataagtcgt gtcttaccgg 2280
gttggactca agacgatagt taccggataa ggcgcagcgg tcgggctgaa cggggggttc 2340
gtgcacacag cccagcttgg agcgaacgac ctacaccgaa ctgagatacc tacagcgtga 2400
gctatgagaa agcgccacgc ttcccgaagg gagaaaggcg gacaggtatc cggtaagcgg 2460
cagggtcgga acaggagagc gcacgaggga gcttccaggg ggaaacgcct ggtatcttta 2520
tagtcctgtc gggtttcgcc acctctgact tgagcgtcga tttttgtgat gctcgtcagg 2580
ggggcggagc ctatggaaaa acgccagcag tcgagtttgt ctccgaccat caggcacctg 2640
agtcgctgtc tttttcgtga cattcagttc gctgcgctca cggctctggc agtgaatggg 2700
ggtaaatggc actacaggcg ccttttatgg attcatgcaa ggaaactacc cataatacaa 2760

gaaaagcccg tcacgggctt ctcagggcgt tttatggcgg gtctgctatg tggtgctatc 2820

tgactttttg ctgttcagca gttcctgccc tctgattttc cagtctgacc acttcggatt 2880

atcccgtcta gtacgcggcc tttttacggt tcctggcctt ttgctggcct tttgctcaca 2940

tgttctttcc tgcgttatcc cctgattctg tggataaccg tattaccgcc tttgagtgag 3000

ctgataccgc tcgccgcagc cgaacgaccg agcgcagcga gtcagtgagc gaggaagcgg 3060

aagagcgccc aatacgcaaa ccgcctctcc ccgcgcgttg gccgattcat taatgcag 3118

<210> SEQ ID No: 15
<211> 42
<212> DNA
<213> Artificial sequence

<220>
<223> Foward oligonucleotide for C976T mutagenisis in the restriction site
BglII of KanR gene

<400> 15

gatggcgcag gggatcaaga tttgatcaag agacaggatg ag 42

<210> SEQ ID No: 16
<211> 42
<212> DNA
<213> Artificial sequence

<220>
<223> Reverse oligonucleotide for C976T mutagenisis in the restriction
site BglII of gene KanR

<400> 16

ctcatcctgt ctcttgatca aatcttgatc ccctgcgcca tc 42

<210> SEQ ID No: 17
<211> 33
<212> DNA
<213> Artificial sequence

<220>
<223> Foward oligonucleotide for T1571C mutagenisis in the restriction
site NcoI of gene KanR

<400> 17

atctcgtcgt gacccacggc gatgcctgct tgc 33

<210> SEQ ID No: 18

<211>    33
<212>    DNA
<213>    Artificial sequence

<220>
<223>   Reverse oligonucleotide for T1571C mutagenisis in the restriction
site NcoI of gene KanR

<400>    18

gcaagcaggc atcgccgtgg gtcacgacga gat                                    33

<210>    SEQ ID No: 19
<211>    3118
<212>    DNA
<213>    Artificial sequence

<220>
<223>   pMRK-derived vector with double mutation C976T and T1571C,
eliminating the restriction sites BglII e NcoI, respectfully, in the gene
KanR

<220>
<221>    promotor
<222>    (20) ... (39)
<223>    T7 promotor

<220>
<221>    CDS
<222>    (100) ... (240)
<223>    Fusio hexe histidine

<220>
<221>    misc_feature
<222>    (103) ... (110)
<223>    6xHis tag

<220>
<221>    mis_feature
<222>    (121) ... (173)
<223>    Multiple cloning sites

<220>
<221>    terminator
<222>    (189) ... (313)
<223>    T7 transcription termination

<220>
<221>    primer_binding
<222>    (223) ... (242)
<223>    T7 Reverse primer

<220>
<221>    rep_origem
<222>    (384) ... (839)
<223>    Origin of Phage f1

<220>
<221>    promotor
<222>    (858) ... (930)

<223>    Promotor of Neomycin Fosfotransferase (gene KanR)

<220>
<221>    CDS
<222>    (1008) ... (1802)
<223>    Gene for Neomycin Fosfotransferase (gene KanR)

<220>
<221>    rep_origin
<222>    (1986) ... (2941)
<223>    Origin of pUC

<220>
<221>    misc_feature
<222>    (2611) ... (2689)
<223>    par sequence

<400>    19

gatctcgatc ccgcgaaatt aatacgactc actataggga gaccacaacg gtttccctct        60

agaaataatt ttgtttaact ttaagaagga gatatacat atg cat cac cat cac        114
                                           Met His His His His
                                           1               5

cat cac ctc gag gga tcc gac ctc gag atc tgc agc tgg tac cat gga        162
His His Leu Glu Gly Ser Asp Leu Glu Ile Cys Ser Trp Tyr His Gly
                10                  15                  20

att cga agc ttg atc cgg ctg cta aca aag ccc gaa agg aag ctg agt        210
Ile Arg Ser Leu Ile Arg Leu Leu Thr Lys Pro Glu Arg Lys Leu Ser
            25                  30                  35

tgg ctg ctg cca ccg ctg agc aat aac tag cataacccct tggggcctct        260
Trp Leu Leu Pro Pro Leu Ser Asn Asn
        40                  45

aaacgggtct tgaggggttt tttgctgaaa ggaggaacta tatccggatc tggcgtaata        320

gcgaagaggc ccgcaccgat cgcccttccc aacagttgcg cagcctgaat ggcgaatggg        380

acgcgccctg tagcggcgca ttaagcgcgg cgggtgtggt ggttacgcgc agcgtgaccg        440

ctacacttgc cagcgcccta gcgcccgctc ctttcgcttt cttcccttcc tttctcgcca        500

cgttcgccgg ctttccccgt caagctctaa atcgggggct ccctttaggg ttccgattta        560

gtgctttacg gcacctcgac cccaaaaaac ttgattaggg tgatggttca cgtagtgggc        620

catcgccctg atagacggtt tttcgccctt tgacgttgga gtccacgttc tttaatagtg        680

gactcttgtt ccaaactgga acaacactca accctatctc ggtctattct tttgatttat        740

aagggatttt gccgatttcg gcctattggt taaaaaatga gctgatttaa caaaaattta        800

acgcgaattt taacaaaata ttaacgctta caatttaggt ggcacgtcga gttatggaca        860

gcaagcgaac cggaattgcc agctggggcg ccctctggta aggttgggaa gccctgcaaa        920

gtaaactgga tggctttctt gccgccaagg atctgatggc gcaggggatc aagatttgat        980

caagagacag gatgaggatc gtttcgc atg att gaa caa gat gga ttg cac gca     1034
                              Met Ile Glu Gln Asp Gly Leu His Ala

                                          50                  55

ggt tct ccg gcc gct tgg gtg gag agg cta ttc ggc tat gac tgg gca 1082
Gly Ser Pro Ala Ala Trp Val Glu Arg Leu Phe Gly Tyr Asp Trp Ala
                60                  65                  70

caa cag aca atc ggc tgc tct gat gcc gcc gtg ttc cgg ctg tca gcg 1130
Gln Gln Thr Ile Gly Cys Ser Asp Ala Ala Val Phe Arg Leu Ser Ala
            75                  80                  85

cag ggg cgc ccg gtt ctt ttt gtc aag acc gac ctg tcc ggt gcc ctg 1178
Gln Gly Arg Pro Val Leu Phe Val Lys Thr Asp Leu Ser Gly Ala Leu
        90                  95                  100

aat gaa ctc caa gac gag gca gcg cgg cta tcg tgg ctg gcc acg acg 1226
Asn Glu Leu Gln Asp Glu Ala Ala Arg Leu Ser Trp Leu Ala Thr Thr
    105                 110                 115

ggc gtt cct tgc gca gct gtg ctc gac gtt gtc act gaa gcg gga agg 1274
Gly Val Pro Cys Ala Ala Val Leu Asp Val Val Thr Glu Ala Gly Arg
120                 125                 130                 135

gac tgg ctg cta ttg ggc gaa gtg ccg ggg cag gat ctc ctg tca tct 1322
Asp Trp Leu Leu Leu Gly Glu Val Pro Gly Gln Asp Leu Leu Ser Ser
                140                 145                 150

cac ctt gct cct gcc gag aaa gta tcc atc atg gct gat gca atg cgg 1370
His Leu Ala Pro Ala Glu Lys Val Ser Ile Met Ala Asp Ala Met Arg
            155                 160                 165

cgg ctg cat acg ctt gat ccg gct acc tgc cca ttc gac cac caa gcg 1418
Arg Leu His Thr Leu Asp Pro Ala Thr Cys Pro Phe Asp His Gln Ala
        170                 175                 180

aaa cat cgc atc gag cga gca cgt act cgg atg gaa gcc ggt ctt gtc 1466
Lys His Arg Ile Glu Arg Ala Arg Thr Arg Met Glu Ala Gly Leu Val
    185                 190                 195

gat cag gat gat ctg gac gaa gag cat cag ggg ctc gcg cca gcc gaa 1514
Asp Gln Asp Asp Leu Asp Glu Glu His Gln Gly Leu Ala Pro Ala Glu
200                 205                 210                 215

ctg ttc gcc agg ctc aag gcg cgg atg ccc gac ggc gag gat ctc gtc 1562
Leu Phe Ala Arg Leu Lys Ala Arg Met Pro Asp Gly Glu Asp Leu Val
                220                 225                 230

gtg acc cac ggc gat gcc tgc ttg ccg aat atc atg gtg gaa aat ggc 1610
Val Thr His Gly Asp Ala Cys Leu Pro Asn Ile Met Val Glu Asn Gly
            235                 240                 245

cgc ttt tct gga ttc atc gac tgt ggc cgg ctg ggt gtg gcg gac cgc 1658
Arg Phe Ser Gly Phe Ile Asp Cys Gly Arg Leu Gly Val Ala Asp Arg
        250                 255                 260

tat cag gac ata gcg ttg gct acc cgt gat att gct gaa gag ctt ggc 1706
Tyr Gln Asp Ile Ala Leu Ala Thr Arg Asp Ile Ala Glu Glu Leu Gly
    265                 270                 275

ggc gaa tgg gct gac cgc ttc ctc gtg ctt tac ggt atc gcc gct ccc 1754
Gly Glu Trp Ala Asp Arg Phe Leu Val Leu Tyr Gly Ile Ala Ala Pro
280                 285                 290                 295

gat tcg cag cgc atc gcc ttc tat cgc ctt ctt gac gag ttc ttc tga 1802

Asp Ser Gln Arg Ile Ala Phe Tyr Arg Leu Leu Asp Glu Phe Phe
                300                 305                 310

gcgggactct ggggttcgaa attctagtgc attggtaact gtcagaccaa gtttactcat 1862

atatacttta gattgattta aaacttcatt tttaatttaa aaggatctag gtgaagatcc 1922

tttttgataa tctcatgacc aaaatccctt aacgtgagtt ttcgttccac tgagcgtcag 1982

accccgtaga aaagatcaaa ggatcttctt gagatccttt ttttctgcgc gtaatctgct 2042

gcttgcaaac aaaaaaacca ccgctaccag cggtggtttg tttgccggat caagagctac 2102

caactctttt tccgaaggta actggcttca gcagagcgca gataccaaat actgttcttc 2162

tagtgtagcc gtagttaggc caccacttca agaactctgt agcaccgcct acatacctcg 2222

ctctgctaat cctgttacca gtggctgctg ccagtggcga taagtcgtgt cttaccgggt 2282

tggactcaag acgatagtta ccggataagg cgcagcggtc gggctgaacg gggggttcgt 2342

gcacacagcc cagcttggag cgaacgacct acaccgaact gagataccta cagcgtgagc 2402

tatgagaaag cgccacgctt cccgaaggga gaaaggcgga caggtatccg gtaagcggca 2462

gggtcggaac aggagagcgc acgagggagc ttccaggggg aaacgcctgg tatctttata 2522

gtcctgtcgg gtttcgccac ctctgacttg agcgtcgatt tttgtgatgc tcgtcagggg 2582

ggcggagcct atggaaaaac gccagcagtc gagtttgtct ccgaccatca ggcacctgag 2642

tcgctgtctt tttcgtgaca ttcagttcgc tgcgctcacg gctctggcag tgaatggggg 2702

taaatggcac tacaggcgcc ttttatggat tcatgcaagg aaactaccca taatacaaga 2762

aaagcccgtc acgggcttct cagggcgttt tatggcgggt ctgctatgtg gtgctatctg 2822

actttttgct gttcagcagt tcctgccctc tgattttcca gtctgaccac ttcggattat 2882

cccgtctagt acgcggcctt tttacggttc ctggcctttt gctggccttt tgctcacatg 2942

ttctttcctg cgttatcccc tgattctgtg gataaccgta ttaccgcctt tgagtgagct 3002

gataccgctc gccgcagccg aacgaccgag cgcagcgagt cagtgagcga ggaagcggaa 3062

gagcgcccaa tacgcaaacc gcctctcccc gcgcgttggc cgattcatta atgcag 3118

<210> SEQ ID No: 20
<211> 46
<212> Protein
<213> Artificial sequence

<220>
<223> Synthetic construct

<400> 20

Met His His His His His His Leu Glu Gly Ser Asp Leu Glu Ile Cys
1               5                   10                  15

Ser Trp Tyr His Gly Ile Arg Ser Leu Ile Arg Leu Leu Thr Lys Pro
20                  25                  30

Glu Arg Lys Leu Ser Trp Leu Leu Pro Pro Leu Ser Asn Asn
35                  40                  45

<210>    SEQ ID No: 21
<211>    264
<212>    Protein
<213>    Artificial sequence

<220>
<223>    Synthetic construct

<400>    21

<210>    SEQ ID No: 22
<211>    66
<212>    DNA
<213>    Artificial sequence

<220>
<223>    Sequence for introduce RBS and start codon ATG in the gene Rrp4

<400>    22

tctagaaata attttgttta actttaagaa ggagatatat atatggctat cgatgcggat        60

atcgcc                                                                    66

<210>    SEQ ID No: 23
<211>    167
<212>    DNA
<213>    Artificial sequence

<220>
<223>    End sequence for the gene Rrp4, promotor and start codon for the
gene Rrp41

<400>    23

agatcttaag gtaccctgca ggcttaagtc gaacagaaag taatcgtatt gtacacggcc        60

gcaacttcga aatcaatacg actcactata gggagaccac aacggtttcc catcttagta       120

tattagttta actttaagaa ggagatatac atatggctac gcgtgca                     167

<210>    SEQ ID No: 24
<211>    162
<212>    DNA
<213>    Artificial sequence

<220>
<223>    End sequence for the gene Rrp41, promotor e start codon for the
gene Rrp42

<400>    24

ggatcctaaa ctagtgaatt cgcttaagtc gaacagaaag taatcgtatt gtacacggcc        60

gcataatcga aattaatacg actcactata gggagaccac aacggtttcc catcttagta       120

tattagttaa gtataagaag gagatatacc atgggtgtcg ac                          162



<210>    SEQ ID No: 25
<211>    23
<212>    DNA
<213>    Artificial sequence

<220>
<223>    End sequence for the gene Rrp42

<400>    25

ctcgagtaag cggccgcaag ctt                                                23



<210>    SEQ ID No: 26
<211>    783
<212>    DNA
<213>    Artificial sequence

<220>
<223>    Synthetic gene for protein Rrp4, aa 2 until 262

<220>
<221>    CDS
<222>    (1) ... (783)
<223>    aa 2 until 262 of the gene Rrp4

<400>    26

aaa cgt atc ttc gta cag aat cgt gaa ctg gtg gtg ccg ggt act ctg          48
Lys Arg Ile Phe Val Gln Asn Arg Glu Leu Val Val Pro Gly Thr Leu
1               5                   10                  15

ctg gcc cag ggt cct tat aaa aac ggt cgt ggc act ttc cgt gaa ggt          96
Leu Ala Gln Gly Pro Tyr Lys Asn Gly Arg Gly Thr Phe Arg Glu Gly
            20                  25                  30

agc cgt atc tac tct acc gtt atc ggt ctg gtg gac atc aaa ggc aac         144
Ser Arg Ile Tyr Ser Thr Val Ile Gly Leu Val Asp Ile Lys Gly Asn
        35                  40                  45

acc att cgt gtg att cct ctg gag ggt ccg tac atc ccg gaa gta ggt         192
Thr Ile Arg Val Ile Pro Leu Glu Gly Pro Tyr Ile Pro Glu Val Gly
    50                  55                  60

gac aac gtt atc ggt aaa att gtg gac gtt aag ttc tct agc tgg gtt         240
Asp Asn Val Ile Gly Lys Ile Val Asp Val Lys Phe Ser Ser Trp Val
65                  70                  75                  80

gta gac att ggt gcg ccg tat ccg gcc aac ttg aag att cag gac ttc          288
Val Asp Ile Gly Ala Pro Tyr Pro Ala Asn Leu Lys Ile Gln Asp Phe
                85                  90                  95

act gat gaa aaa att gat ttg ctg cgt act gac ctt cgt aag ttc ttt          336
Thr Asp Glu Lys Ile Asp Leu Leu Arg Thr Asp Leu Arg Lys Phe Phe
            100                 105                 110

gat att ggt gac att atc tac gct aaa gta aaa gcc atc acc gaa gtt          384
Asp Ile Gly Asp Ile Ile Tyr Ala Lys Val Lys Ala Ile Thr Glu Val
        115                 120                 125

aac aac att gac ctg acg acc aaa ggc atg ccg ttt aac ggt ggt ccg          432
Asn Asn Ile Asp Leu Thr Thr Lys Gly Met Pro Phe Asn Gly Gly Pro
    130                 135                 140

ctg aaa ggt ggt caa atc gtg aaa att acg ccg tct cgt gtg ccg cgt          480
Leu Lys Gly Gly Gln Ile Val Lys Ile Thr Pro Ser Arg Val Pro Arg
145                 150                 155                 160

gtt atc ggt cgc ggt ggc agc atg att aac atg att aaa aaa ctg acc          528
Val Ile Gly Arg Gly Gly Ser Met Ile Asn Met Ile Lys Lys Leu Thr
                165                 170                 175

atg act cgt att att gtt ggc caa aac ggc tgg att tgg gtg tct ggc          576
Met Thr Arg Ile Ile Val Gly Gln Asn Gly Trp Ile Trp Val Ser Gly
            180                 185                 190

aaa aac gat gcg ctg gaa aaa ctt gct att gag gcc att ctg aaa att          624
Lys Asn Asp Ala Leu Glu Lys Leu Ala Ile Glu Ala Ile Leu Lys Ile
        195                 200                 205

gac aaa gag tct cat acc cgt ggt ctg act gac cgt att aaa gcg ctg          672
Asp Lys Glu Ser His Thr Arg Gly Leu Thr Asp Arg Ile Lys Ala Leu
    210                 215                 220

ctg ctg tcc cgc ctg caa gaa ctg aaa gaa aaa ggt gtt att gaa gag          720
Leu Leu Ser Arg Leu Gln Glu Leu Lys Glu Lys Gly Val Ile Glu Glu
225                 230                 235                 240

att ccg aaa ctg gaa gaa gag ccg cag ggc gaa gat gaa gta aac ggc          768
Ile Pro Lys Leu Glu Glu Glu Pro Gln Gly Glu Asp Glu Val Asn Gly
                245                 250                 255

aat gac ggt gaa gct                                                      783
Asn Asp Gly Glu Ala
            260

<210>    SEQ ID No: 27
<211>    261
<212>    Protein
<213>    Artificial sequence

<220>
<223>    Synthetic construct

<400>    27

Lys Arg Ile Phe Val Gln Asn Arg Glu Leu Val Val Pro Gly Thr Leu
1               5                   10                  15

Leu Ala Gln Gly Pro Tyr Lys Asn Gly Arg Gly Thr Phe Arg Glu Gly
            20                  25                  30

Ser Arg Ile Tyr Ser Thr Val Ile Gly Leu Val Asp Ile Lys Gly Asn
        35                  40                  45

Thr Ile Arg Val Ile Pro Leu Glu Gly Pro Tyr Ile Pro Glu Val Gly
    50                  55                  60

Asp Asn Val Ile Gly Lys Ile Val Asp Val Lys Phe Ser Ser Trp Val
65                  70                  75                  80

Val Asp Ile Gly Ala Pro Tyr Pro Ala Asn Leu Lys Ile Gln Asp Phe
                85                  90                  95

Thr Asp Glu Lys Ile Asp Leu Leu Arg Thr Asp Leu Arg Lys Phe Phe
            100                 105                 110

Asp Ile Gly Asp Ile Ile Tyr Ala Lys Val Lys Ala Ile Thr Glu Val
        115                 120                 125

Asn Asn Ile Asp Leu Thr Thr Lys Gly Met Pro Phe Asn Gly Gly Pro
    130                 135                 140

Leu Lys Gly Gly Gln Ile Val Lys Ile Thr Pro Ser Arg Val Pro Arg
145                 150                 155                 160

Val Ile Gly Arg Gly Gly Ser Met Ile Asn Met Ile Lys Lys Leu Thr
                165                 170                 175

Met Thr Arg Ile Ile Val Gly Gln Asn Gly Trp Ile Trp Val Ser Gly
            180                 185                 190

Lys Asn Asp Ala Leu Glu Lys Leu Ala Ile Glu Ala Ile Leu Lys Ile
        195                 200                 205

Asp Lys Glu Ser His Thr Arg Gly Leu Thr Asp Arg Ile Lys Ala Leu
    210                 215                 220

Leu Leu Ser Arg Leu Gln Glu Leu Lys Glu Lys Gly Val Ile Glu Glu
225                 230                 235                 240

Ile Pro Lys Leu Glu Glu Glu Pro Gln Gly Glu Asp Glu Val Asn Gly
                245                 250                 255

Asn Asp Gly Glu Ala
            260

<210>    SEQ ID No: 28
<211>    Size: 732
<212>    DNA
<213>    Artificial sequence

<220>
<223>    Synthetic gene for the protein Rrp41, aa 3 until 246, double mutant
R89T and K91S

<220>
<221>    CDS
<222>    (1) ... (732)
<223>    Synthetic gene for the protein Rrp41, aa 3 until 246, doube mutant
R89T and K91S

<400>    28

gag aaa ccg gaa ggt ctg aaa ctg att gat gaa aac ggc cgc cgt att        48
Glu Lys Pro Glu Gly Leu Lys Leu Ile Asp Glu Asn Gly Arg Arg Ile
1               5                   10                  15

gac ggt cgt aaa aaa tac gaa ctg cgt ccg att aaa atg gaa gtg ggt        96
Asp Gly Arg Lys Lys Tyr Glu Leu Arg Pro Ile Lys Met Glu Val Gly
            20                  25                  30

gta ctg aaa aac gct aac ggt tcc gcg tac atc gaa tgg ggc aaa aac        144
Val Leu Lys Asn Ala Asn Gly Ser Ala Tyr Ile Glu Trp Gly Lys Asn
        35                  40                  45

aaa att atc gca gcg gtt tac ggt ccg cgt gaa ctc cac ccg aaa cac        192
Lys Ile Ile Ala Ala Val Tyr Gly Pro Arg Glu Leu His Pro Lys His
    50                  55                  60

ctt cag cgt ccg gac cgc gcc atc ctg cgt gta cgt tac aac atg gcc        240
Leu Gln Arg Pro Asp Arg Ala Ile Leu Arg Val Arg Tyr Asn Met Ala
65                  70                  75                  80

ccg ttc tct gtt gaa gag act aag agc ccg ggt ccg gac cgt cgt agc        288
Pro Phe Ser Val Glu Glu Thr Lys Ser Pro Gly Pro Asp Arg Arg Ser
                85                  90                  95

att gag att tct aaa gta att aaa ggt gcg ctg gaa ccg gca ctt att        336
Ile Glu Ile Ser Lys Val Ile Lys Gly Ala Leu Glu Pro Ala Leu Ile
            100                 105                 110

ctg gaa atg ttc ccg cgt act gct att gat gta ttc atc gaa gtt ctg        384
Leu Glu Met Phe Pro Arg Thr Ala Ile Asp Val Phe Ile Glu Val Leu
        115                 120                 125

caa gcg gat gcc ggt acg cgc gtt gca ggt atc acc gcg gct agc ctg        432
Gln Ala Asp Ala Gly Thr Arg Val Ala Gly Ile Thr Ala Ala Ser Leu
    130                 135                 140

gca ctg gcc gac gct ggt att ccg atg cgc gac ctg gtt gcc gct tgt        480
Ala Leu Ala Asp Ala Gly Ile Pro Met Arg Asp Leu Val Ala Ala Cys
145                 150                 155                 160

gcg gcg ggt aaa atc gaa ggc gaa att gtt ctg gac ctg aac aaa gaa        528
Ala Ala Gly Lys Ile Glu Gly Glu Ile Val Leu Asp Leu Asn Lys Glu
                165                 170                 175

gaa gat aac tac ggc gaa gcg gac gtg ccg gta gct atc atg ccg ctg 576
Glu Asp Asn Tyr Gly Glu Ala Asp Val Pro Val Ala Ile Met Pro Leu
            180                 185                 190

aag aac gat att acc ctg ctg caa atg gat ggt tac ctg acc aag gac 624
Lys Asn Asp Ile Thr Leu Leu Gln Met Asp Gly Tyr Leu Thr Lys Asp
        195                 200                 205

gag ttc atc gag gcg gtt aaa ctg gct att aaa ggt gct aag gcg gtg 672
Glu Phe Ile Glu Ala Val Lys Leu Ala Ile Lys Gly Ala Lys Ala Val
    210                 215                 220

tat cag aaa cag cgt gaa gca ctg aaa gaa aag tat ctg aaa att gcc 720
Tyr Gln Lys Gln Arg Glu Ala Leu Lys Glu Lys Tyr Leu Lys Ile Ala
225                 230                 235                 240

cag gaa gtt gag 732
Gln Glu Val Glu

<210> SEQ ID No: 29
<211> 244
<212> proteína
<213> Artificial sequence

<220>
<223> Synthetic construct

<400> 29

Glu Lys Pro Glu Gly Leu Lys Leu Ile Asp Glu Asn Gly Arg Arg Ile
1               5                   10                  15

Asp Gly Arg Lys Lys Tyr Glu Leu Arg Pro Ile Lys Met Glu Val Gly
            20                  25                  30

Val Leu Lys Asn Ala Asn Gly Ser Ala Tyr Ile Glu Trp Gly Lys Asn
        35                  40                  45

Lys Ile Ile Ala Ala Val Tyr Gly Pro Arg Glu Leu His Pro Lys His
    50                  55                  60

Leu Gln Arg Pro Asp Arg Ala Ile Leu Arg Val Arg Tyr Asn Met Ala
65                  70                  75                  80

Pro Phe Ser Val Glu Glu Thr Lys Ser Pro Gly Pro Asp Arg Arg Ser
                85                  90                  95

Ile Glu Ile Ser Lys Val Ile Lys Gly Ala Leu Glu Pro Ala Leu Ile
            100                 105                 110

Leu Glu Met Phe Pro Arg Thr Ala Ile Asp Val Phe Ile Glu Val Leu
        115                 120                 125

Gln Ala Asp Ala Gly Thr Arg Val Ala Gly Ile Thr Ala Ala Ser Leu
    130                 135                 140

Ala Leu Ala Asp Ala Gly Ile Pro Met Arg Asp Leu Val Ala Ala Cys
145                 150                 155                 160

Ala Ala Gly Lys Ile Glu Gly Glu Ile Val Leu Asp Leu Asn Lys Glu
                165                 170                 175

Glu Asp Asn Tyr Gly Glu Ala Asp Val Pro Val Ala Ile Met Pro Leu
180                 185                 190

Lys Asn Asp Ile Thr Leu Leu Gln Met Asp Gly Tyr Leu Thr Lys Asp
        195                 200                 205

Glu Phe Ile Glu Ala Val Lys Leu Ala Ile Lys Gly Ala Lys Ala Val
    210                 215                 220

Tyr Gln Lys Gln Arg Glu Ala Leu Lys Glu Lys Tyr Leu Lys Ile Ala
225                 230                 235                 240

Gln Glu Val Glu

<210> SEQ ID No: 30
<211> 819
<212> DNA
<213> Artificial sequence

<220>
<223> Synthetic gene for the Rrp42 protein, aa 2 until 274

<220>
<221> CDS
<222> (1) ... (819)
<223> Rrp42 protein, aa 1 until 274

<400> 30

tct gat aat gaa atc gtg gcg ggt att atg cgc gac cat att att aac          48
Ser Asp Asn Glu Ile Val Ala Gly Ile Met Arg Asp His Ile Ile Asn
1               5                   10                  15

ctg ctt aag gaa ggt aaa cgt att gac gac cgc ggt ttc gaa gat tac          96
Leu Leu Lys Glu Gly Lys Arg Ile Asp Asp Arg Gly Phe Glu Asp Tyr
            20                  25                  30

cgc ccg att gaa att gag gta ggt gtt att gaa aaa gcg gag ggt agc         144
Arg Pro Ile Glu Ile Glu Val Gly Val Ile Glu Lys Ala Glu Gly Ser
        35                  40                  45

gcg ctg gtg aaa ctg ggt agc act cag gtt ctg gtg ggt att aaa acc         192
Ala Leu Val Lys Leu Gly Ser Thr Gln Val Leu Val Gly Ile Lys Thr
    50                  55                  60

tct ctg ggc gag ccg ttt ccg gac acc ccg aac atg ggc gtg atg acc     240
Ser Leu Gly Glu Pro Phe Pro Asp Thr Pro Asn Met Gly Val Met Thr
65                  70                  75                  80

acc aat gtt gaa ctt gtt ccg ctg gcg tct ccg act ttc gaa ccg ggc     288
Thr Asn Val Glu Leu Val Pro Leu Ala Ser Pro Thr Phe Glu Pro Gly
                85                  90                  95

ccg ccg gac gag cgt gct atc gaa ctg gca cgt gtt att gac cgt ggt     336
Pro Pro Asp Glu Arg Ala Ile Glu Leu Ala Arg Val Ile Asp Arg Gly
            100                 105                 110

att cgt gag tcc aaa gca ctg aat ctg gaa aaa atg gtt atc gtg ccg     384
Ile Arg Glu Ser Lys Ala Leu Asn Leu Glu Lys Met Val Ile Val Pro
        115                 120                 125

ggc aaa att gtt cgc gta gtt ttt atc gac gtt cac gtt ctt gac cac     432
Gly Lys Ile Val Arg Val Val Phe Ile Asp Val His Val Leu Asp His
    130                 135                 140

gac ggt aac ctg atg gac gcc atc ggt att gca gct atc gcc gca ctg     480
Asp Gly Asn Leu Met Asp Ala Ile Gly Ile Ala Ala Ile Ala Ala Leu
145                 150                 155                 160

ctg aac gca cgt gtt ccg aaa gtt cgc tac aac gaa gaa acg ggt gaa     528
Leu Asn Ala Arg Val Pro Lys Val Arg Tyr Asn Glu Glu Thr Gly Glu
                165                 170                 175

gtt gaa acc ctg gat gaa act gaa ccg ctg ccg gtt gaa aag att ccg     576
Val Glu Thr Leu Asp Glu Thr Glu Pro Leu Pro Val Glu Lys Ile Pro
            180                 185                 190

gtt ccg gtt act ttc gct aaa att ggt aat atc ctg gta gtt gac ccg     624
Val Pro Val Thr Phe Ala Lys Ile Gly Asn Ile Leu Val Val Asp Pro
        195                 200                 205

agc ctg gat gaa gag ctc gtg atg gac ggc aaa att acc att acc act     672
Ser Leu Asp Glu Glu Leu Val Met Asp Gly Lys Ile Thr Ile Thr Thr
    210                 215                 220

gac gaa acc ggc cat att tct gcc gtt cag aaa tct gaa ggt ggc gct     720
Asp Glu Thr Gly His Ile Ser Ala Val Gln Lys Ser Glu Gly Gly Ala
225                 230                 235                 240

ttc aaa ctg gaa gaa gta atg tac gcg gtg gaa act gca ttc aag aag     768
Phe Lys Leu Glu Glu Val Met Tyr Ala Val Glu Thr Ala Phe Lys Lys
                245                 250                 255

gcg gaa gaa atc cgt aaa ctg att ctg gaa gct gtt gag aag gcc aaa     816
Ala Glu Glu Ile Arg Lys Leu Ile Leu Glu Ala Val Glu Lys Ala Lys
            260                 265                 270

cag                                                                 819
Gln

<210>    SEQ ID No: 31
<211>    273
<212>    Protein
<213>    Artificial sequence

<220>
<223>    Synthetic construct

<400>    31

Ser Asp Asn Glu Ile Val Ala Gly Ile Met Arg Asp His Ile Ile Asn
1               5                   10                  15

Leu Leu Lys Glu Gly Lys Arg Ile Asp Asp Arg Gly Phe Glu Asp Tyr
            20                  25                  30

Arg Pro Ile Glu Ile Glu Val Gly Val Ile Glu Lys Ala Glu Gly Ser
        35                  40                  45

Ala Leu Val Lys Leu Gly Ser Thr Gln Val Leu Val Gly Ile Lys Thr
    50                  55                  60

Ser Leu Gly Glu Pro Phe Pro Asp Thr Pro Asn Met Gly Val Met Thr
65                  70                  75                  80

Thr Asn Val Glu Leu Val Pro Leu Ala Ser Pro Thr Phe Glu Pro Gly
                85                  90                  95

Pro Pro Asp Glu Arg Ala Ile Glu Leu Ala Arg Val Ile Asp Arg Gly
            100                 105                 110

Ile Arg Glu Ser Lys Ala Leu Asn Leu Glu Lys Met Val Ile Val Pro
        115                 120                 125

Gly Lys Ile Val Arg Val Val Phe Ile Asp Val His Val Leu Asp His
    130                 135                 140

Asp Gly Asn Leu Met Asp Ala Ile Gly Ile Ala Ala Ile Ala Ala Leu
145                 150                 155                 160

Leu Asn Ala Arg Val Pro Lys Val Arg Tyr Asn Glu Glu Thr Gly Glu
                165                 170                 175

Val Glu Thr Leu Asp Glu Thr Glu Pro Leu Pro Val Glu Lys Ile Pro
            180                 185                 190

Val Pro Val Thr Phe Ala Lys Ile Gly Asn Ile Leu Val Val Asp Pro
        195                 200                 205

Ser Leu Asp Glu Glu Leu Val Met Asp Gly Lys Ile Thr Ile Thr Thr
    210                 215                 220

Asp Glu Thr Gly His Ile Ser Ala Val Gln Lys Ser Glu Gly Gly Ala

225                 230                 235                 240

Phe Lys Leu Glu Glu Val Met Tyr Ala Val Glu Thr Ala Phe Lys Lys
                245                 250                 255

Ala Glu Glu Ile Arg Lys Leu Ile Leu Glu Ala Val Glu Lys Ala Lys
            260                 265                 270

Gln

<210>    SEQ ID No: 32
<211>    2752
<212>    DNA
<213>    Artificial sequence

<220>
<223>    Synthetic gene for the three components of the P. abyssi exosome
complex

<220>
<221>    misc_feature
<222>    (1) ... (6)
<223>   Restriction site XbaI

<220>
<221>    CDS
<210>    (43) ... (858)
<210>    Rrp4 protein of Pyrococcus abyssi

<220>
<221>    misc_feature
<222>    (49) ... (63)
<223>    Restriction sites ClaI and EcoRV

<220>
<221>    promotor
<222>    (922) ... (942)
<223>    T7 promotor for the gene of Rrp41

<220>
<221>    misc_feature
<222>    (922) ... (942)
<223>    Site Bgl II, Stop codon, restriction sites KpnI and PstI

<220>
<221>    misc_feature
<222>    (999) ... (1013)
<223>    Restriction sites NdeI and MluI

<220>
<221>    CDS
<222>    (1002) ... (1757)
<223>    R89T and K91S double mutant protein of Rrp41 from Pyrococcus abyssi

<220>
<221>    nome: misc_feature
<222>    (1749) ... (1769)
<223>    Site BamHI, Stop codon,  restriction sites SpeI and EcoRI

<220>
<221>    promotor
<222>    (1822) ... (1842)
<223>    T7 promotor for the gene of Rrp42

<220>
<221>    misc_feature
<222>    (1897) ... (1910)
<223>   Restriction sites NcoI and SaII

<220>
<221>    CDS
<222>    (1899) ... (2738)
<223>    Rrp42 protein of Pyrococcus abyssi

<220>
<221>    misc_feature
<222>    (2730) ... (2752)
<223>    Site XhoI, Stop codon,  restriction sites NotI and HindIII

<400> 32

tctagaaata attttgttta actttaagaa ggagatatat at atg gct atc gat          54
                                                Met Ala Ile Asp
                                                1

gcg gat atc gcc aaa cgt atc ttc gta cag aat cgt gaa ctg gtg gtg        102
Ala Asp Ile Ala Lys Arg Ile Phe Val Gln Asn Arg Glu Leu Val Val
5                   10                  15                  20

ccg ggt act ctg ctg gcc cag ggt cct tat aaa aac ggt cgt ggc act        150
Pro Gly Thr Leu Leu Ala Gln Gly Pro Tyr Lys Asn Gly Arg Gly Thr
                25                  30                  35

ttc cgt gaa ggt agc cgt atc tac tct acc gtt atc ggt ctg gtg gac        198
Phe Arg Glu Gly Ser Arg Ile Tyr Ser Thr Val Ile Gly Leu Val Asp
            40                  45                  50

atc aaa ggc aac acc att cgt gtg att cct ctg gag ggt ccg tac atc        246
Ile Lys Gly Asn Thr Ile Arg Val Ile Pro Leu Glu Gly Pro Tyr Ile
        55                  60                  65

ccg gaa gta ggt gac aac gtt atc ggt aaa att gtg gac gtt aag ttc        294
Pro Glu Val Gly Asp Asn Val Ile Gly Lys Ile Val Asp Val Lys Phe
    70                  75                  80

tct agc tgg gtt gta gac att ggt gcg ccg tat ccg gcc aac ttg aag        342
Ser Ser Trp Val Val Asp Ile Gly Ala Pro Tyr Pro Ala Asn Leu Lys
85                  90                  95                  100

att cag gac ttc act gat gaa aaa att gat ttg ctg cgt act gac ctt        390
Ile Gln Asp Phe Thr Asp Glu Lys Ile Asp Leu Leu Arg Thr Asp Leu
                105                 110                 115

cgt aag ttc ttt gat att ggt gac att atc tac gct aaa gta aaa gcc        438
Arg Lys Phe Phe Asp Ile Gly Asp Ile Ile Tyr Ala Lys Val Lys Ala
            120                 125                 130

atc acc gaa gtt aac aac att gac ctg acg acc aaa ggc atg ccg ttt        486
Ile Thr Glu Val Asn Asn Ile Asp Leu Thr Thr Lys Gly Met Pro Phe
        135                 140                 145

aac ggt ggt ccg ctg aaa ggt ggt caa atc gtg aaa att acg ccg tct 534
Asn Gly Gly Pro Leu Lys Gly Gly Gln Ile Val Lys Ile Thr Pro Ser
    150                 155                 160

cgt gtg ccg cgt gtt atc ggt cgc ggt ggc agc atg att aac atg att 582
Arg Val Pro Arg Val Ile Gly Arg Gly Gly Ser Met Ile Asn Met Ile
165                 170                 175                 180

aaa aaa ctg acc atg act cgt att att gtt ggc caa aac ggc tgg att 630
Lys Lys Leu Thr Met Thr Arg Ile Ile Val Gly Gln Asn Gly Trp Ile
                185                 190                 195

tgg gtg tct ggc aaa aac gat gcg ctg gaa aaa ctt gct att gag gcc 678
Trp Val Ser Gly Lys Asn Asp Ala Leu Glu Lys Leu Ala Ile Glu Ala
            200                 205                 210

att ctg aaa att gac aaa gag tct cat acc cgt ggt ctg act gac cgt 726
Ile Leu Lys Ile Asp Lys Glu Ser His Thr Arg Gly Leu Thr Asp Arg
        215                 220                 225

att aaa gcg ctg ctg ctg tcc cgc ctg caa gaa ctg aaa gaa aaa ggt 774
Ile Lys Ala Leu Leu Leu Ser Arg Leu Gln Glu Leu Lys Glu Lys Gly
    230                 235                 240

gtt att gaa gag att ccg aaa ctg gaa gaa gag ccg cag ggc gaa gat 822
Val Ile Glu Glu Ile Pro Lys Leu Glu Glu Glu Pro Gln Gly Glu Asp
245                 250                 255                 260

gaa gta aac ggc aat gac ggt gaa gct aga tct taa ggtaccctgc 868
Glu Val Asn Gly Asn Asp Gly Glu Ala Arg Ser
                265                 270

aggcttaagt cgaacagaaa gtaatcgtat tgtacacggc cgcaacttcg aaatcaatac 928

gactcactat agggagacca caacggtttc ccatcttagt atattagttt aactttaaga 988

aggagatata cat atg gct acg cgt gca gag aaa ccg gaa ggt ctg aaa 1037
Met Ala Thr Arg Ala Glu Lys Pro Glu Gly Leu Lys
            275                 280

ctg att gat gaa aac ggc cgc cgt att gac ggt cgt aaa aaa tac gaa 1085
Leu Ile Asp Glu Asn Gly Arg Arg Ile Asp Gly Arg Lys Lys Tyr Glu
    285                 290                 295

ctg cgt ccg att aaa atg gaa gtg ggt gta ctg aaa aac gct aac ggt 1133
Leu Arg Pro Ile Lys Met Glu Val Gly Val Leu Lys Asn Ala Asn Gly
300                 305                 310                 315

tcc gcg tac atc gaa tgg ggc aaa aac aaa att atc gca gcg gtt tac 1181
Ser Ala Tyr Ile Glu Trp Gly Lys Asn Lys Ile Ile Ala Ala Val Tyr
                320                 325                 330

ggt ccg cgt gaa ctc cac ccg aaa cac ctt cag cgt ccg gac cgc gcc 1229
Gly Pro Arg Glu Leu His Pro Lys His Leu Gln Arg Pro Asp Arg Ala
            335                 340                 345

atc ctg cgt gta cgt tac aac atg gcc ccg ttc tct gtt gaa gag act 1277
Ile Leu Arg Val Arg Tyr Asn Met Ala Pro Phe Ser Val Glu Glu Thr
        350                 355                 360

aag agc ccg ggt ccg gac cgt cgt agc att gag att tct aaa gta att 1325
Lys Ser Pro Gly Pro Asp Arg Arg Ser Ile Glu Ile Ser Lys Val Ile
    365                 370                 375

aaa ggt gcg ctg gaa ccg gca ctt att ctg gaa atg ttc ccg cgt act     1373
Lys Gly Ala Leu Glu Pro Ala Leu Ile Leu Glu Met Phe Pro Arg Thr
380                 385                 390                 395

gct att gat gta ttc atc gaa gtt ctg caa gcg gat gcc ggt acg cgc     1421
Ala Ile Asp Val Phe Ile Glu Val Leu Gln Ala Asp Ala Gly Thr Arg
                400                 405                 410

gtt gca ggt atc acc gcg gct agc ctg gca ctg gcc gac gct ggt att     1469
Val Ala Gly Ile Thr Ala Ala Ser Leu Ala Leu Ala Asp Ala Gly Ile
            415                 420                 425

ccg atg cgc gac ctg gtt gcc gct tgt gcg gcg ggt aaa atc gaa ggc     1517
Pro Met Arg Asp Leu Val Ala Ala Cys Ala Ala Gly Lys Ile Glu Gly
        430                 435                 440

gaa att gtt ctg gac ctg aac aaa gaa gaa gat aac tac ggc gaa gcg     1565
Glu Ile Val Leu Asp Leu Asn Lys Glu Glu Asp Asn Tyr Gly Glu Ala
    445                 450                 455

gac gtg ccg gta gct atc atg ccg ctg aag aac gat att acc ctg ctg     1613
Asp Val Pro Val Ala Ile Met Pro Leu Lys Asn Asp Ile Thr Leu Leu
460                 465                 470                 475

caa atg gat ggt tac ctg acc aag gac gag ttc atc gag gcg gtt aaa     1661
Gln Met Asp Gly Tyr Leu Thr Lys Asp Glu Phe Ile Glu Ala Val Lys
                480                 485                 490

ctg gct att aaa ggt gct aag gcg gtg tat cag aaa cag cgt gaa gca     1709
Leu Ala Ile Lys Gly Ala Lys Ala Val Tyr Gln Lys Gln Arg Glu Ala
            495                 500                 505

ctg aaa gaa aag tat ctg aaa att gcc cag gaa gtt gag gga tcc taa     1757
Leu Lys Glu Lys Tyr Leu Lys Ile Ala Gln Glu Val Glu Gly Ser
        510                 515                 520

actagtgaat tcgcttaagt cgaacagaaa gtaatcgtat tgtacacggc cgcataatcg     1817

aaattaatac gactcactat agggagacca caacggtttc ccatcttagt atattagtta     1877

agtataagaa ggagatatac c atg ggt gtc gac tct gat aat gaa atc gtg     1928
                        Met Gly Val Asp Ser Asp Asn Glu Ile Val
                                    525                 530

gcg ggt att atg cgc gac cat att att aac ctg ctt aag gaa ggt aaa     1976
Ala Gly Ile Met Arg Asp His Ile Ile Asn Leu Leu Lys Glu Gly Lys
        535                 540                 545

cgt att gac gac cgc ggt ttc gaa gat tac cgc ccg att gaa att gag     2024
Arg Ile Asp Asp Arg Gly Phe Glu Asp Tyr Arg Pro Ile Glu Ile Glu
    550                 555                 560

gta ggt gtt att gaa aaa gcg gag ggt agc gcg ctg gtg aaa ctg ggt     2072
Val Gly Val Ile Glu Lys Ala Glu Gly Ser Ala Leu Val Lys Leu Gly
565                 570                 575                 580

agc act cag gtt ctg gtg ggt att aaa acc tct ctg ggc gag ccg ttt     2120
Ser Thr Gln Val Leu Val Gly Ile Lys Thr Ser Leu Gly Glu Pro Phe
                585                 590                 595

ccg gac acc ccg aac atg ggc gtg atg acc acc aat gtt gaa ctt gtt     2168
Pro Asp Thr Pro Asn Met Gly Val Met Thr Thr Asn Val Glu Leu Val

600                 605                 610

ccg ctg gcg tct ccg act ttc gaa ccg ggc ccg ccg gac gag cgt gct          2216
Pro Leu Ala Ser Pro Thr Phe Glu Pro Gly Pro Pro Asp Glu Arg Ala
        615                 620                 625

atc gaa ctg gca cgt gtt att gac cgt ggt att cgt gag tcc aaa gca          2264
Ile Glu Leu Ala Arg Val Ile Asp Arg Gly Ile Arg Glu Ser Lys Ala
    630                 635                 640

ctg aat ctg gaa aaa atg gtt atc gtg ccg ggc aaa att gtt cgc gta          2312
Leu Asn Leu Glu Lys Met Val Ile Val Pro Gly Lys Ile Val Arg Val
645                 650                 655                 660

gtt ttt atc gac gtt cac gtt ctt gac cac gac ggt aac ctg atg gac          2360
Val Phe Ile Asp Val His Val Leu Asp His Asp Gly Asn Leu Met Asp
                665                 670                 675

gcc atc ggt att gca gct atc gcc gca ctg ctg aac gca cgt gtt ccg          2408
Ala Ile Gly Ile Ala Ala Ile Ala Ala Leu Leu Asn Ala Arg Val Pro
            680                 685                 690

aaa gtt cgc tac aac gaa gaa acg ggt gaa gtt gaa acc ctg gat gaa          2456
Lys Val Arg Tyr Asn Glu Glu Thr Gly Glu Val Glu Thr Leu Asp Glu
        695                 700                 705

act gaa ccg ctg ccg gtt gaa aag att ccg gtt ccg gtt act ttc gct          2504
Thr Glu Pro Leu Pro Val Glu Lys Ile Pro Val Pro Val Thr Phe Ala
    710                 715                 720

aaa att ggt aat atc ctg gta gtt gac ccg agc ctg gat gaa gag ctc          2552
Lys Ile Gly Asn Ile Leu Val Val Asp Pro Ser Leu Asp Glu Glu Leu
725                 730                 735                 740

gtg atg gac ggc aaa att acc att acc act gac gaa acc ggc cat att          2600
Val Met Asp Gly Lys Ile Thr Ile Thr Thr Asp Glu Thr Gly His Ile
                745                 750                 755

tct gcc gtt cag aaa tct gaa ggt ggc gct ttc aaa ctg gaa gaa gta          2648
Ser Ala Val Gln Lys Ser Glu Gly Gly Ala Phe Lys Leu Glu Glu Val
            760                 765                 770

atg tac gcg gtg gaa act gca ttc aag aag gcg gaa gaa atc cgt aaa          2696
Met Tyr Ala Val Glu Thr Ala Phe Lys Lys Ala Glu Glu Ile Arg Lys
        775                 780                 785

ctg att ctg gaa gct gtt gag aag gcc aaa cag ctc gag taa                  2738
Leu Ile Leu Glu Ala Val Glu Lys Ala Lys Gln Leu Glu
    790                 795                 800

gcggccgcaa gctt                                                          2752

<210> SEQ ID No: 33
<211> 271
<212> Protein
<213> Artificial sequence

<220>
<223> Synthetic construct

<400> 33

Met Ala Ile Asp Ala Asp Ile Ala Lys Arg Ile Phe Val Gln Asn Arg
1               5                   10                  15

Glu Leu Val Val Pro Gly Thr Leu Leu Ala Gln Gly Pro Tyr Lys Asn
            20                  25                  30

Gly Arg Gly Thr Phe Arg Glu Gly Ser Arg Ile Tyr Ser Thr Val Ile
        35                  40                  45

Gly Leu Val Asp Ile Lys Gly Asn Thr Ile Arg Val Ile Pro Leu Glu
    50                  55                  60

Gly Pro Tyr Ile Pro Glu Val Gly Asp Asn Val Ile Gly Lys Ile Val
65                  70                  75                  80

Asp Val Lys Phe Ser Ser Trp Val Val Asp Ile Gly Ala Pro Tyr Pro
                85                  90                  95

Ala Asn Leu Lys Ile Gln Asp Phe Thr Asp Glu Lys Ile Asp Leu Leu
            100                 105                 110

Arg Thr Asp Leu Arg Lys Phe Phe Asp Ile Gly Asp Ile Ile Tyr Ala
        115                 120                 125

Lys Val Lys Ala Ile Thr Glu Val Asn Asn Ile Asp Leu Thr Thr Lys
    130                 135                 140

Gly Met Pro Phe Asn Gly Gly Pro Leu Lys Gly Gly Gln Ile Val Lys
145                 150                 155                 160

Ile Thr Pro Ser Arg Val Pro Arg Val Ile Gly Arg Gly Gly Ser Met
                165                 170                 175

Ile Asn Met Ile Lys Lys Leu Thr Met Thr Arg Ile Ile Val Gly Gln
            180                 185                 190

Asn Gly Trp Ile Trp Val Ser Gly Lys Asn Asp Ala Leu Glu Lys Leu
        195                 200                 205

Ala Ile Glu Ala Ile Leu Lys Ile Asp Lys Glu Ser His Thr Arg Gly
    210                 215                 220

Leu Thr Asp Arg Ile Lys Ala Leu Leu Leu Ser Arg Leu Gln Glu Leu
225                 230                 235                 240

Lys Glu Lys Gly Val Ile Glu Glu Ile Pro Lys Leu Glu Glu Glu Pro
                245                 250                 255

Gln Gly Glu Asp Glu Val Asn Gly Asn Asp Gly Glu Ala Arg Ser
            260                 265                 270

<210>    SEQ ID No: 34
<211>    251
<212>    Protein
<213>    Artificial sequence

<220>
<223>    Artificial construct

<400>    34

Met Ala Thr Arg Ala Glu Lys Pro Glu Gly Leu Lys Leu Ile Asp Glu
1               5                   10                  15

Asn Gly Arg Arg Ile Asp Gly Arg Lys Lys Tyr Glu Leu Arg Pro Ile
            20                  25                  30

Lys Met Glu Val Gly Val Leu Lys Asn Ala Asn Gly Ser Ala Tyr Ile
        35                  40                  45

Glu Trp Gly Lys Asn Lys Ile Ile Ala Ala Val Tyr Gly Pro Arg Glu
    50                  55                  60

Leu His Pro Lys His Leu Gln Arg Pro Asp Arg Ala Ile Leu Arg Val
65                  70                  75                  80

Arg Tyr Asn Met Ala Pro Phe Ser Val Glu Glu Thr Lys Ser Pro Gly
                85                  90                  95

Pro Asp Arg Arg Ser Ile Glu Ile Ser Lys Val Ile Lys Gly Ala Leu
            100                 105                 110

Glu Pro Ala Leu Ile Leu Glu Met Phe Pro Arg Thr Ala Ile Asp Val
115                 120                 125

Phe Ile Glu Val Leu Gln Ala Asp Ala Gly Thr Arg Val Ala Gly Ile
    130                 135                 140

Thr Ala Ala Ser Leu Ala Leu Ala Asp Ala Gly Ile Pro Met Arg Asp
145                 150                 155                 160

Leu Val Ala Ala Cys Ala Ala Gly Lys Ile Glu Gly Glu Ile Val Leu
                165                 170                 175

Asp Leu Asn Lys Glu Glu Asp Asn Tyr Gly Glu Ala Asp Val Pro Val

180                 185                 190

Ala Ile Met Pro Leu Lys Asn Asp Ile Thr Leu Leu Gln Met Asp Gly
        195                 200                 205

Tyr Leu Thr Lys Asp Glu Phe Ile Glu Ala Val Lys Leu Ala Ile Lys
    210                 215                 220

Gly Ala Lys Ala Val Tyr Gln Lys Gln Arg Glu Ala Leu Lys Glu Lys
225                 230                 235                 240

Tyr Leu Lys Ile Ala Gln Glu Val Glu Gly Ser
                245                 250

<210>    SEQ ID No: 35
<211>    279
<212>    Protein
<213>    Artificial sequence

<220>
<223>    Artificial construct

<400>    35

Met Gly Val Asp Ser Asp Asn Glu Ile Val Ala Gly Ile Met Arg Asp
1               5                   10                  15

His Ile Ile Asn Leu Leu Lys Glu Gly Lys Arg Ile Asp Asp Arg Gly
            20                  25                  30

Phe Glu Asp Tyr Arg Pro Ile Glu Ile Glu Val Gly Val Ile Glu Lys
        35                  40                  45

Ala Glu Gly Ser Ala Leu Val Lys Leu Gly Ser Thr Gln Val Leu Val
    50                  55                  60

Gly Ile Lys Thr Ser Leu Gly Glu Pro Phe Pro Asp Thr Pro Asn Met
65                  70                  75                  80

Gly Val Met Thr Thr Asn Val Glu Leu Val Pro Leu Ala Ser Pro Thr
                85                  90                  95

Phe Glu Pro Gly Pro Pro Asp Glu Arg Ala Ile Glu Leu Ala Arg Val
100                 105                 110

Ile Asp Arg Gly Ile Arg Glu Ser Lys Ala Leu Asn Leu Glu Lys Met
115                 120                 125

Val Ile Val Pro Gly Lys Ile Val Arg Val Val Phe Ile Asp Val His
    130                 135                 140

Val Leu Asp His Asp Gly Asn Leu Met Asp Ala Ile Gly Ile Ala Ala
145                 150                 155                 160

Ile Ala Ala Leu Leu Asn Ala Arg Val Pro Lys Val Arg Tyr Asn Glu
                165                 170                 175

Glu Thr Gly Glu Val Glu Thr Leu Asp Glu Thr Glu Pro Leu Pro Val
            180                 185                 190

Glu Lys Ile Pro Val Pro Val Thr Phe Ala Lys Ile Gly Asn Ile Leu
        195                 200                 205

Val Val Asp Pro Ser Leu Asp Glu Glu Leu Val Met Asp Gly Lys Ile
    210                 215                 220

Thr Ile Thr Thr Asp Glu Thr Gly His Ile Ser Ala Val Gln Lys Ser
225                 230                 235                 240

Glu Gly Gly Ala Phe Lys Leu Glu Glu Val Met Tyr Ala Val Glu Thr
                245                 250                 255

Ala Phe Lys Lys Ala Glu Glu Ile Arg Lys Leu Ile Leu Glu Ala Val
            260                 265                 270

Glu Lys Ala Lys Gln Leu Glu
        275

<210>    SEQ ID No: 36
<211>    5754
<212>    DNA
<213>    Artificial sequence

<220>
<223>   pMRKA-EXO plasmid for the expression of recombinant proteins in
fusion with proteins of Pyrococcus abyssi exosome

<400>    36

gatctcgatc ccgcgaaatt aatacgactc actataggga gaccacaacg gtttccctct      60

agaaataatt ttgtttaact ttaagaagga gatatatata tggctatcga tgcggatatc     120

gccaaacgta tcttcgtaca gaatcgtgaa ctggtggtgc cgggtactct gctggcccag     180

ggtccttata aaaacggtcg tggcactttc cgtgaaggta gccgtatcta ctctaccgtt     240

atcggtctgg tggacatcaa aggcaacacc attcgtgtga ttcctctgga gggtccgtac     300

atcccggaag taggtgacaa cgttatcggt aaaattgtgg acgttaagtt ctctagctgg 360

gttgtagaca ttggtgcgcc gtatccggcc aacttgaaga ttcaggactt cactgatgaa 420

aaaattgatt tgctgcgtac tgaccttcgt aagttctttg atattggtga cattatctac 480

gctaaagtaa aagccatcac cgaagttaac aacattgacc tgacgaccaa aggcatgccg 540

tttaacggtg gtccgctgaa aggtggtcaa atcgtgaaaa ttacgccgtc tcgtgtgccg 600

cgtgttatcg gtcgcggtgg cagcatgatt aacatgatta aaaaactgac catgactcgt 660

attattgttg gccaaaacgg ctggatttgg gtgtctggca aaaacgatgc gctggaaaaa 720

cttgctattg aggccattct gaaaattgac aaagagtctc atacccgtgg tctgactgac 780

cgtattaaag cgctgctgct gtcccgcctg caagaactga aagaaaaagg tgttattgaa 840

gagattccga aactggaaga agagccgcag ggcgaagatg aagtaaacgg caatgacggt 900

gaagctagat cttaaggtac cctgcaggct taagtcgaac agaaagtaat cgtattgtac 960

acggccgcaa cttcgaaatc aatacgactc actataggga gaccacaacg gtttcccatc 1020

ttagtatatt agtttaactt taagaaggag atatacatat ggctacgcgt gcagagaaac 1080

cggaaggtct gaaactgatt gatgaaaacg gccgccgtat tgacggtcgt aaaaaatacg 1140

aactgcgtcc gattaaaatg gaagtgggtg tactgaaaaa cgctaacggt tccgcgtaca 1200

tcgaatgggg caaaaacaaa attatcgcag cggtttacgg tccgcgtgaa ctccacccga 1260

aacaccttca gcgtccggac cgcgccatcc tgcgtgtacg ttacaacatg gccccgttct 1320

ctgttgaaga gactaagagc ccgggtccgg accgtcgtag cattgagatt tctaaagtaa 1380

ttaaaggtgc gctggaaccg gcacttattc tggaaatgtt cccgcgtact gctattgatg 1440

tattcatcga agttctgcaa gcggatgccg gtacgcgcgt tgcaggtatc accgcggcta 1500

gcctggcact ggccgacgct ggtattccga tgcgcgacct ggttgccgct tgtgcggcgg 1560

gtaaaatcga aggcgaaatt gttctggacc tgaacaaaga agaagataac tacggcgaag 1620

cggacgtgcc ggtagctatc atgccgctga agaacgatat taccctgctg caaatggatg 1680

gttacctgac caaggacgag ttcatcgagg cggttaaact ggctattaaa ggtgctaagg 1740

cggtgtatca gaaacagcgt gaagcactga aagaaaagta tctgaaaatt gcccaggaag 1800

ttgagggatc ctaaactagt gaattcgctt aagtcgaaca gaaagtaatc gtattgtaca 1860

cggccgcata atcgaaatta atacgactca ctatagggag accacaacgg tttcccatct 1920

tagtatatta gttaagtata agaaggagat ataccatggg tgtcgactct gataatgaaa 1980

tcgtggcggg tattatgcgc gaccatatta ttaacctgct taaggaaggt aaacgtattg 2040

acgaccgcgg tttcgaagat taccgcccga ttgaaattga ggtaggtgtt attgaaaaag 2100

cggagggtag cgcgctggtg aaactgggta gcactcaggt tctggtgggt attaaaacct 2160

ctctgggcga gccgtttccg gacaccccga acatgggcgt gatgaccacc aatgttgaac 2220

ttgttccgct ggcgtctccg actttcgaac cgggcccgcc ggacgagcgt gctatcgaac 2280
tggcacgtgt tattgaccgt ggtattcgtg agtccaaagc actgaatctg gaaaaaatgg 2340
ttatcgtgcc gggcaaaatt gttcgcgtag tttttatcga cgttcacgtt cttgaccacg 2400
acggtaacct gatggacgcc atcggtattg cagctatcgc cgcactgctg aacgcacgtg 2460
ttccgaaagt tcgctacaac gaagaaacgg gtgaagttga aaccctggat gaaactgaac 2520
cgctgccggt tgaaaagatt ccggttccgg ttactttcgc taaaattggt aatatcctgg 2580
tagttgaccc gagcctggat gaagagctcg tgatggacgg caaaattacc attaccactg 2640
acgaaaccgg ccatatttct gccgttcaga aatctgaagg tggcgctttc aaactggaag 2700
aagtaatgta cgcggtggaa actgcattca agaaggcgga agaaatccgt aaactgattc 2760
tggaagctgt tgagaaggcc aaacagctcg agtaagcggc cgcaagcttg atccggctgc 2820
taacaaagcc cgaaaggaag ctgagttggc tgctgccacc gctgagcaat aactagcata 2880
accccttggg gcctctaaac gggtcttgag gggttttttg ctgaaaggag gaactatatc 2940
cggatctggc gtaatagcga agaggcccgc accgatcgcc cttcccaaca gttgcgcagc 3000
ctgaatggcg aatgggacgc gccctgtagc ggcgcattaa gcgcggcggg tgtggtggtt 3060
acgcgcagcg tgaccgctac acttgccagc gccctagcgc ccgctccttt cgctttcttc 3120
ccttcctttc tcgccacgtt cgccggcttt ccccgtcaag ctctaaatcg ggggctccct 3180
ttagggttcc gatttagtgc tttacggcac ctcgacccca aaaaacttga ttagggtgat 3240
ggttcacgta gtgggccatc gccctgatag acggtttttc gccctttgac gttggagtcc 3300
acgttcttta atagtggact cttgttccaa actggaacaa cactcaaccc tatctcggtc 3360
tattcttttg atttataagg gattttgccg atttcggcct attggttaaa aaatgagctg 3420
atttaacaaa aatttaacgc gaattttaac aaaatattaa cgcttacaat ttaggtggca 3480
cgtcgagtta tggacagcaa gcgaaccgga attgccagct ggggcgccct ctggtaaggt 3540
tgggaagccc tgcaaagtaa actggatggc tttcttgccg ccaaggatct gatggcgcag 3600
gggatcaaga tttgatcaag agacaggatg aggatcgttt cgcatgattg aacaagatgg 3660
attgcacgca ggttctccgg ccgcttgggt ggagaggcta ttcggctatg actgggcaca 3720
acagacaatc ggctgctctg atgccgccgt gttccggctg tcagcgcagg ggcgcccggt 3780
tctttttgtc aagaccgacc tgtccggtgc cctgaatgaa ctccaagacg aggcagcgcg 3840
gctatcgtgg ctggccacga cgggcgttcc ttgcgcagct gtgctcgacg ttgtcactga 3900
agcgggaagg gactggctgc tattgggcga agtgccgggg caggatctcc tgtcatctca 3960
ccttgctcct gccgagaaag tatccatcat ggctgatgca atgcggcggc tgcatacgct 4020
tgatccggct acctgcccat tcgaccacca agcgaaacat cgcatcgagc gagcacgtac 4080

tcggatggaa gccggtcttg tcgatcagga tgatctggac gaagagcatc aggggctcgc 4140

gccagccgaa ctgttcgcca ggctcaaggc gcggatgccc gacggcgagg atctcgtcgt 4200

gacccacggc gatgcctgct tgccgaatat catggtggaa aatggccgct tttctggatt 4260

catcgactgt ggccggctgg gtgtggcgga ccgctatcag gacatagcgt tggctacccg 4320

tgatattgct gaagagcttg gcggcgaatg ggctgaccgc ttcctcgtgc tttacggtat 4380

cgccgctccc gattcgcagc gcatcgcctt ctatcgcctt cttgacgagt tcttctgagc 4440

gggactctgg ggttcgaaat tctagtgcat tggtaactgt cagaccaagt ttactcatat 4500

atactttaga ttgatttaaa acttcatttt taatttaaaa ggatctaggt gaagatcctt 4560

tttgataatc tcatgaccaa aatcccttaa cgtgagtttt cgttccactg agcgtcagac 4620

cccgtagaaa agatcaaagg atcttcttga gatccttttt ttctgcgcgt aatctgctgc 4680

ttgcaaacaa aaaaaccacc gctaccagcg gtggtttgtt tgccggatca agagctacca 4740

actctttttc cgaaggtaac tggcttcagc agagcgcaga taccaaatac tgttcttcta 4800

gtgtagccgt agttaggcca ccacttcaag aactctgtag caccgcctac atacctcgct 4860

ctgctaatcc tgttaccagt ggctgctgcc agtggcgata agtcgtgtct taccgggttg 4920

gactcaagac gatagttacc ggataaggcg cagcggtcgg gctgaacggg gggttcgtgc 4980

acacagccca gcttggagcg aacgacctac accgaactga gatacctaca gcgtgagcta 5040

tgagaaagcg ccacgcttcc cgaagggaga aaggcggaca ggtatccggt aagcggcagg 5100

gtcggaacag gagagcgcac gagggagctt ccagggggaa acgcctggta tctttatagt 5160

cctgtcgggt ttcgccacct ctgacttgag cgtcgatttt tgtgatgctc gtcagggggg 5220

cggagcctat ggaaaaacgc cagcagtcga gtttgtctcc gaccatcagg cacctgagtc 5280

gctgtctttt tcgtgacatt cagttcgctg cgctcacggc tctggcagtg aatgggggta 5340

aatggcacta caggcgcctt ttatggattc atgcaaggaa actacccata atacaagaaa 5400

agcccgtcac gggcttctca gggcgtttta tggcgggtct gctatgtggt gctatctgac 5460

tttttgctgt tcagcagttc ctgccctctg attttccagt ctgaccactt cggattatcc 5520

cgtctagtac gcggcctttt tacggttcct ggccttttgc tggccttttg ctcacatgtt 5580

ctttcctgcg ttatcccctg attctgtgga taaccgtatt accgcctttg agtgagctga 5640

taccgctcgc cgcagccgaa cgaccgagcg cagcgagtca gtgagcgagg aagcggaaga 5700

gcgcccaata cgcaaaccgc ctctccccgc gcgttggccg attcattaat gcag 5754

<210> SEQ ID No: 37
<211> 4795
<212> DNA
<213> Artificial sequence

<220>
<223>    Expression vector by fusion antigen with Rrp41, Rrp42 proteins of
Pyrococcus abyssi exosome

<400>    37

gatctcgatc ccgcgaaatt aatacgactc actataggga gaccacaacg gtttccctct      60

agaaataatt ttgtttaact ttaagaagga gatatacata tggctacgcg tgcagagaaa     120

ccggaaggtc tgaaactgat tgatgaaaac ggccgccgta ttgacggtcg taaaaaatac     180

gaactgcgtc cgattaaaat ggaagtgggt gtactgaaaa acgctaacgg ttccgcgtac     240

atcgaatggg gcaaaaacaa aattatcgca gcggtttacg gtccgcgtga actccacccg     300

aaacaccttc agcgtccgga ccgcgccatc ctgcgtgtac gttacaacat ggccccgttc     360

tctgttgaag agactaagag cccgggtccg gaccgtcgta gcattgagat ttctaaagta     420

attaaaggtg cgctggaacc ggcacttatt ctggaaatgt tcccgcgtac tgctattgat     480

gtattcatcg aagttctgca agcggatgcc ggtacgcgcg ttgcaggtat caccgcggct     540

agcctggcac tggccgacgc tggtattccg atgcgcgacc tggttgccgc ttgtgcggcg     600

ggtaaaatcg aaggcgaaat tgttctggac ctgaacaaag aagaagataa ctacggcgaa     660

gcggacgtgc cggtagctat catgccgctg aagaacgata ttaccctgct gcaaatggat     720

ggttacctga ccaaggacga gttcatcgag gcggttaaac tggctattaa aggtgctaag     780

gcggtgtatc agaaacagcg tgaagcactg aaagaaaagt atctgaaaat tgcccaggaa     840

gttgagggat cctaaactag tgaattcgct taagtcgaac agaaagtaat cgtattgtac     900

acggccgcat aatcgaaatt aatacgactc actataggga gaccacaacg gtttcccatc     960

ttagtatatt agttaagtat aagaaggaga tataccatgg gtgtcgactc tgataatgaa    1020

atcgtggcgg gtattatgcg cgaccatatt attaacctgc ttaaggaagg taaacgtatt    1080

gacgaccgcg gtttcgaaga ttaccgcccg attgaaattg aggtaggtgt tattgaaaaa    1140

gcggaggtta gcgcgctggt gaaactgggt agcactcagg ttctggtggg tattaaaacc    1200

tctctgggcg agccgtttcc ggacaccccg aacatgggcg tgatgaccac caatgttgaa    1260

cttgttccgc tggcgtctcc gactttcgaa ccgggcccgc cggacgagcg tgctatcgaa    1320

ctggcacgtg ttattgaccg tggtattcgt gagtccaaag cactgaatct ggaaaaaatg    1380

gttatcgtgc cgggcaaaat tgttcgcgta gtttttatcg acgttcacgt tcttgaccac    1440

gacggtaacc tgatggacgc catcggtatt gcagctatcg ccgcactgct gaacgcacgt    1500

gttccgaaag ttcgctacaa cgaagaaacg ggtgaagttg aaaccctgga tgaaactgaa    1560

ccgctgccgg ttgaaaagat tccggttccg gttactttcg ctaaaattgg taatatcctg    1620

gtagttgacc cgagcctgga tgaagagctc gtgatggacg gcaaaattac cattaccact    1680

gacgaaaccg gccatatttc tgccgttcag aaatctgaag gtggcgcttt caaactggaa    1740

gaagtaatgt acgcggtgga aactgcattc aagaaggcgg aagaaatccg taaactgatt 1800

ctggaagctg ttgagaaggc caaacagctc gagtaagcgg ccgcaagctt gatccggctg 1860

ctaacaaagc ccgaaaggaa gctgagttgg ctgctgccac cgctgagcaa taactagcat 1920

aaccccttgg ggcctctaaa cgggtcttga ggggtttttt gctgaaagga ggaactatat 1980

ccggatctgg cgtaatagcg aagaggcccg caccgatcgc ccttcccaac agttgcgcag 2040

cctgaatggc gaatgggacg cgccctgtag cggcgcatta agcgcggcgg gtgtggtggt 2100

tacgcgcagc gtgaccgcta cacttgccag cgccctagcg cccgctcctt tcgctttctt 2160

cccttccttt ctcgccacgt tcgccggctt tccccgtcaa gctctaaatc gggggctccc 2220

tttagggttc cgatttagtg ctttacggca cctcgacccc aaaaaacttg attagggtga 2280

tggttcacgt agtgggccat cgccctgata gacggttttt cgccctttga cgttggagtc 2340

cacgttcttt aatagtggac tcttgttcca aactggaaca acactcaacc ctatctcggt 2400

ctattctttt gatttataag ggattttgcc gatttcggcc tattggttaa aaaatgagct 2460

gatttaacaa aaatttaacg cgaattttaa caaaatatta acgcttacaa tttaggtggc 2520

acgtcgagtt atggacagca agcgaaccgg aattgccagc tggggcgccc tctggtaagg 2580

ttgggaagcc ctgcaaagta aactggatgg ctttcttgcc gccaaggatc tgatggcgca 2640

ggggatcaag atttgatcaa gagacaggat gaggatcgtt tcgcatgatt gaacaagatg 2700

gattgcacgc aggttctccg gccgcttggg tggagaggct attcggctat gactgggcac 2760

aacagacaat cggctgctct gatgccgccg tgttccggct gtcagcgcag gggcgcccgg 2820

ttctttttgt caagaccgac ctgtccggtg ccctgaatga actccaagac gaggcagcgc 2880

ggctatcgtg gctggccacg acgggcgttc cttgcgcagc tgtgctcgac gttgtcactg 2940

aagcgggaag ggactggctg ctattgggcg aagtgccggg gcaggatctc ctgtcatctc 3000

accttgctcc tgccgagaaa gtatccatca tggctgatgc aatgcggcgg ctgcatacgc 3060

ttgatccggc tacctgccca ttcgaccacc aagcgaaaca tcgcatcgag cgagcacgta 3120

ctcggatgga agccggtctt gtcgatcagg atgatctgga cgaagagcat caggggctcg 3180

cgccagccga actgttcgcc aggctcaagg cgcggatgcc cgacggcgag gatctcgtcg 3240

tgacccacgg cgatgcctgc ttgccgaata tcatggtgga aaatggccgc ttttctggat 3300

tcatcgactg tggccggctg ggtgtggcgg accgctatca ggacatagcg ttggctaccc 3360

gtgatattgc tgaagagctt ggcggcgaat gggctgaccg cttcctcgtg ctttacggta 3420

tcgccgctcc cgattcgcag cgcatcgcct tctatcgcct tcttgacgag ttcttctgag 3480

cgggactctg gggttcgaaa ttctagtgca ttggtaactg tcagaccaag tttactcata 3540

tatactttag attgatttaa aacttcattt ttaatttaaa aggatctagg tgaagatcct 3600

ttttgataat ctcatgacca aaatccctta acgtgagttt tcgttccact gagcgtcaga 3660

ccccgtagaa aagatcaaag gatcttcttg agatcctttt tttctgcgcg taatctgctg 3720

cttgcaaaca aaaaaaccac cgctaccagc ggtggtttgt ttgccggatc aagagctacc 3780

aactcttttt ccgaaggtaa ctggcttcag cagagcgcag ataccaaata ctgttcttct 3840

agtgtagccg tagttaggcc accacttcaa gaactctgta gcaccgccta catacctcgc 3900

tctgctaatc ctgttaccag tggctgctgc cagtggcgat aagtcgtgtc ttaccgggtt 3960

ggactcaaga cgatagttac cggataaggc gcagcggtcg ggctgaacgg ggggttcgtg 4020

cacacagccc agcttggagc gaacgaccta caccgaactg agatacctac agcgtgagct 4080

atgagaaagc gccacgcttc ccgaagggag aaaggcggac aggtatccgg taagcggcag 4140

ggtcggaaca ggagagcgca cgagggagct tccaggggga aacgcctggt atctttatag 4200

tcctgtcggg tttcgccacc tctgacttga gcgtcgattt ttgtgatgct cgtcaggggg 4260

gcggagccta tggaaaaacg ccagcagtcg agtttgtctc cgaccatcag gcacctgagt 4320

cgctgtcttt ttcgtgacat tcagttcgct gcgctcacgg ctctggcagt gaatgggggt 4380

aaatggcact acaggcgcct tttatggatt catgcaagga aactacccat aatacaagaa 4440

aagcccgtca cgggcttctc agggcgtttt atggcgggtc tgctatgtgg tgctatctga 4500

ctttttgctg ttcagcagtt cctgccctct gattttccag tctgaccact tcggattatc 4560

ccgtctagta cgcggccttt ttacggttcc tggccttttg ctggcctttt gctcacatgt 4620

tctttcctgc gttatcccct gattctgtgg ataaccgtat taccgccttt gagtgagctg 4680

ataccgctcg ccgcagccga acgaccgagc gcagcgagtc agtgagcgag gaagcggaag 4740

agcgcccaat acgcaaaccg cctctccccg cgcgttggcc gattcattaa tgcag 4795

<210> SEQ ID No: 38
<211> 29
<212> DNA
<213> Artificial sequence

<220>
<223> Foward primer for PCR amplification of Rrp42 gene

<400> 38

accatatggg ttctgataat gaaatcgtg 29

<210> SEQ ID No: 39
<211> 47
<212> DNA
<213> Artificial sequence

<220>
<223> Reverse primer for PCR amplification of Rrp42 gene

<400>    39

aactgcaggg atccctcgag accaccctgt ttggccttct caacagc                47

<210>    SEQ ID No: 40
<211>    3917
<212>    DNA
<213>    Artificial sequence

<220>
<223>    Epxression vector for recombinant fusion proteins by fusion with
Rrp42 of P. abyssi exosome

<400>    40

gatctcgatc ccgcgaaatt aatacgactc actataggga gaccacaacg gtttccctct      60

agaaataatt ttgtttaact ttaagaagga gatatacata tgggttctga taatgaaatc     120

gtggcgggta ttatgcgcga ccatattatt aacctgctta aggaaggtaa acgtattgac     180

gaccgcggtt tcgaagatta ccgcccgatt gaaattgagg taggtgttat tgaaaaagcg     240

gagggtagcg cgctggtgaa actgggtagc actcaggttc tggtgggtat taaaacctct     300

ctgggcgagc cgtttccgga caccccgaac atgggcgtga tgaccaccaa tgttgaactt     360

gttccgctgg cgtctccgac tttcgaaccg ggcccgccgg acgagcgtgc tatcgaactg     420

gcacgtgtta ttgaccgtgg tattcgtgag tccaaagcac tgaatctgga aaaaatggtt     480

atcgtgccgg gcaaaattgt tcgcgtagtt tttatcgacg ttcacgttct tgaccacgac     540

ggtaacctga tggacgccat cggtattgca gctatcgccg cactgctgaa cgcacgtgtt     600

ccgaaagttc gctacaacga agaaacgggt gaagttgaaa ccctggatga aactgaaccg     660

ctgccggttg aaaagattcc ggttccggtt actttcgcta aaattggtaa tatcctggta     720

gttgacccga gcctggatga agagctcgtg atggacggca aaattaccat taccactgac     780

gaaaccggcc atatttctgc cgttcagaaa tctgaaggtg gcgctttcaa actggaagaa     840

gtaatgtacg cggtggaaac tgcattcaag aaggcggaag aaatccgtaa actgattctg     900

gaagctgttg agaaggccaa acagggtggt ctcgagggat ccctgcagct ggtaccatgg     960

aattcgaagc ttgatccggc tgctaacaaa gcccgaaagg aagctgagtt ggctgctgcc    1020

accgctgagc aataactagc ataacccctt ggggcctcta aacgggtctt gaggggtttt    1080

ttgctgaaag gaggaactat atccggatct ggcgtaatag cgaagaggcc cgcaccgatc    1140

gcccttccca acagttgcgc agcctgaatg gcgaatggga cgcgccctgt agcggcgcat    1200

taagcgcggc gggtgtggtg gttacgcgca gcgtgaccgc tacacttgcc agcgccctag    1260

cgcccgctcc tttcgctttc ttcccttcct ttctcgccac gttcgccggc tttccccgtc    1320

aagctctaaa tcgggggctc cctttagggt tccgatttag tgctttacgg cacctcgacc    1380

ccaaaaaact tgattagggt gatggttcac gtagtgggcc atcgccctga tagacggttt 1440

ttcgcccttt gacgttggag tccacgttct ttaatagtgg actcttgttc caaactggaa 1500

caacactcaa ccctatctcg gtctattctt ttgatttata agggattttg ccgatttcgg 1560

cctattggtt aaaaaatgag ctgatttaac aaaaatttaa cgcgaatttt aacaaaatat 1620

taacgcttac aatttaggtg gcacgtcgag ttatggacag caagcgaacc ggaattgcca 1680

gctggggcgc cctctggtaa ggttgggaag ccctgcaaag taaactggat ggctttcttg 1740

ccgccaagga tctgatggcg caggggatca agatttgatc aagagacagg atgaggatcg 1800

tttcgcatga ttgaacaaga tggattgcac gcaggttctc cggccgcttg ggtggagagg 1860

ctattcggct atgactgggc acaacagaca atcggctgct ctgatgccgc cgtgttccgg 1920

ctgtcagcgc aggggcgccc ggttcttttt gtcaagaccg acctgtccgg tgccctgaat 1980

gaactccaag acgaggcagc gcggctatcg tggctggcca cgacgggcgt tccttgcgca 2040

gctgtgctcg acgttgtcac tgaagcggga agggactggc tgctattggg cgaagtgccg 2100

gggcaggatc tcctgtcatc tcaccttgct cctgccgaga aagtatccat catggctgat 2160

gcaatgcggc ggctgcatac gcttgatccg gctacctgcc cattcgacca ccaagcgaaa 2220

catcgcatcg agcgagcacg tactcggatg gaagccggtc ttgtcgatca ggatgatctg 2280

gacgaagagc atcaggggct cgcgccagcc gaactgttcg ccaggctcaa ggcgcggatg 2340

cccgacggcg aggatctcgt cgtgacccac ggcgatgcct gcttgccgaa tatcatggtg 2400

gaaaatggcc gcttttctgg attcatcgac tgtggccggc tgggtgtggc ggaccgctat 2460

caggacatag cgttggctac ccgtgatatt gctgaagagc ttggcggcga atgggctgac 2520

cgcttcctcg tgctttacgg tatcgccgct cccgattcgc agcgcatcgc cttctatcgc 2580

cttcttgacg agttcttctg agcgggactc tggggttcga aattctagtg cattggtaac 2640

tgtcagacca agtttactca tatatacttt agattgattt aaaacttcat ttttaattta 2700

aaaggatcta ggtgaagatc ctttttgata atctcatgac caaaatccct taacgtgagt 2760

tttcgttcca ctgagcgtca gaccccgtag aaaagatcaa aggatcttct tgagatcctt 2820

tttttctgcg cgtaatctgc tgcttgcaaa caaaaaaacc accgctacca gcggtggttt 2880

gtttgccgga tcaagagcta ccaactcttt ttccgaaggt aactggcttc agcagagcgc 2940

agataccaaa tactgttctt ctagtgtagc cgtagttagg ccaccacttc aagaactctg 3000

tagcaccgcc tacatacctc gctctgctaa tcctgttacc agtggctgct gccagtggcg 3060

ataagtcgtg tcttaccggg ttggactcaa gacgatagtt accggataag gcgcagcggt 3120

cgggctgaac ggggggttcg tgcacacagc ccagcttgga gcgaacgacc tacaccgaac 3180

tgagatacct acagcgtgag ctatgagaaa gcgccacgct tcccgaaggg agaaaggcgg 3240

acaggtatcc ggtaagcggc agggtcggaa caggagagcg cacgagggag cttccagggg 3300

gaaacgcctg gtatctttat agtcctgtcg ggtttcgcca cctctgactt gagcgtcgat 3360

ttttgtgatg ctcgtcaggg gggcggagcc tatggaaaaa cgccagcagt cgagtttgtc 3420

tccgaccatc aggcacctga gtcgctgtct ttttcgtgac attcagttcg ctgcgctcac 3480

ggctctggca gtgaatgggg gtaaatggca ctacaggcgc cttttatgga ttcatgcaag 3540

gaaactaccc ataatacaag aaaagcccgt cacgggcttc tcagggcgtt ttatggcggg 3600

tctgctatgt ggtgctatct gactttttgc tgttcagcag ttcctgccct ctgattttcc 3660

agtctgacca cttcggatta tcccgtctag tacgcggcct ttttacggtt cctggccttt 3720

tgctggcctt ttgctcacat gttctttcct gcgttatccc ctgattctgt ggataaccgt 3780

attaccgcct ttgagtgagc tgataccgct cgccgcagcc gaacgaccga gcgcagcgag 3840

tcagtgagcg aggaagcgga agagcgccca atacgcaaac cgcctctccc cgcgcgttgg 3900

ccgattcatt aatgcag 3917

<210> 41
<211> 368
<212> DNA
<213> Artificial sequence

<220>
<223> Coding artificial sequence of Z-Z domain of protein A of
S. aureus

<220>
<221> CDS
<222> (3)..(368)
<223> Z-Z domain of protein A of S. aureus

<400> 41

cc atg ggt gtg gat aac aaa ttt aac aaa gaa cag cag aat gcg ttt 47
Met Gly Val Asp Asn Lys Phe Asn Lys Glu Gln Gln Asn Ala Phe
1               5                   10                  15

tac gaa att ctg cac ctg ccg aac ctt aac gaa gaa cag cgt aac gct 95
Tyr Glu Ile Leu His Leu Pro Asn Leu Asn Glu Glu Gln Arg Asn Ala
                20                  25                  30

ttc att cag tct ctg aaa gat gac cct tct cag tct gcg aac ctg ctg 143
Phe Ile Gln Ser Leu Lys Asp Asp Pro Ser Gln Ser Ala Asn Leu Leu
            35                  40                  45

gct gaa gct aaa aaa ctg aac gac gct cag gct cct aaa gtt gac aac 191
Ala Glu Ala Lys Lys Leu Asn Asp Ala Gln Ala Pro Lys Val Asp Asn
        50                  55                  60

aaa ttc aac aaa gaa cag cag aac gct ttc tac gaa atc ttg cat ctg 239
Lys Phe Asn Lys Glu Gln Gln Asn Ala Phe Tyr Glu Ile Leu His Leu
    65                  70                  75

ccg aac ctg aac gaa gaa cag cgc aac gct ttc atc cag tcc ctg aaa 287

Pro Asn Leu Asn Glu Glu Gln Arg Asn Ala Phe Ile Gln Ser Leu Lys
80                  85                  90                  95

gac gac cct tcc cag tct gct aac ctg ctg gcg gaa gcg aaa aaa ctg        335
Asp Asp Pro Ser Gln Ser Ala Asn Leu Leu Ala Glu Ala Lys Lys Leu
                100                 105                 110

aac gat gct cag gcg cct aaa gtg ggc gtc gac                            368
Asn Asp Ala Gln Ala Pro Lys Val Gly Val Asp
            115                 120
<210>    42
<211>    122
<212>    Protein
<213>    Artificial sequence

<220>
<223>    Synthetic construct

<400>    42

Met Gly Val Asp Asn Lys Phe Asn Lys Glu Gln Gln Asn Ala Phe Tyr
1               5                   10                  15

Glu Ile Leu His Leu Pro Asn Leu Asn Glu Glu Gln Arg Asn Ala Phe
            20                  25                  30

Ile Gln Ser Leu Lys Asp Asp Pro Ser Gln Ser Ala Asn Leu Leu Ala
        35                  40                  45

Glu Ala Lys Lys Leu Asn Asp Ala Gln Ala Pro Lys Val Asp Asn Lys
    50                  55                  60

Phe Asn Lys Glu Gln Gln Asn Ala Phe Tyr Glu Ile Leu His Leu Pro
65                  70                  75                  80

Asn Leu Asn Glu Glu Gln Arg Asn Ala Phe Ile Gln Ser Leu Lys Asp
                85                  90                  95

Asp Pro Ser Gln Ser Ala Asn Leu Leu Ala Glu Ala Lys Lys Leu Asn
            100                 105                 110

Asp Ala Gln Ala Pro Lys Val Gly Val Asp
        115                 120

<210>    43
<211>    5148
<212>    DNA
<213>    Artificial sequence

<220>
<223>    Expression vector by fusion of Rrp41 and ZZ-Rrp42 proteins

<400>    43

gatctcgatc ccgcgaaatt aatacgactc actataggga gaccacaacg gtttccctct        60

agaataattt tgtttaactt taagaaggag atatacatat ggctacgcgt gcagagaaac 120

cggaaggtct gaaactgatt gatgaaaacg gccgccgtat tgacggtcgt aaaaaatacg 180

aactgcgtcc gattaaaatg gaagtgggtg tactgaaaaa cgctaacggt tccgcgtaca 240

tcgaatgggg caaaaacaaa attatcgcag cggtttacgg tccgcgtgaa ctccacccga 300

aacaccttca gcgtccggac cgcgccatcc tgcgtgtacg ttacaacatg gccccgttct 360

ctgttgaaga gactaagagc ccgggtccgg accgtcgtag cattgagatt tctaaagtaa 420

ttaaaggtgc gctggaaccg gcacttattc tggaaatgtt cccgcgtact gctattgatg 480

tattcatcga agttctgcaa gcggatgccg gtacgcgcgt tgcaggtatc accgcggcta 540

gcctggcact ggccgacgct ggtattccga tgcgcgacct ggttgccgct tgtgcggcgg 600

gtaaaatcga aggcgaaatt gttctggacc tgaacaaaga agaagataac tacggcgaag 660

cggacgtgcc ggtagctatc atgccgctga agaacgatat taccctgctg caaatggatg 720

gttacctgac caaggacgag ttcatcgagg cggttaaact ggctattaaa ggtgctaagg 780

cggtgtatca gaaacagcgt gaagcactga aagaaaagta tctgaaaatt gcccaggaag 840

ttgagggatc ctaaactagt gaattcgctt aagtcgaaca gaaagtaatc gtattgtaca 900

cggccgcata atcgaaatta atacgactca ctatagggag accacaacgg tttcccatct 960

tagtatatta gttaagtata agaaggagat ataccatggg tgtggataac aaatttaaca 1020

aagaacagca gaatgcgttt tacgaaattc tgcacctgcc gaaccttaac gaagaacagc 1080

gtaacgcttt cattcagtct ctgaaagatg acccttctca gtctgcgaac ctgctggctg 1140

aagctaaaaa actgaacgac gctcaggctc ctaaagttga caacaaattc aacaaagaac 1200

agcagaacgc tttctacgaa atcttgcatc tgccgaacct gaacgaagaa cagcgcaacg 1260

ctttcatcca gtccctgaaa gacgaccctt cccagtctgc taacctgctg gcggaagcga 1320

aaaaactgaa cgatgctcag gcgcctaaag tgggcgtcga ctctgataat gaaatcgtgg 1380

cgggtattat gcgcgaccat attattaacc tgcttaagga aggtaaacgt attgacgacc 1440

gcggtttcga agattaccgc ccgattgaaa ttgaggtagg tgttattgaa aaagcggagg 1500

gtagcgcgct ggtgaaactg ggtagcactc aggttctggt gggtattaaa acctctctgg 1560

gcgagccgtt tccggacacc ccgaacatgg gcgtgatgac caccaatgtt gaacttgttc 1620

cgctggcgtc tccgactttc gaaccgggcc cgccggacga gcgtgctatc gaactggcac 1680

gtgttattga ccgtggtatt cgtgagtcca aagcactgaa tctggaaaaa atggttatcg 1740

tgccgggcaa aattgttcgc gtagttttta tcgacgttca cgttcttgac cacgacggta 1800

acctgatgga cgccatcggt attgcagcta tcgccgcact gctgaacgca cgtgttccga 1860

aagttcgcta caacgaagaa acgggtgaag ttgaaaccct ggatgaaact gaaccgctgc 1920

cggttgaaaa gattccggtt ccggttactt tcgctaaaat tggtaatatc ctggtagttg 1980

acccgagcct ggatgaagag ctcgtgatgg acggcaaaat taccattacc actgacgaaa 2040

ccggccatat ttctgccgtt cagaaatctg aaggtggcgc tttcaaactg gaagaagtaa 2100

tgtacgcggt ggaaactgca ttcaagaagg cggaagaaat ccgtaaactg attctggaag 2160

ctgttgagaa ggccaaacag ctcgagtaag cggccgcaag cttgatccgg ctgctaacaa 2220

agcccgaaag gaagctgagt tggctgctgc caccgctgag caataactag cataacccct 2280

tggggcctct aaacgggtct tgaggggttt tttgctgaaa ggaggaacta tatccggatc 2340

tggcgtaata gcgaagaggc ccgcaccgat cgcccttccc aacagttgcg cagcctgaat 2400

ggcgaatggg acgcgccctg tagcggcgca ttaagcgcgg cgggtgtggt ggttacgcgc 2460

agcgtgaccg ctacacttgc cagcgcccta gcgcccgctc ctttcgcttt cttcccttcc 2520

tttctcgcca cgttcgccgg ctttccccgt caagctctaa atcgggggct ccctttaggg 2580

ttccgattta gtgctttacg gcacctcgac cccaaaaaac ttgattaggg tgatggttca 2640

cgtagtgggc catcgccctg atagacggtt tttcgccctt tgacgttgga gtccacgttc 2700

tttaatagtg gactcttgtt ccaaactgga acaacactca accctatctc ggtctattct 2760

tttgatttat aagggatttt gccgatttcg gcctattggt taaaaaatga gctgatttaa 2820

caaaaattta acgcgaattt taacaaaata ttaacgctta caatttaggt ggcacgtcga 2880

gttatggaca gcaagcgaac cggaattgcc agctggggcg ccctctggta aggttgggaa 2940

gccctgcaaa gtaaactgga tggctttctt gccgccaagg atctgatggc gcaggggatc 3000

aagatttgat caagagacag gatgaggatc gtttcgcatg attgaacaag atggattgca 3060

cgcaggttct ccggccgctt gggtggagag gctattcggc tatgactggg cacaacagac 3120

aatcggctgc tctgatgccg ccgtgttccg gctgtcagcg caggggcgcc cggttctttt 3180

tgtcaagacc gacctgtccg gtgccctgaa tgaactccaa gacgaggcag cgcggctatc 3240

gtggctggcc acgacgggcg ttccttgcgc agctgtgctc gacgttgtca ctgaagcggg 3300

aagggactgg ctgctattgg gcgaagtgcc ggggcaggat ctcctgtcat ctcaccttgc 3360

tcctgccgag aaagtatcca tcatggctga tgcaatgcgg cggctgcata cgcttgatcc 3420

ggctacctgc ccattcgacc accaagcgaa acatcgcatc gagcgagcac gtactcggat 3480

ggaagccggt cttgtcgatc aggatgatct ggacgaagag catcaggggc tcgcgccagc 3540

cgaactgttc gccaggctca aggcgcggat gcccgacggc gaggatctcg tcgtgaccca 3600

cggcgatgcc tgcttgccga atatcatggt ggaaaatggc cgcttttctg gattcatcga 3660

ctgtggccgg ctgggtgtgg cggaccgcta tcaggacata gcgttggcta cccgtgatat 3720

tgctgaagag cttggcggcg aatgggctga ccgcttcctc gtgctttacg gtatcgccgc 3780

tcccgattcg cagcgcatcg ccttctatcg ccttcttgac gagttcttct gagcgggact 3840

ctggggttcg aaattctagt gcattggtaa ctgtcagacc aagtttactc atatatactt 3900

tagattgatt taaaacttca tttttaattt aaaaggatct aggtgaagat cctttttgat 3960

aatctcatga ccaaaatccc ttaacgtgag ttttcgttcc actgagcgtc agaccccgta 4020

gaaaagatca aaggatcttc ttgagatcct ttttttctgc gcgtaatctg ctgcttgcaa 4080

acaaaaaaac caccgctacc agcggtggtt tgtttgccgg atcaagagct accaactctt 4140

tttccgaagg taactggctt cagcagagcg cagataccaa atactgttct tctagtgtag 4200

ccgtagttag gccaccactt caagaactct gtagcaccgc ctacatacct cgctctgcta 4260

atcctgttac cagtggctgc tgccagtggc gataagtcgt gtcttaccgg gttggactca 4320

agacgatagt taccggataa ggcgcagcgg tcgggctgaa cggggggttc gtgcacacag 4380

cccagcttgg agcgaacgac ctacaccgaa ctgagatacc tacagcgtga gctatgagaa 4440

agcgccacgc ttcccgaagg gagaaaggcg gacaggtatc cggtaagcgg cagggtcgga 4500

acaggagagc gcacgaggga gcttccaggg ggaaacgcct ggtatcttta tagtcctgtc 4560

gggtttcgcc acctctgact tgagcgtcga tttttgtgat gctcgtcagg ggggcggagc 4620

ctatggaaaa acgccagcag tcgagtttgt ctccgaccat caggcacctg agtcgctgtc 4680

tttttcgtga cattcagttc gctgcgctca cggctctggc agtgaatggg ggtaaatggc 4740

actacaggcg ccttttatgg attcatgcaa ggaaactacc cataatacaa gaaaagcccg 4800

tcacgggctt ctcagggcgt tttatggcgg gtctgctatg tggtgctatc tgactttttg 4860

ctgttcagca gttcctgccc tctgattttc cagtctgacc acttcggatt atcccgtcta 4920

gtacgcggcc tttttacggt tcctggcctt ttgctggcct tttgctcaca tgttctttcc 4980

tgcgttatcc cctgattctg tggataaccg tattaccgcc tttgagtgag ctgataccgc 5040

tcgccgcagc cgaacgaccg agcgcagcga gtcagtgagc gaggaagcgg aagagcgccc 5100

aatacgcaaa ccgcctctcc ccgcgcgttg gccgattcat taatgcag 5148

<210> 44
<211> 405
<212> Protein
<213> Artificial sequence

<220>
<223> Synthetic construct

<400> 42

Leu Ser Ile Arg Arg Arg Tyr Thr Met Gly Val Asp Asn Lys Phe Asn
1               5                   10                  15

Lys Glu Gln Gln Asn Ala Phe Tyr Glu Ile Leu His Leu Pro Asn Leu
            20                  25                  30

Asn Glu Glu Gln Arg Asn Ala Phe Ile Gln Ser Leu Lys Asp Asp Pro
        35                  40                  45

Ser Gln Ser Ala Asn Leu Leu Ala Glu Ala Lys Lys Leu Asn Asp Ala
    50                  55                  60

Gln Ala Pro Lys Val Asp Asn Lys Phe Asn Lys Glu Gln Gln Asn Ala
65                  70                  75                  80

Phe Tyr Glu Ile Leu His Leu Pro Asn Leu Asn Glu Glu Gln Arg Asn
                85                  90                  95

Ala Phe Ile Gln Ser Leu Lys Asp Asp Pro Ser Gln Ser Ala Asn Leu
            100                 105                 110

Leu Ala Glu Ala Lys Lys Leu Asn Asp Ala Gln Ala Pro Lys Val Gly
        115                 120                 125

Val Asp Ser Asp Asn Glu Ile Val Ala Gly Ile Met Arg Asp His Ile
    130                 135                 140

Ile Asn Leu Leu Lys Glu Gly Lys Arg Ile Asp Asp Arg Gly Phe Glu
145                 150                 155                 160

Asp Tyr Arg Pro Ile Glu Ile Glu Val Gly Val Ile Glu Lys Ala Glu
                165                 170                 175

Gly Ser Ala Leu Val Lys Leu Gly Ser Thr Gln Val Leu Val Gly Ile
            180                 185                 190

Lys Thr Ser Leu Gly Glu Pro Phe Pro Asp Thr Pro Asn Met Gly Val
        195                 200                 205

Met Thr Thr Asn Val Glu Leu Val Pro Leu Ala Ser Pro Thr Phe Glu
    210                 215                 220

Pro Gly Pro Pro Asp Glu Arg Ala Ile Glu Leu Ala Arg Val Ile Asp
225                 230                 235                 240

Arg Gly Ile Arg Glu Ser Lys Ala Leu Asn Leu Glu Lys Met Val Ile
                245                 250                 255

Val Pro Gly Lys Ile Val Arg Val Val Phe Ile Asp Val His Val Leu
            260                 265                 270

Asp His Asp Gly Asn Leu Met Asp Ala Ile Gly Ile Ala Ala Ile Ala

275                 280                 285

Ala Leu Leu Asn Ala Arg Val Pro Lys Val Arg Tyr Asn Glu Glu Thr
    290                 295                 300

Gly Glu Val Glu Thr Leu Asp Glu Thr Glu Pro Leu Pro Val Glu Lys
305                 310                 315                 320

Ile Pro Val Pro Val Thr Phe Ala Lys Ile Gly Asn Ile Leu Val Val
                325                 330                 335

Asp Pro Ser Leu Asp Glu Glu Leu Val Met Asp Gly Lys Ile Thr Ile
            340                 345                 350

Thr Thr Asp Glu Thr Gly His Ile Ser Ala Val Gln Lys Ser Glu Gly
        355                 360                 365

Gly Ala Phe Lys Leu Glu Glu Val Met Tyr Ala Val Glu Thr Ala Phe
    370                 375                 380

Lys Lys Ala Glu Glu Ile Arg Lys Leu Ile Leu Glu Ala Val Glu Lys
385                 390                 395                 400

Ala Lys Gln Leu Glu
                405

<210>   45
<211>   5148
<212>   DNA
<213>   Artificial sequence

<220>
<223>   Expression vector by fusion of recombinant proteins with Rrp41 and
ZZ-Rrp42 of P. abyssi exosome

<220>
<221>   CDS
<222>   (966)..(2189)
<223>   Z-Z Rrp42 Protein

<400>   41

gatctcgatc ccgcgaaatt aatacgactc actataggga gaccacaacg gtttccctct      60

agaataattt tgtttaactt taagaaggag atatacatat ggctacgcgt gcagagaaac     120

cggaaggtct gaaactgatt gatgaaaacg gccgccgtat tgacggtcgt aaaaaatacg     180

aactgcgtcc gattaaaatg gaagtgggtg tactgaaaaa cgctaacggt tccgcgtaca     240

tcgaatgggg caaaaacaaa attatcgcag cggtttacgg tccgcgtgaa ctccacccga     300

aacaccttca gcgtccggac cgcgccatcc tgcgtgtacg ttacaacatg gccccgttct 360

ctgttgaaga gactaagagc ccgggtccgg accgtcgtag cattgagatt tctaaagtaa 420

ttaaaggtgc gctggaaccg gcacttattc tggaaatgtt cccgcgtact gctattgatg 480

tattcatcga agttctgcaa gcggatgccg gtacgcgcgt tgcaggtatc accgcggcta 540

gcctggcact ggccgacgct ggtattccga tgcgcgacct ggttgccgct tgtgcggcgg 600

gtaaaatcga aggcgaaatt gttctggacc tgaacaaaga agaagataac tacggcgaag 660

cggacgtgcc ggtagctatc atgccgctga agaacgatat taccctgctg caaatggatg 720

gttacctgac caaggacgag ttcatcgagg cggttaaact ggctattaaa ggtgctaagg 780

cggtgtatca gaaacagcgt gaagcactga aagaaaagta tctgaaaatt gcccaggaag 840

ttgagggatc ctaaactagt gaattcgctt aagtcgaaca gaaagtaatc gtattgtaca 900

cggccgcata atcgaaatta atacgactca ctatagggag accacaacgg tttcccatct 960

tagta tat tag tta agt ata aga agg aga tat acc atg ggt gtg gat aac 1010
Tyr Leu Ser Ile Arg Arg Arg Tyr Thr Met Gly Val Asp Asn
1               5                   10

aaa ttt aac aaa gaa cag cag aat gcg ttt tac gaa att ctg cac ctg 1058
Lys Phe Asn Lys Glu Gln Gln Asn Ala Phe Tyr Glu Ile Leu His Leu
15                  20                  25                  30

ccg aac ctt aac gaa gaa cag cgt aac gct ttc att cag tct ctg aaa 1106
Pro Asn Leu Asn Glu Glu Gln Arg Asn Ala Phe Ile Gln Ser Leu Lys
                35                  40                  45

gat gac cct tct cag tct gcg aac ctg ctg gct gaa gct aaa aaa ctg 1154
Asp Asp Pro Ser Gln Ser Ala Asn Leu Leu Ala Glu Ala Lys Lys Leu
            50                  55                  60

aac gac gct cag gct cct aaa gtt gac aac aaa ttc aac aaa gaa cag 1202
Asn Asp Ala Gln Ala Pro Lys Val Asp Asn Lys Phe Asn Lys Glu Gln
        65                  70                  75

cag aac gct ttc tac gaa atc ttg cat ctg ccg aac ctg aac gaa gaa 1250
Gln Asn Ala Phe Tyr Glu Ile Leu His Leu Pro Asn Leu Asn Glu Glu
    80                  85                  90

cag cgc aac gct ttc atc cag tcc ctg aaa gac gac cct tcc cag tct 1298
Gln Arg Asn Ala Phe Ile Gln Ser Leu Lys Asp Asp Pro Ser Gln Ser
95                  100                 105                 110

gct aac ctg ctg gcg gaa gcg aaa aaa ctg aac gat gct cag gcg cct 1346
Ala Asn Leu Leu Ala Glu Ala Lys Lys Leu Asn Asp Ala Gln Ala Pro
                115                 120                 125

aaa gtg ggc gtc gac tct gat aat gaa atc gtg gcg ggt att atg cgc 1394
Lys Val Gly Val Asp Ser Asp Asn Glu Ile Val Ala Gly Ile Met Arg
            130                 135                 140

gac cat att att aac ctg ctt aag gaa ggt aaa cgt att gac gac cgc 1442
Asp His Ile Ile Asn Leu Leu Lys Glu Gly Lys Arg Ile Asp Asp Arg
        145                 150                 155

ggt ttc gaa gat tac cgc ccg att gaa att gag gta ggt gtt att gaa 1490
Gly Phe Glu Asp Tyr Arg Pro Ile Glu Ile Glu Val Gly Val Ile Glu
    160                 165                 170

aaa gcg gag ggt agc gcg ctg gtg aaa ctg ggt agc act cag gtt ctg 1538
Lys Ala Glu Gly Ser Ala Leu Val Lys Leu Gly Ser Thr Gln Val Leu
175                 180                 185                 190

gtg ggt att aaa acc tct ctg ggc gag ccg ttt ccg gac acc ccg aac 1586
Val Gly Ile Lys Thr Ser Leu Gly Glu Pro Phe Pro Asp Thr Pro Asn
                195                 200                 205

atg ggc gtg atg acc acc aat gtt gaa ctt gtt ccg ctg gcg tct ccg 1634
Met Gly Val Met Thr Thr Asn Val Glu Leu Val Pro Leu Ala Ser Pro
            210                 215                 220

act ttc gaa ccg ggc ccg ccg gac gag cgt gct atc gaa ctg gca cgt 1682
Thr Phe Glu Pro Gly Pro Pro Asp Glu Arg Ala Ile Glu Leu Ala Arg
        225                 230                 235

gtt att gac cgt ggt att cgt gag tcc aaa gca ctg aat ctg gaa aaa 1730
Val Ile Asp Arg Gly Ile Arg Glu Ser Lys Ala Leu Asn Leu Glu Lys
    240                 245                 250

atg gtt atc gtg ccg ggc aaa att gtt cgc gta gtt ttt atc gac gtt 1778
Met Val Ile Val Pro Gly Lys Ile Val Arg Val Val Phe Ile Asp Val
255                 260                 265                 270

cac gtt ctt gac cac gac ggt aac ctg atg gac gcc atc ggt att gca 1826
His Val Leu Asp His Asp Gly Asn Leu Met Asp Ala Ile Gly Ile Ala
                275                 280                 285

gct atc gcc gca ctg ctg aac gca cgt gtt ccg aaa gtt cgc tac aac 1874
Ala Ile Ala Ala Leu Leu Asn Ala Arg Val Pro Lys Val Arg Tyr Asn
            290                 295                 300

gaa gaa acg ggt gaa gtt gaa acc ctg gat gaa act gaa ccg ctg ccg 1922
Glu Glu Thr Gly Glu Val Glu Thr Leu Asp Glu Thr Glu Pro Leu Pro
        305                 310                 315

gtt gaa aag att ccg gtt ccg gtt act ttc gct aaa att ggt aat atc 1970
Val Glu Lys Ile Pro Val Pro Val Thr Phe Ala Lys Ile Gly Asn Ile
    320                 325                 330

ctg gta gtt gac ccg agc ctg gat gaa gag ctc gtg atg gac ggc aaa 2018
Leu Val Val Asp Pro Ser Leu Asp Glu Glu Leu Val Met Asp Gly Lys
335                 340                 345                 350

att acc att acc act gac gaa acc ggc cat att tct gcc gtt cag aaa 2066
Ile Thr Ile Thr Thr Asp Glu Thr Gly His Ile Ser Ala Val Gln Lys
                355                 360                 365

tct gaa ggt ggc gct ttc aaa ctg gaa gaa gta atg tac gcg gtg gaa 2114
Ser Glu Gly Gly Ala Phe Lys Leu Glu Glu Val Met Tyr Ala Val Glu
            370                 375                 380

act gca ttc aag aag gcg gaa gaa atc cgt aaa ctg att ctg gaa gct 2162
Thr Ala Phe Lys Lys Ala Glu Glu Ile Arg Lys Leu Ile Leu Glu Ala
        385                 390                 395

gtt gag aag gcc aaa cag ctc gag taa gcggccgcaa gcttgatccg 2209
Val Glu Lys Ala Lys Gln Leu Glu
    400                 405

gctgctaaca aagcccgaaa ggaagctgag ttggctgctg ccaccgctga gcaataacta 2269

gcataacccc ttggggcctc taaacgggtc ttgaggggtt ttttgctgaa aggaggaact 2329

atatccggat ctggcgtaat agcgaagagg cccgcaccga tcgcccttcc caacagttgc 2389

gcagcctgaa tggcgaatgg gacgcgccct gtagcggcgc attaagcgcg gcgggtgtgg 2449

tggttacgcg cagcgtgacc gctacacttg ccagcgccct agcgcccgct cctttcgctt 2509

tcttcccttc ctttctcgcc acgttcgccg gctttccccg tcaagctcta aatcgggggc 2569

tccctttagg gttccgattt agtgctttac ggcacctcga ccccaaaaaa cttgattagg 2629

gtgatggttc acgtagtggg ccatcgccct gatagacggt ttttcgccct ttgacgttgg 2689

agtccacgtt ctttaatagt ggactcttgt tccaaactgg aacaacactc aaccctatct 2749

cggtctattc ttttgattta taagggattt tgccgatttc ggcctattgg ttaaaaaatg 2809

agctgattta acaaaaattt aacgcgaatt ttaacaaaat attaacgctt acaatttagg 2869

tggcacgtcg agttatggac agcaagcgaa ccggaattgc cagctggggc gccctctggt 2929

aaggttggga agccctgcaa agtaaactgg atggctttct tgccgccaag gatctgatgg 2989

cgcaggggat caagatttga tcaagagaca ggatgaggat cgtttcgcat gattgaacaa 3049

gatggattgc acgcaggttc tccggccgct tgggtggaga ggctattcgg ctatgactgg 3109

gcacaacaga caatcggctg ctctgatgcc gccgtgttcc ggctgtcagc gcaggggcgc 3169

ccggttcttt ttgtcaagac cgacctgtcc ggtgccctga atgaactcca agacgaggca 3229

gcgcggctat cgtggctggc cacgacgggc gttccttgcg cagctgtgct cgacgttgtc 3289

actgaagcgg gaagggactg gctgctattg ggcgaagtgc cggggcagga tctcctgtca 3349

tctcaccttg ctcctgccga gaaagtatcc atcatggctg atgcaatgcg gcggctgcat 3409

acgcttgatc cggctacctg cccattcgac caccaagcga aacatcgcat cgagcgagca 3469

cgtactcgga tggaagccgg tcttgtcgat caggatgatc tggacgaaga gcatcagggg 3529

ctcgcgccag ccgaactgtt cgccaggctc aaggcgcgga tgcccgacgg cgaggatctc 3589

gtcgtgaccc acggcgatgc ctgcttgccg aatatcatgg tggaaaatgg ccgcttttct 3649

ggattcatcg actgtggccg gctgggtgtg gcggaccgct atcaggacat agcgttggct 3709

acccgtgata ttgctgaaga gcttggcggc gaatgggctg accgcttcct cgtgctttac 3769

ggtatcgccg ctcccgattc gcagcgcatc gccttctatc gccttcttga cgagttcttc 3829

tgagcgggac tctggggttc gaaattctag tgcattggta actgtcagac caagtttact 3889

catatatact ttagattgat ttaaaacttc atttttaatt taaaaggatc taggtgaaga 3949

tcctttttga taatctcatg accaaaatcc cttaacgtga gttttcgttc cactgagcgt 4009

cagaccccgt agaaaagatc aaaggatctt cttgagatcc tttttttctg cgcgtaatct 4069

gctgcttgca aacaaaaaaa ccaccgctac cagcggtggt ttgtttgccg gatcaagagc 4129

taccaactct ttttccgaag gtaactggct tcagcagagc gcagatacca aatactgttc 4189

ttctagtgta gccgtagtta ggccaccact tcaagaactc tgtagcaccg cctacatacc 4249

tcgctctgct aatcctgtta ccagtggctg ctgccagtgg cgataagtcg tgtcttaccg 4309

ggttggactc aagacgatag ttaccggata aggcgcagcg gtcgggctga acggggggtt 4369

cgtgcacaca gcccagcttg gagcgaacga cctacaccga actgagatac ctacagcgtg 4429

agctatgaga aagcgccacg cttcccgaag ggagaaaggc ggacaggtat ccggtaagcg 4489

gcagggtcgg aacaggagag cgcacgaggg agcttccagg gggaaacgcc tggtatcttt 4549

atagtcctgt cgggtttcgc cacctctgac ttgagcgtcg atttttgtga tgctcgtcag 4609

gggggcggag cctatggaaa aacgccagca gtcgagtttg tctccgacca tcaggcacct 4669

gagtcgctgt ctttttcgtg acattcagtt cgctgcgctc acggctctgg cagtgaatgg 4729

gggtaaatgg cactacaggc gccttttatg gattcatgca aggaaactac ccataataca 4789

agaaaagccc gtcacgggct tctcagggcg ttttatggcg ggtctgctat gtggtgctat 4849

ctgacttttt gctgttcagc agttcctgcc ctctgatttt ccagtctgac cacttcggat 4909

tatcccgtct agtacgcggc ctttttacgg ttcctggcct tttgctggcc ttttgctcac 4969

atgttctttc ctgcgttatc ccctgattct gtggataacc gtattaccgc ctttgagtga 5029

gctgataccg ctcgccgcag ccgaacgacc gagcgcagcg agtcagtgag cgaggaagcg 5089

gaagagcgcc caatacgcaa accgcctctc cccgcgcgtt ggccgattca ttaatgcag 5148